(19) Europäisches Patentamt
European Patent Office
Office européen des brevets

(11) EP 4 083 142 B1

(12) EUROPEAN PATENT SPECIFICATION

(45) Date of publication and mention of the grant of the patent:
**13.08.2025 Bulletin 2025/33**

(21) Application number: **20904524.4**

(22) Date of filing: **09.11.2020**

(51) International Patent Classification (IPC):
***C08K 5/00*** *(2006.01)* ***C08K 5/45*** *(2006.01)*
***C07D 409/04*** *(2006.01)* ***C07D 409/14*** *(2006.01)*
***C07D 495/04*** *(2006.01)* ***C09D 125/12*** *(2006.01)*
***G02B 1/04*** *(2006.01)* ***G02B 5/20*** *(2006.01)*
***G02B 5/22*** *(2006.01)*

(52) Cooperative Patent Classification (CPC):
(C-Sets available)
**G02B 5/223; C07D 409/04; C07D 409/14; C07D 495/04; C08K 5/45; C09D 125/12; G02B 1/041; G02B 5/208;** C08K 5/005 (Cont.)

(86) International application number:
**PCT/JP2020/041670**

(87) International publication number:
**WO 2021/131355 (01.07.2021 Gazette 2021/26)**

(54) **RESIN COMPOSITION, CURED PRODUCT, UV ABSORBER, UV CUT FILTER, LENS, PROTECTIVE MATERIAL, COMPOUND, AND METHOD FOR SYNTHESIZING COMPOUND**

HARZZUSAMMENSETZUNG, GEHÄRTETES PRODUKT, UV-ABSORBER, UV-SCHNITTFILTER, LINSE, SCHUTZMATERIAL, VERBINDUNG UND VERFAHREN ZUR SYNTHETISIERUNG EINER VERBINDUNG

COMPOSITION DE RÉSINE, PRODUIT DURCI, ABSORBEUR UV, FILTRE BLOQUANT LES UV, LENTILLE, MATÉRIAU PROTECTEUR, COMPOSÉ ET PROCÉDÉ DE SYNTHÈSE DE COMPOSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2019 JP 2019233986**
**06.03.2020 JP 2020039393**
**03.09.2020 JP 2020148522**

(43) Date of publication of application:
**02.11.2022 Bulletin 2022/44**

(73) Proprietor: **FUJIFILM Corporation**
**Tokyo 106-8620 (JP)**

(72) Inventors:
- **FURUYAMA, Hidetomo**
  **Fujinomiya-shi, Shizuoka 418-8666 (JP)**
- **SASAKI, Daisuke**
  **Fujinomiya-shi, Shizuoka 418-8666 (JP)**
- **JIMBO, Yoshihiro**
  **Fujinomiya-shi, Shizuoka 418-8666 (JP)**
- **KUWAHARA, Hiroki**
  **Fujinomiya-shi, Shizuoka 418-8666 (JP)**

(74) Representative: **Hoffmann Eitle**
**Patent- und Rechtsanwälte PartmbB**
**Arabellastraße 30**
**81925 München (DE)**

(56) References cited:
**WO-A1-2019/044863**
**WO-A1-2019/142539**
**WO-A1-2019/159570**
**JP-A- 2009 209 126**
**JP-A- 2009 263 616**
**JP-A- 2009 263 617**
**JP-A- 2009 298 898**
**JP-A- 2010 180 288**
**JP-A- 2011 053 596**
**JP-A- 2019 191 219**
**JP-B2- 6 563 116**
**US-A1- 2009 189 120**
**US-A1- 2010 210 762**
**US-A1- 2018 371 255**
**US-A1- 2020 199 095**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE CA [online] CHEMICAL ABSTRACTS SERVICE, COLUMBUS, OHIO, US; 1 November 2019 (2019-11-01), KATO, TAKASHI: "Spectacle lens containing a benzodithyrane-based UV absorber and spectacles", XP002808300, retrieved from STN Database accession no. 2019:2066796**

(52) Cooperative Patent Classification (CPC): (Cont.)

C-Sets
**C08K 5/45, C08L 33/08;**
**C09D 125/12, C08K 5/45;**
**G02B 1/041, C08K 5/0041;**
C08K 5/005, C08L 33/08

## Description

## BACKGROUND OF THE INVENTION

1. Field of the Invention

The present invention relates to a resin composition. More specifically, the present invention relates to a resin composition containing a benzodithiol compound. Further, the present invention relates to a cured substance, an ultraviolet absorbing agent, an ultraviolet cut filter, a lens, a protective material, a compound, and a method of synthesizing a compound.

2. Description of the Related Art

A benzodithiol compound has excellent absorbency of ultraviolet rays and has been used as an ultraviolet absorbing agent or the like. For example, WO2019/159570A describes that a specific benzodithiol compound is used as an ultraviolet absorbing agent.

## SUMMARY OF THE INVENTION

An ultraviolet absorbing agent is required to have less coloring as one of the characteristics to be required. Further, in recent years, the ultraviolet absorbing agent has also been required to have a high absorption ability with respect to ultraviolet rays having a long wavelength of approximately 400 nm.

Therefore, an object of the present invention is to provide a resin composition, a cured substance, an ultraviolet absorbing agent, an ultraviolet cut filter, a lens, a protective material, a compound, and a method of synthesizing a compound, that enable production of a cured substance with an excellent absorption ability with respect to ultraviolet rays having a wavelength of approximately 400 nm.

As a result of intensive research on a compound having a skeleton which is represented by Formula (1), the present inventors found that the compound represented by Formula (1) described below is a compound which has an excellent absorption ability with respect to ultraviolet rays having a wavelength of approximately 400 nm, has less coloring, and is useful as an ultraviolet absorbing agent, thereby completing the present invention. Therefore, the present invention provides the followings.

<1> A resin composition comprising: a compound represented by Formula (1) as defined in claim 1; and a resin,

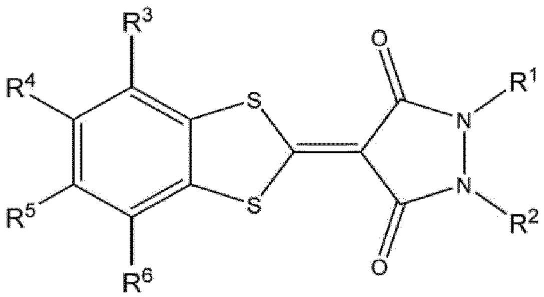

(1)

in Formula (1), $R^1$ and $R^2$ each independently represent an alkyl group, an aryl group or a heterocyclic group,
$R^3$ and $R^6$ each independently represent an alkoxy group, an acyloxy group, a carbamoyloxy group, or an alkoxycarbonyloxy group,
$R^4$ represents an alkyl group, an aryl group, an alkoxy group, or an aryloxy group,
$R^5$ represents a hydrogen atom, an alkyl group, an aryl group, an alkoxy group, or an aryloxy group
$R^1$ and $R^2$ may be bonded to each other to form a ring,
$R^3$ and $R^4$ may be bonded to each other to form a ring,
$R^4$ and $R^5$ may be bonded to each other to form a ring, and
$R^5$ and $R^6$ may be bonded to each other to form a ring,
where in a case where $R^3$ and $R^6$ each independently represent an acyloxy group or a carbamoyloxy group, at least one of $R^4$ or $R^5$ represents an aryl group, an alkoxy group, or an aryloxy group.

<3> The resin composition according to <1>, in which in Formula (1), at least one of $R^3$ or $R^6$ represents an alkoxy group.

<4> The resin composition according to <1>, in which the compound represented by Formula (1) is a compound represented by Formula (1a),

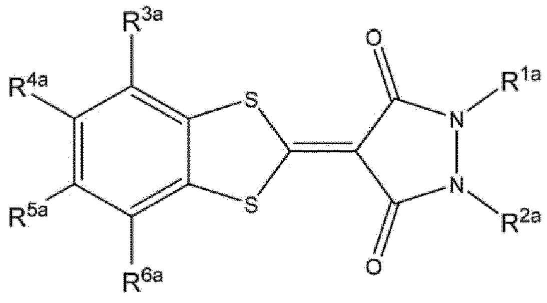

(1a)

in Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,
$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group.

<5> The resin composition according to any one of <1> to <4>, further comprising: a compound represented by Formula (2),

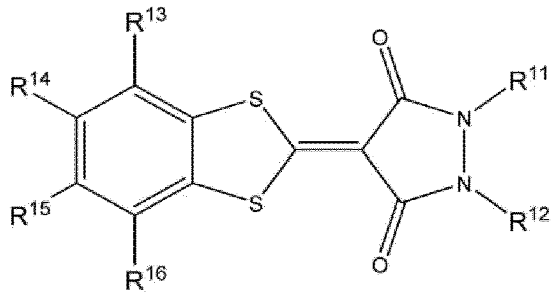

(2)

in Formula (2), $R^{11}$ and $R^{12}$ each independently represent an alkyl group, an aryl group, or a heterocyclic group,
$R^{13}$ and $R^{16}$ each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group,
$R^{14}$ represents an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,
$R^{15}$ represents a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,
$R^{11}$ and $R^{12}$ may be bonded to each other to form a ring,
$R^{13}$ and $R^{14}$ may be bonded to each other to form a ring,
$R^{14}$ and $R^{15}$ may be bonded to each other to form a ring, and
$R^{15}$ and $R^{16}$ may be bonded to each other to form a ring, where at least one of $R^{13}$ or $R^{16}$ represents a hydroxy

group.

<6> The resin composition according to any one of <1> to <5>, further comprising: an ultraviolet absorbing agent other than the compound represented by Formula (1).
<7> The resin composition according to any one of <1> to <6>, in which the resin is at least one selected from a (meth) acrylic resin, a polystyrene resin, a polyester resin, a polyurethane resin, a polythiourethane resin, a polyimide resin, an epoxy resin, a polycarbonate resin, or a cellulose acylate resin.
<8> A cured substance which is formed of the resin composition according to any one of <1> to <7>.
<9> An ultraviolet absorbing agent comprising: a compound represented by Formula (1) as defined in claim 1,

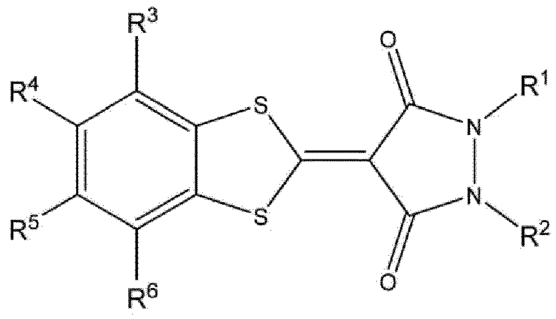

(1)

in Formula (1), $R^1$ and $R^2$ each independently represent an alkyl group, an aryl group or a heterocyclic group,
$R^3$ and $R^6$ each independently represent an alkoxy group, an acyloxy group, a carbamoyloxy group, or an alkoxycarbonyloxy group,
$R^4$ represents an alkyl group, an aryl group, an alkoxy group, or an aryloxy group,
$R^5$ represents a hydrogen atom, an alkyl group, an aryl group, an alkoxy group, or an aryloxy group,
$R^1$ and $R^2$ may be bonded to each other to form a ring,
$R^3$ and $R^4$ may be bonded to each other to form a ring,
$R^4$ and $R^5$ may be bonded to each other to form a ring, and
$R^5$ and $R^6$ may be bonded to each other to form a ring,
where in a case where $R^3$ and $R^6$ each independently represent an acyloxy group or a carbamoyloxy group, at least one of $R^4$ or $R^5$ represents an aryl group, an alkoxy group, or an aryloxy group.

<10> The ultraviolet absorbing agent according to <9>, further comprising: the compound represented by Formula (2),

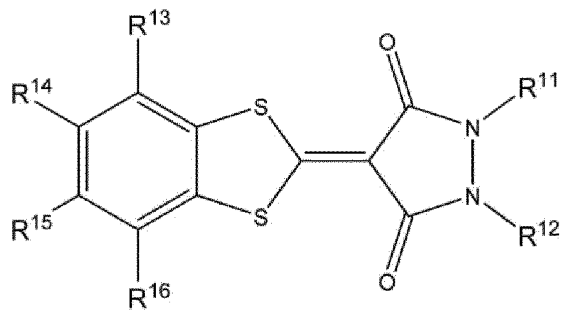

(2)

in Formula (2), $R^{11}$ and $R^{12}$ each independently represent an alkyl group, an aryl group, or a heterocyclic group,
$R^{13}$ and $R^{16}$ each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group,
$R^{14}$ represents an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,
$R^{15}$ represents a hydrogen atom, an alkyl group, an aryl group, an alkoxy group, an aryloxy group, an acyloxy

group, an alkylamino group, an anilino group, an acylamino group, an alkylsulfonylamino group, an arylsulfonylamino group, an alkylthio group, or an arylthio group,
$R^{11}$ and $R^{12}$ may be bonded to each other to form a ring,
$R^{13}$ and $R^{14}$ may be bonded to each other to form a ring,
$R^{14}$ and $R^{15}$ may be bonded to each other to form a ring, and
$R^{15}$ and $R^{16}$ may be bonded to each other to form a ring,
where at least one of $R^{13}$ or $R^{16}$ represents a hydroxy group.

<11> An ultraviolet cut filter comprising: the ultraviolet absorbing agent according to <9> or <10>.
<12> A lens comprising: the ultraviolet absorbing agent according to <9> or <10>.
<13> A protective material comprising: the ultraviolet absorbing agent according to <9> or <10>.
<14> A compound which is represented by Formula (1a),

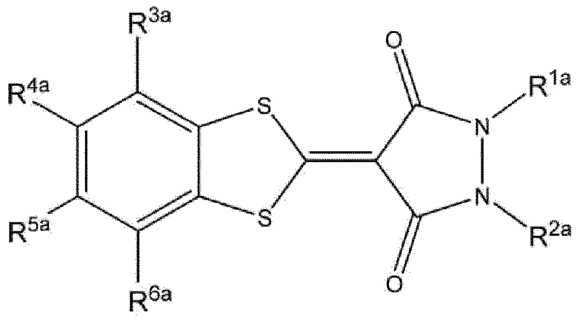

(1a)

in Formula (1a), $R^{a}$ and $R^{2a}$ each independently represent an alkyl group,
$R^{3a}$ and $R^{6a}$ each independently represent an alkoxy group or an acyloxy group,
$R^4$a represents an alkyl group or an alkoxy group,
$R^{5a}$ represents a hydrogen atom, an alkyl group, or an alkoxy group,
$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group.

<15> A method of synthesizing a compound represented by Formula (1a), comprising: reacting a compound represented by Formula (2a) with an alkyl halide compound or a carboxylic acid halide,

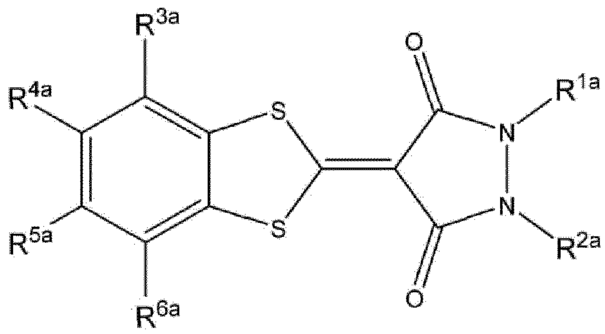

(1a)

in Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,

$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group,

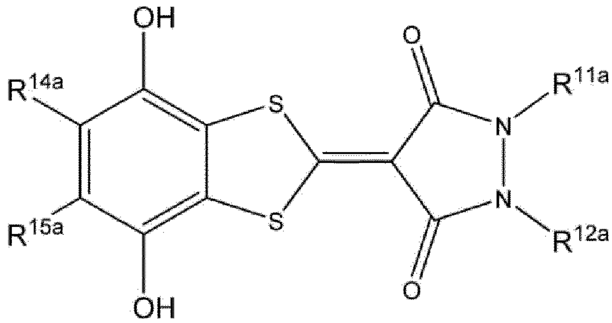
(2a)

in Formula (2a), $R^{11a}$ and $R^{12a}$ each independently represent an alkyl group,
$R^{14a}$ represents an alkyl group or an alkoxy group,
$R^{15a}$ represents a hydrogen atom, an alkyl group, or an alkoxy group, and
$R^{14a}$ and $R^{15a}$ may be bonded to each other to form a ring.

According to the present invention, it is possible to provide a resin composition, a cured substance, an ultraviolet absorbing agent, an ultraviolet cut filter, a lens, a protective material, a compound, and a method of synthesizing a compound, that enable production of a cured substance with an excellent absorption ability with respect to ultraviolet rays having a wavelength of approximately 400 nm.

## BRIEF DESCRIPTION OF THE DRAWINGS

Fig. 1 is a schematic view illustrating an embodiment of a liquid crystal display device configured such that an ultraviolet absorbing agent of the present invention is added to a polarizing plate protective film on a front side.
Fig. 2 is a schematic view illustrating an embodiment of a liquid crystal display device configured such that the ultraviolet absorbing agent of the present invention is added to a polarizing plate protective film on a backlight side.
Fig. 3 is a schematic view illustrating an embodiment of a liquid crystal display device configured such that the ultraviolet absorbing agent of the present invention is added to an inner protective film on a front side.
Fig. 4 is a schematic view illustrating an embodiment of a liquid crystal display device configured such that the ultraviolet absorbing agent of the present invention is added to an inner protective film on a backlight side.
Fig. 5 is a schematic view illustrating an embodiment of a liquid crystal display device configured such that an optical film containing the ultraviolet absorbing agent of the present invention is bonded to a phase difference film on a front side via an adhesive or a pressure sensitive adhesive.
Fig. 6 is a schematic view illustrating an embodiment of a liquid crystal display device configured such that an optical film containing the ultraviolet absorbing agent of the present invention is bonded to a phase difference film on a backlight side via an adhesive or a pressure sensitive adhesive.
Fig. 7 is a schematic view illustrating an embodiment of a liquid crystal display device configured such that the ultraviolet absorbing agent of the present invention is added to an adhesive or a pressure sensitive adhesive on a front side.
Fig. 8 is a schematic view illustrating an embodiment of a liquid crystal display device configured such that the ultraviolet absorbing agent of the present invention is added to an adhesive or a pressure sensitive adhesive on a backlight side.
Fig. 9 is a schematic view illustrating an embodiment of a liquid crystal display device configured such that the ultraviolet absorbing agent of the present invention is added to a functional layer on a front side.
Fig. 10 is a schematic view illustrating an embodiment of a liquid crystal display device configured such that the ultraviolet absorbing agent of the present invention is added to a functional layer on a backlight side.
Fig. 11 is a schematic view illustrating an embodiment of an organic electroluminescence display device configured such that the ultraviolet absorbing agent of the present invention is added to a polarizing plate protective film.

Fig. 12 is a schematic view illustrating an embodiment of an organic electroluminescence display device configured such that the ultraviolet absorbing agent of the present invention is added to an adhesive or a pressure sensitive adhesive.

Fig. 13 is a schematic view illustrating an embodiment of an organic electroluminescence display device configured such that an optical film containing the ultraviolet absorbing agent of the present invention is bonded to a touch panel via an adhesive or a pressure sensitive adhesive.

## DESCRIPTION OF THE PREFERRED EMBODIMENTS

Hereinafter, the contents of the present disclosure will be described in detail.

In a case where substitution or unsubstitution is not specified in the notation of a group (atomic group) in the present specification, the group includes both a group which has no substituent and a group which has a substituent. For example, "alkyl group" includes not only an alkyl group having no substituent (unsubstituted alkyl group), but also an alkyl group having a substituent (substituted alkyl group).

In the present specification, a numerical range shown using "to" indicates a range including the numerical values described before and after "to" as the lower limit and the upper limit.

In the present specification, the total solid content denotes the total amount of components excluding solvents from all the components of the resin composition.

In the present specification, "(meth)acrylate" denotes both or any one of acrylate and methacrylate, "(meth)acryl" denotes both or any one of acryl and methacryl, "(meth)allyl" denotes both or any one of allyl and methallyl, and "(meth) acryloyl" denotes both or any one of acryloyl and methacryloyl.

In the present specification, the meaning of the term "step" includes not only an independent step but also a step whose intended purpose is achieved even in a case where the step is not clearly distinguished from other steps.

In the present specification, the weight-average molecular weight (Mw) and the number average molecular weight (Mn) are defined as values in terms of polystyrene, measured by gel permeation chromatography (GPC).

<Resin composition>

A resin composition according to the embodiment of the present invention contains a compound represented by Formula (1) and a resin.

The compound represented by Formula (1) is a compound which has an excellent absorption ability with respect to ultraviolet rays having a wavelength of approximately 400 nm and has less coloring. Therefore, the resin composition according to the embodiment of the present invention can produce a cured substance having an excellent absorption ability with respect to ultraviolet rays having a wavelength of approximately 400 nm.

Further, the compound represented by Formula (1) has satisfactory compatibility with a resin and can suppress surface unevenness on a surface of a cured substance. The detailed reason why such an effect is obtained is unknown, but it is presumed that the compound represented by Formula (1) is likely to be twisted between $R^3$ and $R^4$ due to the influence of steric repulsion It is presumed that occurrence of such twisting leads to a decrease in the crystallinity of the compound and improvement of compatibility with a resin.

Hereinafter, the resin composition according to the embodiment of the present invention will be described in detail.

<<Compound represented by Formula (1) (compound (1))>>

The resin composition according to the embodiment of the present invention contains a compound represented by Formula (1) as defined in claim 1 (hereinafter, also referred to as a compound (1)).

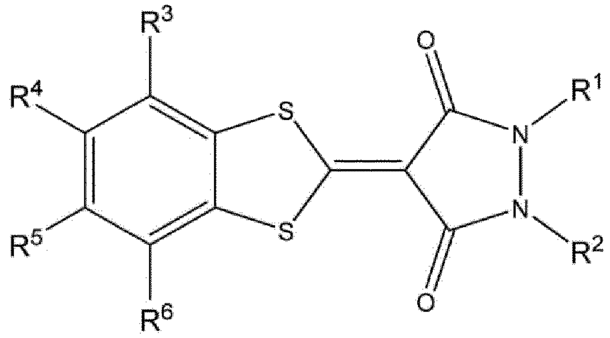

(1)

in Formula (1), $R^1$ and $R^2$ each independently represent an alkyl group, an aryl group or a heterocyclic group,
$R^3$ and $R^6$ each independently represent an alkoxy group, an acyloxy group, a carbamoyloxy group, or an alkoxycarbonyloxy group,
$R^4$ represents an alkyl group, an aryl group, an alkoxy group, or an aryloxy group,
$R^5$ represents a hydrogen atom, an alkyl group, an aryl group, an alkoxy group, or an aryloxy group,
$R^1$ and $R^2$ may be bonded to each other to form a ring,
$R^3$ and $R^4$ may be bonded to each other to form a ring,
$R^4$ and $R^5$ may be bonded to each other to form a ring, and
$R^5$ and $R^6$ may be bonded to each other to form a ring,
where in a case where $R^3$ and $R^6$ each independently represent an acyloxy group or a carbamoyloxy group, at least one of $R^4$ or $R^5$ represents an aryl group, an alkoxy group, or an aryloxy group.

In Formula (1), $R^1$ and $R^2$ each independently represent an alkyl group, an aryl group, or a heterocyclic group and preferably an alkyl group or an aryl group. From the viewpoint of light resistance, it is preferable that $R^1$ and $R^2$ are each independently represent an alkyl group. Further, from the viewpoint of the absorbency of ultraviolet rays having a wavelength of approximately 400 nm, it is preferable that $R^1$ and $R^2$ each independently represent an aryl group.

The number of carbon atoms of the alkyl group represented by $R^1$ and $R^2$ is preferably in a range of 1 to 30, more preferably in a range of 1 to 20, still more preferably in a range of 1 to 15, particularly preferably in a range of 1 to 10, and most preferably in a range of 1 to 8. The alkyl group may be linear, branched, or cyclic and preferably linear or branched. The alkyl group may have a substituent. Examples of the substituent include groups described in the section of the substituent T described below.

The number of carbon atoms of the aryl group represented by $R^1$ and $R^2$ is preferably in a range of 6 to 40, more preferably in a range of 6 to 30, still more preferably in a range of 6 to 20, particularly preferably in a range of 6 to 15, and most preferably in a range of 6 to 12. As the aryl group, a phenyl group or a naphthyl group is preferable, and a phenyl group is more preferable. Further, the aryl group may have a substituent. Examples of the substituent include groups described in the section of the substituent T described below.

It is preferable that the heterocyclic ring in the heterocyclic group represented by $R^1$ and $R^2$ contains a 5- or 6-membered saturated or unsaturated heterocyclic ring. The heterocyclic ring may be fused with an aliphatic ring, an aromatic ring, or another heterocyclic ring. Examples of the heteroatom constituting the ring of the heterocyclic ring include B, N, O, S, Se, and Te. Among these, N, O and S are preferable. It is preferable that the carbon atom of the heterocyclic ring has a free valence (monovalent) (the heterocyclic group is bonded at the carbon atom). The number of carbon atoms of the heterocyclic group is preferably in a range of 1 to 40, more preferably in a range of 1 to 30, and still more preferably in a range of 1 to 20. Examples of the saturated heterocyclic ring in the heterocyclic group include a pyrrolidine ring, a morpholine ring, a 2-bora-1,3-dioxolane ring, and a 1,3-thiazolidine ring. Examples of the unsaturated heterocyclic ring in the heterocyclic group include an imidazole ring, a thiazole ring, a benzothiazole ring, a benzoxazole ring, a benzotriazole ring, a benzoselenazole ring, a pyridine ring, a pyrimidine ring, and a quinoline ring. The heterocyclic group may have a substituent. Examples of the substituent include groups described in the section of the substituent T described below.

$R^1$ and $R^2$ may be bonded to each other to form a ring. It is preferable that the ring formed by $R^1$ and $R^2$ being bonded to each other is a 5- or 6-membered ring. The ring formed by $R^1$ and $R^2$ being bonded to each other may have a substituent. Examples of the substituent include groups described in the section of the substituent T described below.

In Formula (1), $R^3$ and $R^6$ each independently represent an alkoxy group, an acyloxy group, a carbamoyloxy group, or an alkoxycarbonyloxy group and preferably an alkoxy group or an acyloxy group. Further, from the viewpoint of easily enhancing the absorbency of ultraviolet rays having a wavelength of approximately 400 nm while suppressing coloring, it is more preferable that at least one of $R^3$ and $R^6$ represents an alkoxy group. As a result of examination

conducted by the present inventors, it was found that the maximum absorption wavelength of the compound is easily shifted to a longer wavelength side as the substituent on a benzene ring of benzodithiol is a group with a higher electron donating ability. Since the alkoxy group is a substituent with a higher electron donating ability, it is presumed that the maximum absorption wavelength of the compound can be shifted to a longer wavelength side. It is particularly preferable that both $R^3$ and $R^6$ represent an alkoxy group.

The number of carbon atoms of the alkoxy group represented by $R^3$ and $R^6$ is preferably in a range of 1 to 30, more preferably in a range of 1 to 20, still more preferably in a range of 1 to 15, particularly preferably in a range of 1 to 10, and most preferably in a range of 1 to 8. The alkoxy group may be linear or branched. The alkoxy group may have a substituent. Examples of the substituent include groups described in the section of the substituent T described below.

The number of carbon atoms of the acyloxy group represented by $R^3$ and $R^6$ is preferably in a range of 2 to 30, more preferably in a range of 2 to 20, still more preferably in a range of 2 to 15, and particularly preferably in a range of 2 to 10. The acyloxy group may have a substituent. Examples of the substituent include groups described in the section of the substituent T described below.

The number of carbon atoms of the carbamoyloxy group represented by $R^3$ and $R^6$ is preferably in a range of 2 to 30, more preferably in a range of 2 to 20, still more preferably in a range of 2 to 15, particularly preferably in a range of 2 to 10, and most preferably in a range of 2 to 8. The carbamoyloxy group may be linear or branched. The carbamoyloxy group may have a substituent. Examples of the substituent include groups described in the section of the substituent T described below.

The number of carbon atoms of the alkoxycarbonyloxy group represented by $R^3$ and $R^6$ is preferably in a range of 2 to 30, more preferably in a range of 2 to 20, still more preferably in a range of 2 to 15, particularly preferably in a range of 2 to 10, and most preferably in a range of 2 to 8. The alkoxycarbonyloxy group may be linear or branched. The alkoxycarbonyloxy group may have a substituent. Examples of the substituent include groups described in the section of the substituent T described below.

In Formula (1), $R^4$ represents an alkyl group, an aryl group, an alkoxy group, or an aryloxy group, and $R^5$ represents a hydrogen atom, an alkyl group, an aryl group, an alkoxy group, or an aryloxy group.

The number of carbon atoms of the alkyl group represented by $R^4$ and $R^5$ is preferably in a range of 1 to 30, more preferably in a range of 1 to 20, still more preferably in a range of 1 to 15, particularly preferably in a range of 1 to 10, and most preferably in a range of 1 to 8. The alkyl group may be linear, branched, or cyclic and preferably linear or branched. The alkyl group may have a substituent. Examples of the substituent include groups described in the section of the substituent T described below.

The number of carbon atoms of the aryl group represented by $R^4$ and $R^5$ is preferably in a range of 6 to 40, more preferably in a range of 6 to 30, still more preferably in a range of 6 to 20, particularly preferably in a range of 6 to 15, and most preferably in a range of 6 to 12. As the aryl group, a phenyl group or a naphthyl group is preferable, and a phenyl group is more preferable. Further, the aryl group may have a substituent. Examples of the substituent include groups described in the section of the substituent T described below.

The number of carbon atoms of the alkoxy group represented by $R^4$ and $R^5$ is preferably in a range of 1 to 30, more preferably in a range of 1 to 20, still more preferably in a range of 1 to 15, particularly preferably in a range of 1 to 10, and most preferably in a range of 1 to 8. The alkoxy group may be linear or branched. The alkoxy group may have a substituent. Examples of the substituent include groups described in the section of the substituent T described below.

The number of carbon atoms of the aryloxy group represented by $R^4$ and $R^5$ is preferably in a range of 6 to 40, more preferably in a range of 6 to 30, still more preferably in a range of 6 to 20, particularly preferably in a range of 6 to 15, and most preferably in a range of 6 to 12. The aryloxy group may have a substituent. Examples of the substituent include groups described in the section of the substituent T described below.

In Formula (1), $R^3$ and $R^4$ may be bonded to each other to form a ring, $R^4$ and $R^5$ may be bonded to each other to form a ring, and $R^5$ and $R^6$ may be bonded to each other to form a ring. It is preferable that the ring formed by these groups being bonded to each other is a 5- or 6-membered ring. The ring formed by these groups being bonded to each other may have a substituent. Examples of the substituent include groups described in the section of the substituent T described below.

From the viewpoint of easily enhancing the absorbency of ultraviolet rays having a wavelength of approximately 400 nm while suppressing coloring,$R^4$ represents an alkyl group, an aryl group, an alkoxy group, or an aryloxy group and $R^5$ represents a hydrogen atom, an alkyl group, an aryl group, an alkoxy group, or an aryloxy group and more preferable that $R^4$ represents an alkyl group or an alkoxy group and $R^5$ represents a hydrogen atom, an alkyl group, or an alkoxy group.

Further, from the viewpoint of ease of synthesis, it is preferable that $R^4$ represents an alkyl group, an aryl group, an alkoxy group, or an aryloxy group and $R^5$ represents a hydrogen atom and more preferable that $R^4$ represents an alkyl group or an alkoxy group and $R^5$ represents a hydrogen atom.

Further, from the viewpoint of lengthening the wavelength of the absorption spectrum, $R^4$ and $R^5$ each independently represent preferably an alkyl group, an aryl group, an alkoxy group, or an aryloxy group and more

preferably an alkyl group or an alkoxy group. It is still more preferable that both $R^4$ and $R^5$ represent an alkyl group or both $R^4$ and $R^5$ represent an alkoxy group.

It is also preferable that $R^4$ and $R^5$ are bonded to each other to form a ring.

Here, in Formula (1), in a case where $R^3$ and $R^6$ represent an acyloxy group, at least one of $R^4$ or $R^5$ represents an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group, preferably an aryl group, an alkoxy group, an aryloxy group, or an acyloxy group, and more preferably an alkoxy group.

It is preferable that the compound represented by Formula (1) (compound (1)) is a compound represented by Formula (1a).

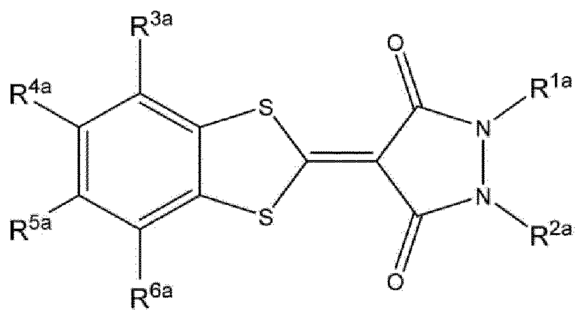

(1a)

In Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,
$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group.

The number of carbon atoms of the alkyl group represented by $R^{1a}$ and $R^{2a}$ is preferably in a range of 1 to 30, more preferably in a range of 1 to 20, still more preferably in a range of 1 to 15, particularly preferably in a range of 1 to 10, and most preferably in a range of 1 to 8. The alkyl group may be linear, branched, or cyclic and preferably linear or branched. The alkyl group may have a substituent. Examples of the substituent include groups described in the section of the substituent T described below.

$R^{1a}$ and $R^{2a}$ may be bonded to each other to form a ring. It is preferable that the ring formed by $R^{1a}$ and $R^{2a}$ being bonded to each other is a 5-membered or 6-membered ring. The ring formed by $R^{1a}$ and $R^{2a}$ being bonded to each other may have a substituent. Examples of the substituent include groups described in the section of the substituent T described below.

In Formula (1a), $R^{3a}$ and $R^{6a}$ each independently represent an alkoxy group or an acyloxy group. From the viewpoint of easily enhancing the absorbency of ultraviolet rays having a wavelength of approximately 400 nm while suppressing coloring, it is preferable that at least one of $R^{3a}$ or $R^{6a}$ represents an alkoxy group and more preferable that both $R^{3a}$ and $R^{6a}$ represent an alkoxy group.

The number of carbon atoms of the alkoxy group represented by $R^{3a}$ and $R^{6a}$ is preferably in a range of 1 to 30, more preferably in a range of 1 to 20, still more preferably in a range of 1 to 15, particularly preferably in a range of 1 to 10, and most preferably in a range of 1 to 8. The alkoxy group may be linear or branched. The alkoxy group may have a substituent. Examples of the substituent include groups described in the section of the substituent T described below.

The number of carbon atoms of the acyloxy group represented by $R^{3a}$ and $R^{6a}$ is preferably in a range of 2 to 30, more preferably in a range of 2 to 20, still more preferably in a range of 2 to 15, and particularly preferably in a range of 2 to 10. The acyloxy group may have a substituent. Examples of the substituent include groups described in the section of the substituent T described below.

In Formula (1a), $R^{4a}$ represents an alkyl group or an alkoxy group, and $R^{5a}$ represents a hydrogen atom, an alkyl group, or an alkoxy group.

The number of carbon atoms of the alkyl group represented by $R^{4a}$ and $R^{5a}$ is preferably in a range of 1 to 30, more preferably in a range of 1 to 20, still more preferably in a range of 1 to 15, particularly preferably in a range of 1 to 10, and

most preferably in a range of 1 to 8. The alkyl group may be linear, branched, or cyclic and preferably linear or branched. The alkyl group may have a substituent. Examples of the substituent include groups described in the section of the substituent T described below.

The number of carbon atoms of the alkoxy group represented by $R^{4a}$ and $R^{5a}$ is preferably in a range of 1 to 30, more preferably in a range of 1 to 20, still more preferably in a range of 1 to 15, particularly preferably in a range of 1 to 10, and most preferably in a range of 1 to 8. The alkoxy group may be linear or branched. The alkoxy group may have a substituent. Examples of the substituent include groups described in the section of the substituent T described below.

In Formula (1a), $R^{3a}$ and $R^{4a}$ may be bonded to each other to form a ring, $R^{4a}$ and $R^{5a}$ may be bonded to each other to form a ring, and $R^{5a}$ and $R^{6a}$ may be bonded to each other to form a ring. It is preferable that the ring formed by these groups being bonded to each other is a 5- or 6-membered ring. The ring formed by these groups being bonded to each other may have a substituent. Examples of the substituent include groups described in the section of the substituent T described below.

(Substituent T)

Examples of the substituent T include the following groups.

Examples thereof include a halogen atom (such as a chlorine atom, a bromine atom, or an iodine atom), an alkyl group [a linear, branched, or cyclic alkyl group, specific examples thereof include a linear or branched alkyl group (preferably a linear or branched alkyl group having 1 to 30 carbon atoms, and examples thereof include a methyl group, an ethyl group, an n-propyl group, an isopropyl group, a t-butyl group, an n-octyl group, an eicosyl group, a 2-chloroethyl group, a 2-cyanoethyl group, and a 2-ethylhexyl group), a cycloalkyl group (preferably a cycloalkyl group having 3 to 30 carbon atoms, and examples thereof include a cyclohexyl group, a cyclopentyl group, and a 4-n-dodecylcyclohexyl group), a bicycloalkyl group (preferably a bicycloalkyl group having 5 to 30 carbon atoms, that is, a monovalent group obtained by removing one hydrogen atom from a bicycloalkane having 5 to 30 carbon atoms, and examples thereof include a bicyclo [1,2,2]heptane-2-yl group and a bicyclo[2,2,2]octane-3-yl group), and those having a tricyclo structure with a plurality of ring structures, and alkyl groups in the substituents described below (for example, an alkyl group in an alkylthio group) are alkyl groups of such a concept], an alkenyl group [linear, branched, or cyclic alkenyl group, specific exmaples thereof include a linear or branched alkenyl group (preferably a linear or branched alkenyl group having 2 to 30 carbon atoms, and examples thereof include a vinyl group, an allyl group, a prenyl group, a geranyl group, and an oleyl group), a cycloalkenyl group (preferably a cycloalkenyl group having 3 to 30 carbon atoms, that is, a monovalent group obtained by removing one hydrogen atom from a cycloalkene having 3 to 30 carbon atoms, and examples thereof include a 2-cyclopentene-1-yl group and a 2-cyclohexene-1-yl group), and a bicycloalkenyl group (preferably a bicycloalkenyl group having 5 to 30 carbon atoms, that is, a monovalent group obtained by removing one hydrogen atom from a bicycloalkene having one double bond, and examples thereof include a bicyclo[2,2,1]hepto-2-en-1-yl group and a bicyclo[2,2,2]octo-2-en-4-yl group)], an alkynyl group (preferably a linear or branched alkynyl group having 2 to 30 carbon atoms, examples thereof include an ethynyl group and a propargyl group),

an aryl group (preferably an aryl group having 6 to 30 carbon atoms, examples thereof include a phenyl group, a p-tolyl group, a naphthyl group, an m-chlorophenyl group, an o-hexadecanoylaminophenyl group), a heterocyclic group (preferably a monovalent group obtained by removing one hydrogen atom from a 5- or 6-membered aromatic or non-aromatic heterocyclic compound and more preferably a 5- or 6-membered aromatic heterocyclic group having 3 to 30 carbon atoms, and examples thereof include a 2-furyl group, a 2-thienyl group, a 2-pyrimidinyl group, and a 2-benzothiazolyl group), a cyano group, a hydroxy group, a nitro group, a carboxyl group, an alkoxy group (preferably a linear or branched alkoxy group having 1 to 30 carbon atoms, and examples thereof include a methoxy group, an ethoxy group, an isopropoxy group, a t-butoxy group, an n-octyloxy group, and a 2-methoxyethoxy group), an aryloxy group (preferably an aryloxy group having 6 to 30 carbon atoms, and examples thereof include a phenoxy group, a 2-methylphenoxy group, a 4-t-butylphenoxy group, a 3-nitrophenoxy group, and a 2-tetradecanoylaminophenoxy group), a heterocyclic oxy group (preferably a heterocyclic oxy group having 2 to 30 carbon atoms, and examples thereof include a 1-phenyltetrazole-5-oxy group and a 2-tetrahydropyranyloxy group), an acyloxy group (preferably a formyloxy group, an alkylcarbonyloxy group having 2 to 30 carbon atoms, or an arylcarbonyloxy group having 6 to 30 carbon atoms, and examples thereof include a formyloxy group, an acetyloxy group, a pivaloyloxy group, a stearoyloxy group, a benzoyloxy group, and a p-methoxyphenylcarbonyloxy group),

a carbamoyloxy group (preferably a carbamoyloxy group having 1 to 30 carbon atoms, and examples thereof include a N,N-dimethylcarbamoyloxy group, a N,N-diethylcarbamoyloxy group, a morpholinocarbonyloxy group, a N,N-di-n-octylaminocarbonyloxy group, and a N-n-octylcarbamoyloxy group), an alkoxycarbonyloxy group (preferably an alkoxycarbonyloxy group having 2 to 30 carbon atoms, and examples thereof include a methoxycarbonyloxy group, an ethoxycarbonyloxy group, a t-butoxycarbonyloxy group, and an n-octylcarbonyloxy group), an aryloxycarbonyloxy group (preferably an aryloxycarbonyloxy group having 7 to 30 carbon atoms, and examples thereof include a

phenoxycarbonyloxy group, a p-methoxyphenoxycarbonyloxy group, and a p-n-hexadecyloxyphenoxycarbonyloxy group), an amino group (preferably an amino group, an alkylamino group having 1 to 30 carbon atoms, or an anilino group having 6 to 30 carbon atoms, and examples thereof include an amino group, a methylamino group, a dimethylamino group, an anilino group, a N-methyl-anilino group, and a diphenylamino group), an acylamino group (preferably a formylamino group, an alkylcarbonylamino group having 2 to 30 carbon atoms, or an arylcarbonylamino group having 6 to 30 carbon atoms, and examples thereof include a formylamino group, an acetylamino group, a pivaloylamino group, a lauroylamino group, a benzoylamino group, and a 3,4,5-tri-n-octyloxyphenylcarbonylamino group),

an aminocarbonylamino group (preferably an aminocarbonylamino group having 1 to 30 carbon atoms, and examples thereof include a carbamoylamino group, a N,N-dimethylaminocarbonylamino group, a N,N-diethylaminocarbonylamino group, and a morpholinocarbonylamino group), an alkoxycarbonylamino group (preferably an alkoxycarbonylamino group having 2 to 30 carbon atoms, and examples thereof include a methoxycarbonylamino group, an ethoxycarbonylamino group, a t-butoxycarbonylamino group, an n-octadecyloxycarbonylamino group, and a N-methyl-methoxycarbonylamino group), an aryloxycarbonylamino group (preferably an aryloxycarbonylamino group having 7 to 30 carbon atoms, and examples thereof include a phenoxycarbonylamino group, a p-chlorophenoxycarbonylamino group, and an m-n-octyloxyphenoxycarbonylamino group), a sulfamoylamino group (preferably a sulfamoylamino group having 0 to 30 carbon atoms, and examples thereof include a sulfamoylamino group, a N,N-dimethylaminosulfonylamino group, and a N-n-octylaminosulfonylamino group), an alkyl or aryl sulfonylamino group (preferably an alkyl sulfonylamino group having 1 to 30 carbon atoms or an aryl sulfonylamino group having 6 to 30 carbon atoms, and examples thereof include a methylsulfonylamino group, a butylsulfonylamino group, a phenylsulfonylamino group, a 2,3,5-trichlorophenylsulfonylamino group, and a p-methylphenylsulfonylamino group), a mercapto group, an alkylthio group (preferably an alkylthio group having 1 to 30 carbon atoms, and examples thereof include a methylthio group, an ethylthio group, and an n-hexadecylthio group), an arylthio group (preferably an arylthio group having 6 to 30 carbon atoms, and examples thereof include a phenylthio group, a p-chlorophenylthio group, and an m-methoxyphenylthio group), a heterocyclic thio group (preferably a heterocyclic thio group having 2 to 30 carbon atoms, and examples thereof include a 2-benzothiazolylthio group and a 1-phenyltetrazole-5-ylthio group),

a sulfamoyl group (preferably a sulfamoyl group having 0 to 30 carbon atoms, and examples thereof include a N-ethylsulfamoyl group, a N-(3-dodecyloxypropyl)sulfamoyl group, a N,N-dimethylsulfamoyl group, a N-acetylsulfamoyl group, a N-benzoylsulfamoyl group, a N-(N'-phenylcarbamoyl)sulfamoyl group), a sulfo group, an alkyl or aryl sulfinyl group (preferably an alkyl sulfinyl group having 1 to 30 carbon atoms or an aryl sulfinyl group having 6 to 30 carbon atoms, and examples thereof include a methylsulfinyl group, an ethylsulfinyl group, a phenylsulfinyl group, and a p-methylphenylsulfinyl group), an alkyl or aryl sulfonyl group (preferably an alkyl sulfonyl group having 1 to 30 carbon atoms or an aryl sulfonyl group having 6 to 30 carbon atoms, and examples thereof include a methylsulfonyl group, an ethylsulfonyl group, a phenylsulfonyl group, and a p-methylphenylsulfonyl group),

an acyl group (preferably a formyl group, an alkylcarbonyl group having 2 to 30 carbon atoms, an arylcarbonyl group having 7 to 30 carbon atoms, or a heterocyclic carbonyl group having 4 to 30 carbon atoms and bonded to a carbonyl group, and examples include an acetyl group, a pivaloyl group, a 2-chloroacetyl group, a stearoyl group, a benzoyl group, a p-n-octyloxyphenylcarbonyl group, a 2-pyridylcarbonyl group, and a 2-furylcarbonyl group), an aryloxycarbonyl group (preferably an aryloxycarbonyl group having 7 to 30 carbon atoms, and examples thereof include a phenoxycarbonyl group, an o-chlorophenoxycarbonyl group, an m-nitrophenoxycarbonyl group, and a p-t-butylphenoxycarbonyl group), an alkoxycarbonyl group (preferably an alkoxycarbonyl group having 2 to 30 carbon atoms, and examples thereof include a methoxycarbonyl group, an ethoxycarbonyl group, a t-butoxycarbonyl group, and an n-octadecyloxycarbonyl group), a carbamoyl group (preferably a carbamoyl group having 1 to 30 carbon atoms, and examples thereof include a carbamoyl group, a N-methylcarbamoyl group, a N,N-dimethylcarbamoyl group, a N,N-di-n-octylcarbamoyl group, and a N-(methylsulfonyl)carbamoyl group), an aryl or heterocyclic azo group (preferably an arylazo group having 6 to 30 carbon atoms or a heterocyclic azo group having 3 to 30 carbon atoms, and examples thereof include a phenylazo group, a p-chlorophenylazo group, and a 5-ethylthio-1,3,4-thiadiazole-2-ylazo group), an imide group (preferably a N-succinimide group or a N-phthalimide group), a phosphino group (preferably a phosphino group having 2 to 30 carbon atoms, and examples thereof include a dimethylphosphino group, a diphenylphosphino group, and a methylphenoxyphosphino group), a phosphinyl group (preferably a phosphinyl group having 2 to 30 carbon atoms, and examples thereof include a phosphinyl group, a dioctyloxyphosphinyl group, and a diethoxyphosphinyl group), a phosphinyloxy group (preferably a phosphinyloxy group having 2 to 30 carbon atoms, and examples thereof include a diphenoxyphosphinyloxy group and a dioctyloxyphosphinyloxy group), and a phosphinylamino group (preferably a phosphinylamino group having 2 to 30 carbon atoms, and examples thereof include a dimethoxyphosphinylamino group and a dimethylaminophosphinylamino group).

Among the groups described above, one or more hydrogen atoms of groups having hydrogen atoms may be substituted with the above-described substituents T. Examples of such substituents include an alkylcarbonylaminosulfonyl group, an arylcarbonylaminosulfonyl group, an alkylsulfonylaminocarbonyl group, and an arylsulfonylaminocarbonyl group. Specific examples include a methylsulfonylaminocarbonyl group, a p-methylphenylsulfonylaminocarbonyl group, an acetylaminosulfonyl group, and a benzoylaminosulfonyl group.

Specific examples of the compound (1) include compounds having the following structures. In the structural formulae shown below, Me represents a methyl group, Et represents an ethyl group, Bu represents a butyl group, tBu represents a tert-butyl group, Pr represents a propyl group, and Ph represents a phenyl group.

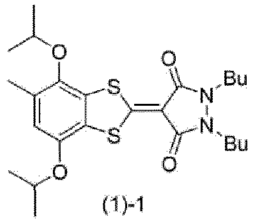
(1)-1

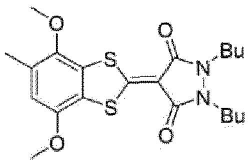
(1)-2

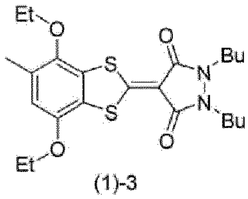
(1)-3

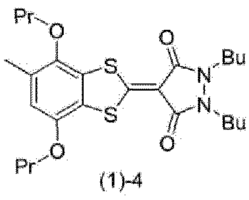
(1)-4

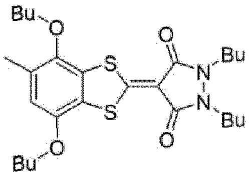
(1)-5

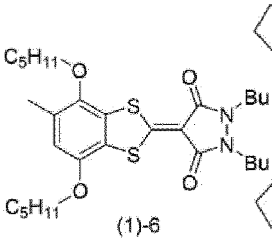
(1)-6

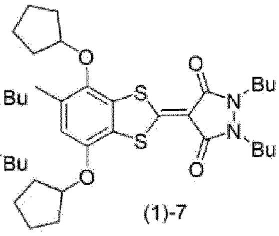
(1)-7

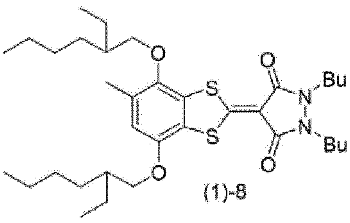
(1)-8

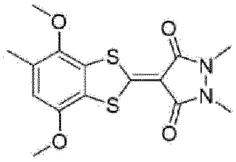
(1)-9

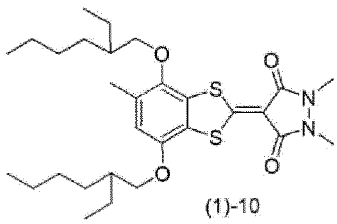
(1)-10

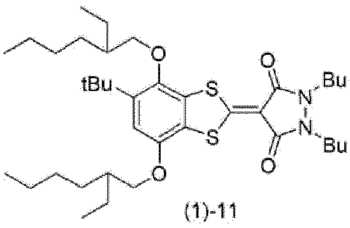
(1)-11

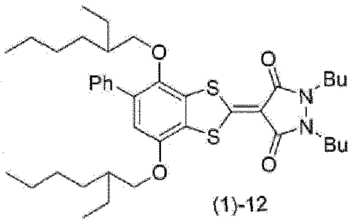
(1)-12

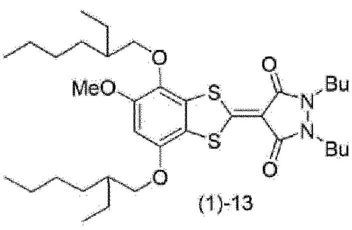
(1)-13

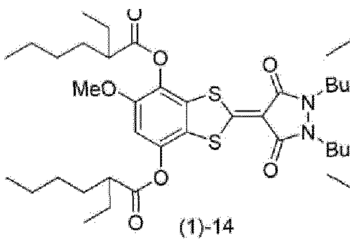
(1)-14

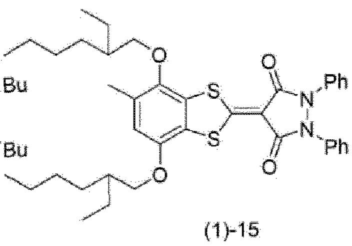
(1)-15

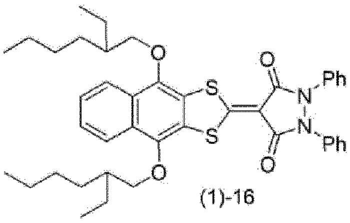

(1)-16

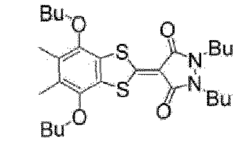

(1)-17

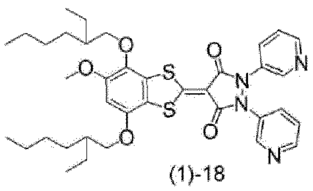

(1)-18

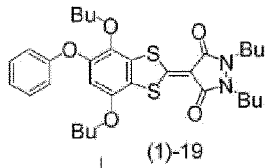

(1)-19

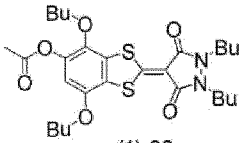

(1)-20

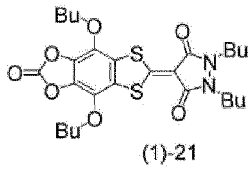

(1)-21

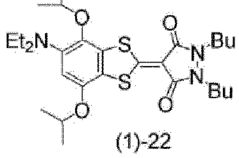

(1)-22

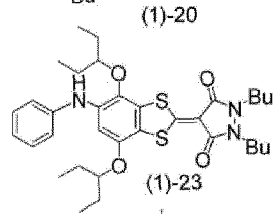

(1)-23

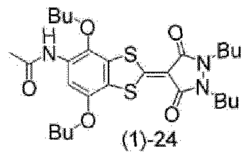

(1)-24

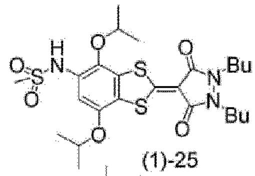

(1)-25

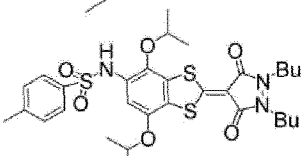

(1)-26

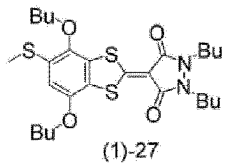

(1)-27

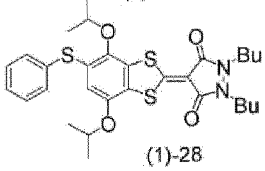

(1)-28

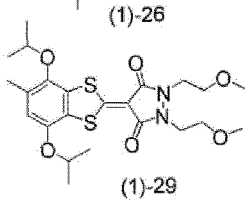

(1)-29

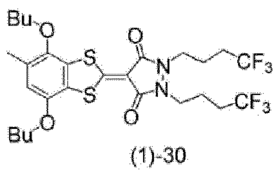

(1)-30

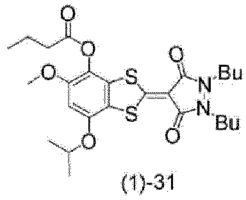

(1)-31

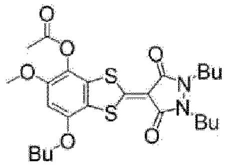

(1)-32

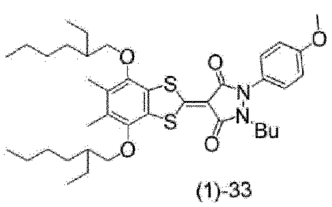

(1)-33

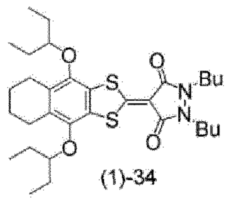

(1)-34

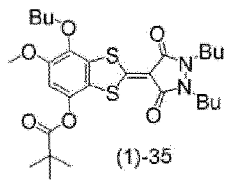

(1)-35

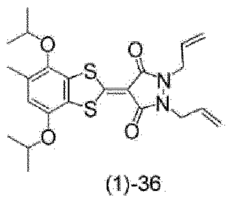

(1)-36

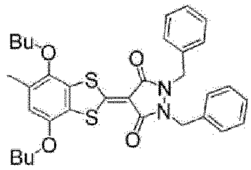

(1)-37

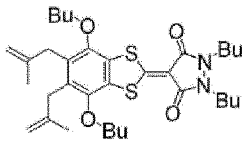

(1)-38

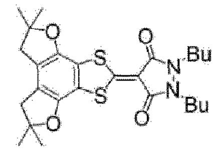

(1)-39

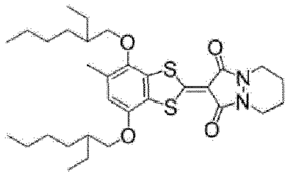

(1)-40

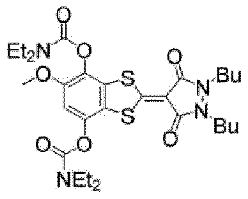
(1)-41

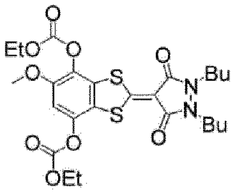
(1)-42

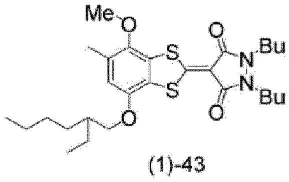
(1)-43

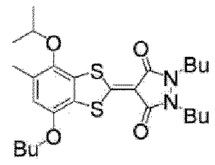
(1)-44

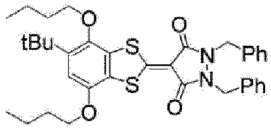
(1)-45

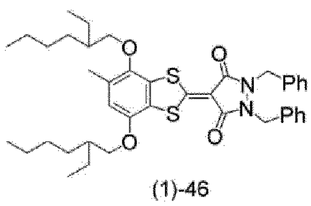
(1)-46

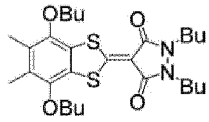
(1)-47

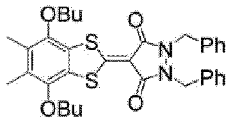
(1)-48

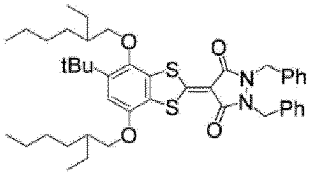
(1)-49

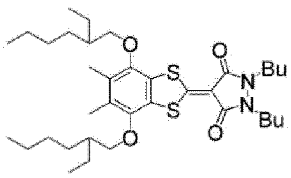
(1)-50

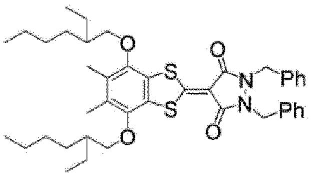
(1)-51

The compound (1) is preferably used as an ultraviolet absorbing agent. The maximum absorption wavelength of the compound (1) is present preferably in a wavelength range of 381 to 420 nm and more preferably in a wavelength range of 381 to 400 nm.

The molar absorption coefficient $\varepsilon_{405}$ of the compound (1) calculated by the following formula at a wavelength of 405 nm is preferably 500 or greater, more preferably 1000 or greater, still more preferably 2000 or greater, and particularly preferably 3000 or greater.

$$\varepsilon_{405} = \varepsilon_{max} \times (A_{405}/A_{max})$$

$\varepsilon_{405}$ denotes the molar absorption coefficient of the compound (1) at a wavelength of 405 nm, $\varepsilon_{max}$ denotes the molar absorption coefficient of the compound (1) at the maximum absorption wavelength, $A_{405}$ denotes the absorbance of the compound (1) at a wavelength of 405 nm, and $A_{max}$ denotes the absorbance of the compound (1) at the maximum absorption wavelength.

In the spectral absorption spectrum of the compound (1) measured in ethyl acetate, the ratio ($A_{430}/A_{405}$) of the absorbance $A_{430}$ at a wavelength of 430 nm to the absorbance $A_{405}$ at a wavelength of 405 nm is preferably less than 0.13 and more preferably 0.1 or less. The lower limit of the ratio is not particularly limited, but can be set to 0 or greater. Those having such an absorbance ratio are excellent in light transmittance in a visible region near the ultraviolet region despite high absorption near the wavelength of 405 nm, and thus have excellent visible transparency while having excellent absorbency of ultraviolet rays on a longer wavelength side. Further, in a case where the ultraviolet absorption region of a compound is intended to be shifted to a longer wavelength side, the light transmittance in a visible region (particularly, the light transmittance in a visible region near the ultraviolet region) tends to decrease, but the compound (1) of the present invention exhibits a technically excellent effect of excellent absorbency of ultraviolet rays on a longer wavelength side while maintaining a high level of light transmittance in a visible region.

The compound (1) can be synthesized with reference to the synthetic methods described in JP2016-081035A, JP5376885B, and the like.

The content of the compound (1) in the total solid content of the resin composition is preferably in a range of 0.01% to 50% by mass. The lower limit thereof is preferably 0.05% by mass or greater and more preferably 0.1% by mass or greater. The upper limit thereof is preferably 40% by mass or less, more preferably 30% by mass or less, and more preferably 20% by mass or less.

The content of the compound (1) is preferably in a range of 0.01 to 50 parts by mass with respect to 100 parts by mass of the resin. The lower limit thereof is preferably 0.05 parts by mass or greater and more preferably 0.1 parts by mass

or greater. The upper limit thereof is preferably 40 parts by mass or less, more preferably 30 parts by mass or less, and more preferably 20 parts by mass or less.

The resin composition may contain only one or two or more kinds of the compounds (1). In a case where the resin composition contains two or more kinds of the compounds (1), it is preferable that the total amount thereof is in the above-described range.

<<Compound represented by Formula (2) (compound (2))>>

It is preferable that the resin composition according to the embodiment of the present invention further contains a compound represented by Formula (2) (hereinafter, also referred to as a compound (2)). According to this aspect, the storage stability of the resin composition can be further improved.

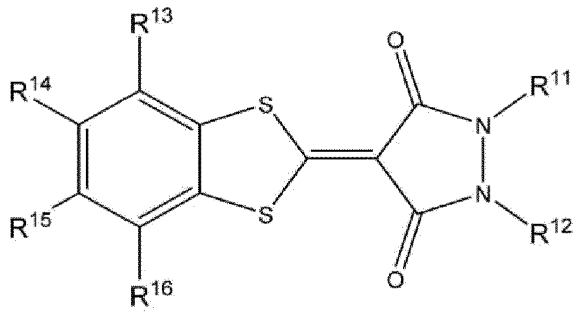

(2)

In Formula (2), $R^{11}$ and $R^{12}$ each independently represent an alkyl group, an aryl group, or a heterocyclic group, $R^{13}$ and $R^{16}$ each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group, $R^{14}$ represents a halogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. $R^{15}$ represents a hydrogen atom, a halogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group, $R^{11}$ and $R^{12}$ may be bonded to each other to form a ring, $R^{13}$ and $R^{14}$ may be bonded to each other to form a ring, $R^{14}$ and $R^{15}$ may be bonded to each other to form a ring, and $R^{15}$ and $R^{16}$ may be bonded to each other to form a ring, where at least one of $R^{13}$ or $R^{16}$ represents a hydroxy group.

The alkyl group, the aryl group, and the heterocyclic group represented by $R^{11}$ and $R^{12}$ in Formula (2) each have the same definition as that for the alkyl group, the aryl group, and the heterocyclic group represented by $R^1$ and $R^2$ in Formula (1), and the preferable ranges are the same as described above. It is preferable that $R^{11}$ and $R^{12}$ in Formula (2) represent an alkyl group or an aryl group.

$R^{13}$ and $R^{16}$ in Formula (2) each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group, preferably a hydroxy group, an alkoxy group, or an acyloxy group, more preferably a hydroxy group or an alkoxy group, and still more preferably a hydroxy group. Here, at least one of $R^{13}$ or $R^{16}$ represents a hydroxy group.

The number of carbon atoms of the alkoxy group represented by $R^{13}$ and $R^{16}$ is preferably in a range of 1 to 30, more preferably in a range of 1 to 20, still more preferably in a range of 1 to 15, particularly preferably in a range of 1 to 10, and most preferably in a range of 1 to 8. The alkoxy group may be linear or branched. The alkoxy group may have a substituent. Examples of the substituent include the groups described in the section of the substituent T above.

The number of carbon atoms of the aryloxy group represented by $R^{13}$ and $R^{16}$ is preferably in a range of 6 to 40, more preferably in a range of 6 to 30, still more preferably in a range of 6 to 20, particularly preferably in a range of 6 to 15, and most preferably in a range of 6 to 12. The aryloxy group may have a substituent. Examples of the substituent include the groups described in the section of the substituent T above.

The number of carbon atoms of the acyloxy group represented by $R^{13}$ and $R^{16}$ is preferably in a range of 2 to 30, more preferably in a range of 2 to 20, still more preferably in a range of 2 to 15, and particularly preferably in a range of 2 to 10. The acyloxy group may have a substituent. Examples of the substituent include groups described in the section of the substituent T described below.

The number of carbon atoms of the carbamoyloxy group represented by $R^{13}$ and $R^{16}$ is preferably in a range of 2 to 30, more preferably in a range of 2 to 20, still more preferably in a range of 2 to 15, particularly preferably in a range of 2 to 10, and most preferably in a range of 2 to 8. The carbamoyloxy group may be linear or branched. The carbamoyloxy group

may have a substituent. Examples of the substituent include the groups described in the section of the substituent T above.

The number of carbon atoms of the alkoxycarbonyloxy group represented by $R^{13}$ and $R^{16}$ is preferably in a range of 2 to 30, more preferably in a range of 2 to 20, still more preferably in a range of 2 to 15, particularly preferably in a range of 2 to 10, and most preferably in a range of 2 to 8. The alkoxycarbonyloxy group may be linear or branched. The alkoxycarbonyloxy group may have a substituent. Examples of the substituent include the groups described in the section of the substituent T above.

The number of carbon atoms of the aryloxycarbonyloxy group represented by $R^{13}$ and $R^{16}$ is preferably in a range of 7 to 40, more preferably in a range of 7 to 30, still more preferably in a range of 7 to 20, particularly preferably in a range of 7 to 15, and most preferably in a range of 7 to 12. The aryloxycarbonyloxy group may have a substituent. Examples of the substituent include the groups described in the section of the substituent T above.

The number of carbon atoms of the sulfinyloxy group represented by $R^{13}$ and $R^{16}$ is preferably in a range of 1 to 30, more preferably in a range of 1 to 20, and still more preferably in a range of 1 to 15. The sulfinyloxy group may have a substituent. Examples of the substituent include the groups described in the section of the substituent T above.

The number of carbon atoms of the sulfonyloxy group represented by $R^{13}$ and $R^{16}$ is preferably in a range of 1 to 30, more preferably in a range of 1 to 20, and still more preferably in a range of 1 to 15. The sulfonyloxy group may have a substituent. Examples of the substituent include the groups described in the section of the substituent T above.

$R^{14}$ in Formula (2) represents an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group, and $R^{15}$ represents a hydrogen atom, a halogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group. $R^{14}$ and $R^{15}$ in Formula (2) each have the same definition as that for $R^4$ and $R^5$ in Formula (1), and the preferable ranges are the same as described above.

It is preferable that $R^{14}$ represents an alkyl group, an aryl group, an alkoxy group, or an aryloxy group and $R^{15}$ represents a hydrogen atom, an alkyl group, an aryl group, an alkoxy group, or an aryloxy group and more preferable that $R^{14}$ represents an alkyl group or an alkoxy group and $R^{15}$ represents a hydrogen atom, an alkyl group, or an alkoxy group.

As one preferable aspect, an aspect in which $R^{14}$ represents an alkyl group, an aryl group, an alkoxy group, or an aryloxy group, and $R^{15}$ represents a hydrogen atom is exemplified. In this aspect, it is preferable that $R^{14}$ represents an alkyl group or an alkoxy group and $R^{15}$ represents a hydrogen atom.

Further, as another preferable aspect, an aspect in which $R^{14}$ and $R^{15}$ each independently represent an alkyl group, an aryl group, an alkoxy group, or an aryloxy group is exemplified. In this aspect, it is preferable that $R^{14}$ and $R^{15}$ each independently represent an alkyl group or an alkoxy group and more preferable that both $R^{14}$ and $R^{15}$ represent an alkyl group or both $R^{14}$ and $R^{15}$ represent an alkoxy group.

Further, as still another preferable aspect, an aspect in which $R^{14}$ and $R^{15}$ are bonded to each other to form a ring is exemplified.

Specific examples of the compound (2) include compounds having the following structures. In the structural formulae shown below, Me represents a methyl group, Et represents an ethyl group, tBu represents a tert-butyl group, Pr represents a propyl group, Bu represents a butyl group, and Ph represents a phenyl group.

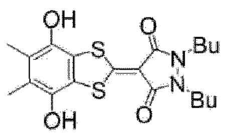

(2)-1

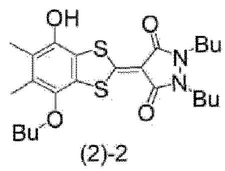

(2)-2

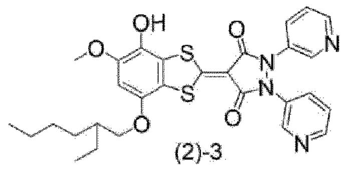

(2)-3

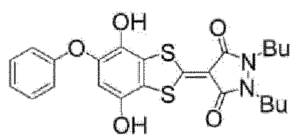

(2)-4

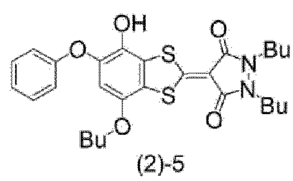

(2)-5

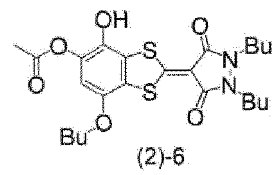

(2)-6

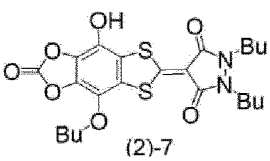
(2)-7

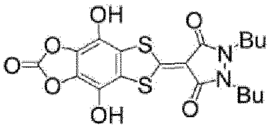
(2)-8

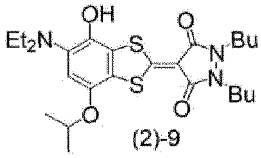
(2)-9

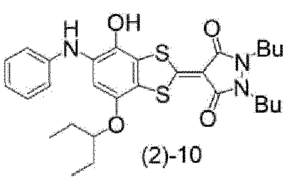
(2)-10

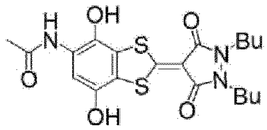
(2)-11

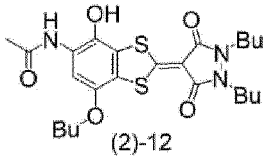
(2)-12

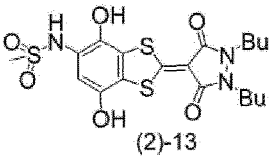
(2)-13

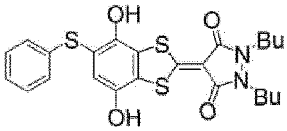
(2)-14

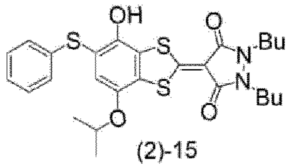
(2)-15

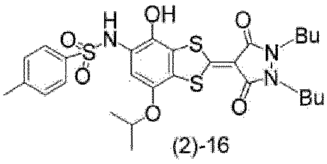
(2)-16

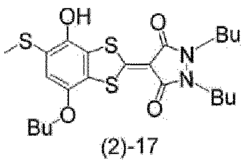
(2)-17

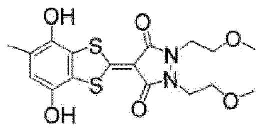
(2)-18

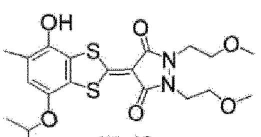
(2)-19

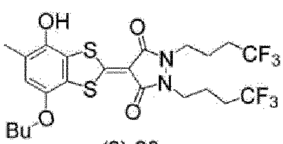
(2)-20

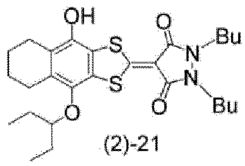
(2)-21

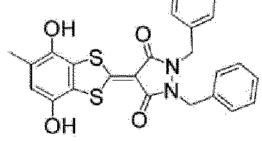
(2)-22

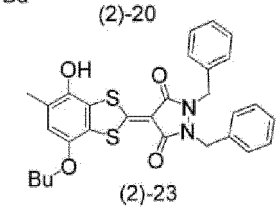
(2)-23

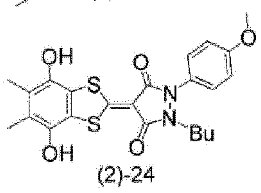
(2)-24

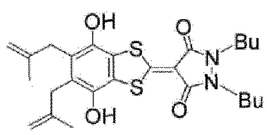
(2)-25

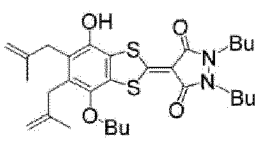
(2)-26

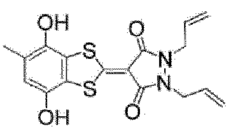
(2)-27

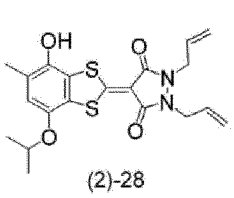
(2)-28

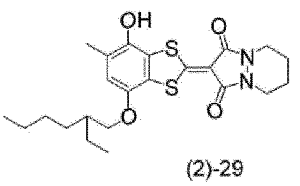
(2)-29

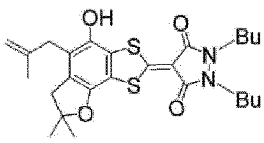
(2)-30

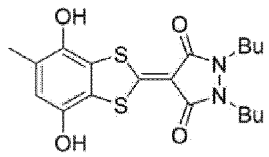

(2)-31

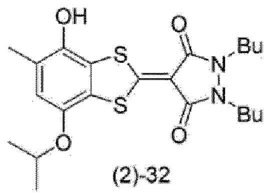

(2)-32

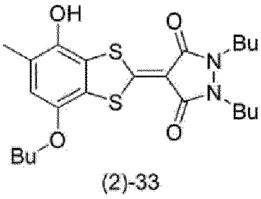

(2)-33

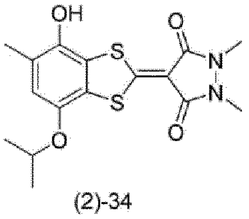

(2)-34

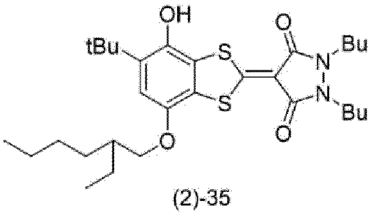

(2)-35

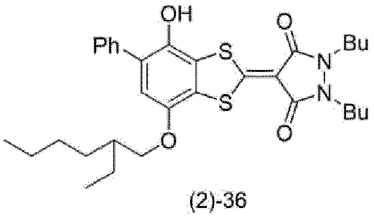

(2)-36

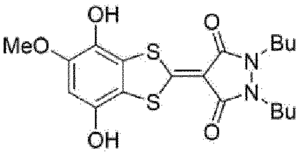

(2)-37

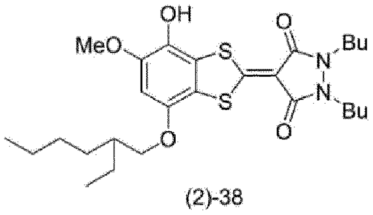

(2)-38

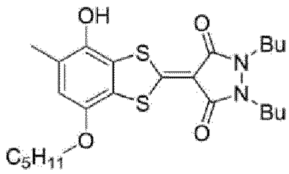

(2)-39

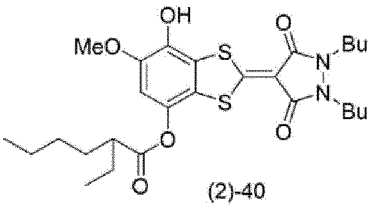

(2)-40

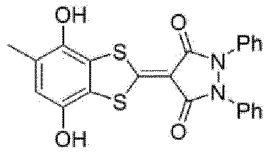

(2)-41

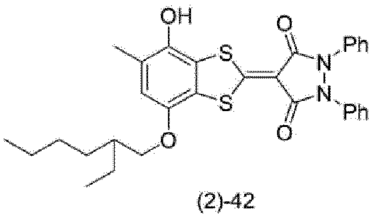

(2)-42

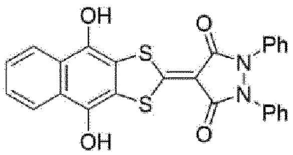

(2)-43

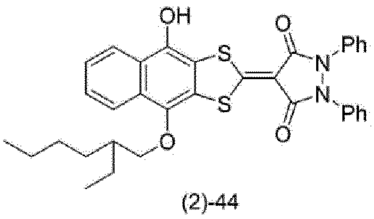

(2)-44

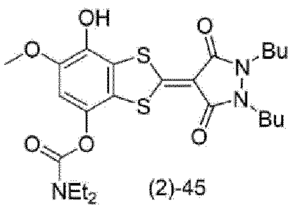

(2)-45

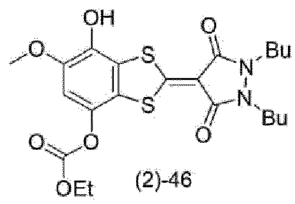

(2)-46

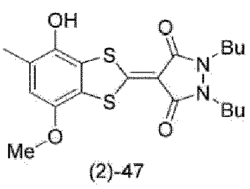

(2)-47

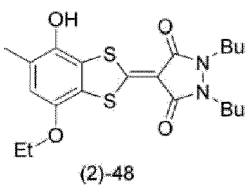

(2)-48

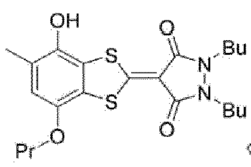

(2)-49

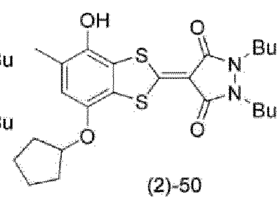

(2)-50

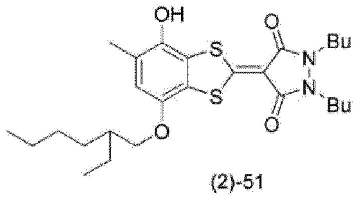
(2)-51

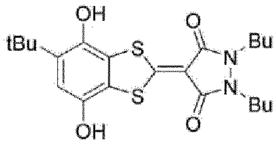
(2)-52

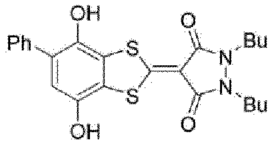
(2)-53

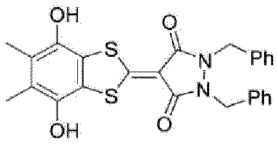
(2)-54

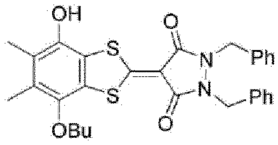
(2)-55

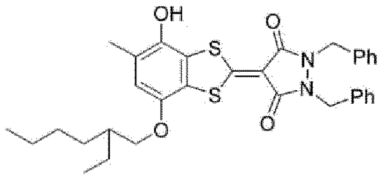
(2)-56

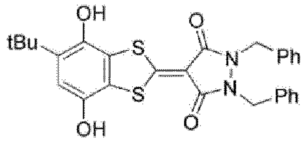
(2)-57

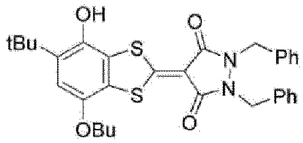
(2)-58

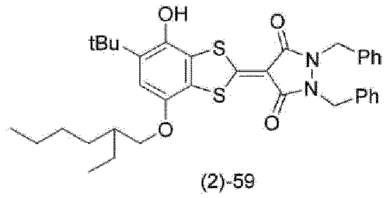
(2)-59

The compound (2) can be synthesized with reference to the methods described in JP5376885B and WO2019/142539A.

The compound (2) is preferably used as an ultraviolet absorbing agent. The maximum absorption wavelength of the compound (2) is present preferably in a wavelength range of 381 to 420 nm and more preferably in a wavelength range of 381 to 400 nm.

In a case where the resin composition contains the compound (2), the content of the compound (2) is preferably 5 parts by mass or less, more preferably 3 parts by mass or less, and still more preferably 1 part by mass or less with respect to 100 parts by mass of the compound (1). The lower limit thereof is preferably 0.1 part by mass or greater.

Further, the total content of the compound (1) and the compound (2) in the total solid content of the resin composition is preferably in a range of 0.01% to 50% by mass. The lower limit thereof is preferably 0.05% by mass or greater and more preferably 0.1% by mass or greater. The upper limit thereof is preferably 40% by mass or less, more preferably 30% by mass or less, and more preferably 20% by mass or less.

The total content of the compound (1) and the compound (2) is preferably in a range of 0.01 to 50 parts by mass with respect to 100 parts by mass of the resin. The lower limit thereof is preferably 0.05 parts by mass or greater and more preferably 0.1 parts by mass or greater. The upper limit thereof is preferably 40 parts by mass or less, more preferably 30 parts by mass or less, and more preferably 20 parts by mass or less.

The resin composition may contain only one or two or more kinds of the compounds (2). In a case where the resin composition contains two or more kinds of the compounds (2), it is preferable that the total amount thereof is in the above-described range.

It is also preferable that the resin composition does not substantially contain the compound (2). According to this aspect, more excellent light resistance is likely to be obtained. In the present specification, the expression "the resin composition does not substantially contain the compound (2)" denotes that the content of the compound (2) in the total solid content of the resin composition is 0.001% by mass or less, preferably 0.0001% by mass or less, and more preferably zero.

<<Other ultraviolet absorbing agents>>

The resin composition according to the embodiment of the present invention can contain other ultraviolet absorbing agents in addition to the above-described compound (1) (hereinafter, also referred to as other ultraviolet absorbing agents). According to this aspect, a cured substance capable of blocking light having a wavelength in the ultraviolet region over a wide range can be formed.

The maximum absorption wavelength of other ultraviolet absorbing agents is present preferably in a wavelength range of 390 nm or less and more preferably in a wavelength range of 380 nm or less.

Examples of other ultraviolet absorbing agents include a benzotriazole-based ultraviolet absorbing agent, a

benzophenone-based ultraviolet absorbing agent, a salicylic acid-based ultraviolet absorbing agent, an acrylate-based ultraviolet absorbing agent, a benzodithiol-based ultraviolet absorbing agent, and a triazine-based ultraviolet absorbing agents. Among these, a benzotriazole-based ultraviolet absorbing agent, a benzophenone-based ultraviolet absorbing agent, and a triazine-based ultraviolet absorbing agent are preferable, and a benzotriazole-based ultraviolet absorbing agent and a triazine-based ultraviolet absorbing agents are more preferable. Specific examples of the benzotriazole-based ultraviolet absorbing agent, the benzophenone-based ultraviolet absorbing agent, the salicylic acid-based ultraviolet absorbing agent, the acrylate-based ultraviolet absorbing agent, and the triazine-based ultraviolet absorbing agent include compounds described in paragraphs 0065 to 0070 of JP2009-263616A and compounds described in paragraph 0065 of WO2017/122503A. Further, compounds having the following structures can also be preferably used as other ultraviolet absorbing agents.

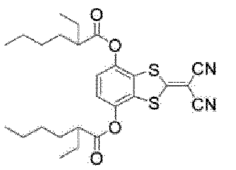
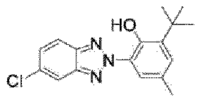
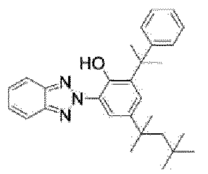
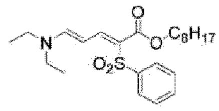

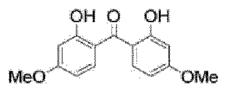
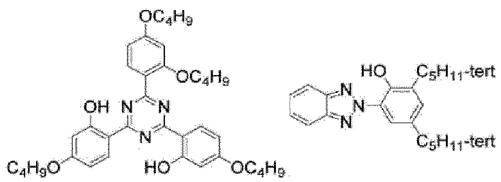
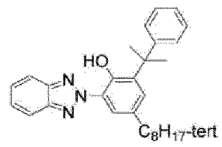

In a case where the resin composition contains other ultraviolet absorbing agents, the content of the other ultraviolet absorbing agents in the total solid content of the resin composition is preferably in a range of 0.01% to 50% by mass. The lower limit thereof is preferably 0.05% by mass or greater and more preferably 0.1% by mass or greater. The upper limit thereof is preferably 40% by mass or less, more preferably 30% by mass or less, and more preferably 20% by mass or less.

Further, the total content of the compound (1), the compound (2), and other ultraviolet absorbing agents in the total solid content of the resin composition is preferably in a range of 0.01% to 50% by mass. The lower limit thereof is preferably 0.05% by mass or greater and more preferably 0.1% by mass or greater. The upper limit thereof is preferably 40% by mass or less, more preferably 30% by mass or less, and more preferably 20% by mass or less.

The resin composition may contain only one or two or more kinds of other ultraviolet absorbing agents. In a case where the resin composition contains two or more kinds of other ultraviolet absorbing agents, it is preferable that the total amount thereof is in the above-described range.

<<Resin>>

The resin composition according to the embodiment of the present invention contains a resin. Examples of the kind of resin include a (meth)acrylic resin, a polyester resin, a polycarbonate resin, a vinyl polymer [such as a polydiene resin, a polyalkene resin, a polystyrene resin, a polyvinyl ether resin, a polyvinyl alcohol resin, a polyvinyl ketone resin, a polyfluorovinyl resin, or a polyvinyl bromide resin], a polythioether resin, a polyphenylene resin, a polyurethane resin, a polythiourethane resin, a polysulfonate resin, a nitroso polymer resin, a polysiloxane resin, a polysulfide resin, a polythioester resin, a polysulfone resin, a polysulfonamide resin, a polyamide resin, a polyimine resin, a polyurea resin, a polyphosphazene resin, a polysilane resin, a polysilazane resin, a polyfuran resin, a polybenzoxazole resin, a polyoxadiazole resin, a polybenzothiazinophenothiazine resin, a polybenzothiazole resin, a polypyrazinoquinoxaline resin, a polypyromellitimide resin, a polyquinoxaline resin, a polybenzoimidazoline resin, a polyoxoisoindoline resin, a polydioxoisoindoline resin, a polytriazine resin, a polypyridazine resin, a polypiperazine resin, a polypyridine resin, a polypiperidine resin, a polytriazole resin, a polypyrazole resin, a polypyrrolidine resin, a polycarborane resin, a polyoxabicyclononane resin, a polydibenzofuran resin, a polyphthalide resin, a polyacetal resin, a polyimide resin, an olefin resin, a cyclic olefin resin, an epoxy resin, and a cellulose acylate resin. The kind of resin can be appropriately selected according to the intended use and the purpose. For the details, the description in paragraphs 0075 to 0097 of JP2009-263616A can be referred to.

Further, a resin containing a polymerizable group can also be used as the resin. Examples of commercially available products of the resin containing a polymerizable group include DIANAL BR Series (polymethyl methacrylate (PMMA), for example, DIANAL BR-80, BR-83, and BR-87; manufactured by Mitsubishi Chemical Corporation); Photomer

6173 (COOH-containing polyurethane acrylic oligomer, manufactured by Diamond Shamrock Co., Ltd.); VISCOAT R-264, KS Resist 106 (both manufactured by Osaka Organic Chemical Industry Ltd.); CYCLOMER P Series (for example, ACA230AA), PLACCEL CF200 Series (all manufactured by Dycel Corporation); Ebecryl 3800 (manufactured by Dycel UCB Co.); and Acrylic-RD-F8 (manufactured by Nippon Shokubai Co., Ltd.).

From the viewpoint of satisfactory compatibility with the compound (1) and easily obtaining a cured substance with suppressed surface unevenness, at least one selected from a (meth)acrylic resin, a polystyrene resin, a polyester resin, a polyurethane resin, a polythiourethane resin, a polyimide resin, an epoxy resin, a polycarbonate resin, and a cellulose acylate resin is preferable, and at least one selected from a (meth)acrylic resin, a polystyrene resin, a polyester resin, a polyurethane resin, a polythiourethane resin, a polycarbonate resin, and a cellulose acylate resin is more preferable as the resin.

As the cellulose acylate resin, the cellulose acylate described in paragraphs 0016 to 0021 of JP2012-215689A is preferably used. As the polyester resin, a commercially available product such as the VYLON Series (for example, VYLON 500, manufactured by Toyobo Co., Ltd.) can also be used. As a commercially available product of the (meth)acrylic resin, SK Dyne Series (for example, SK Dyne-SF2147, manufactured by Soken Chemical & Engineering Co., Ltd.) can also be used.

It is preferable to use the polystyrene-based resin described below as the polystyrene resin. Here, the styrene-based resin denotes a resin in which the monomer unit having the highest ratio among the monomer units constituting the resin is the monomer unit derived from the styrene monomer. For example, in a case of a resin consisting of two components, the styrene-based resin denotes a resin containing 50% by mass or greater of a monomer unit derived from a styrene-based monomer. Here, the styrene-based monomer denotes a monomer having a styrene skeleton in the structure. The styrene-based resin contains preferably 70% by mass or greater of the monomer units derived from the styrene-based monomer and more preferably 85% by mass or greater of the monomer unit derived from the styrene-based monomer.

Specific examples of the styrene-based monomer include a homopolymer of styrene or a derivative thereof and a binary or higher copolymer of styrene or a derivative thereof and other copolymerizable monomers. Here, the styrene derivative is a compound in which another group is bonded to styrene, and examples thereof include alkylstyrene such as o-methylstyrene, m-methylstyrene, p-methylstyrene, 2,4-dimethylstyrene, o-ethylstyrene, or p-ethylstyrene, and substituted styrene in which a hydroxyl group, an alkoxy group, a carboxyl group, or halogen is introduced to a benzene nucleus of styrene such as hydroxystyrene, tert-butoxystyrene, vinyl benzoic acid, o-chlorostyrene, or p-chlorostyrene.

Further, examples of the styrene-based resin also include those obtained by copolymerizing other polymer components with styrene-based monomer components. Examples of the copolymerizable monomer include an unsaturated carboxylic acid alkyl ester monomer, for example, alkyl methacrylate such as methyl methacrylate, cyclohexyl methacrylate, methyl phenyl methacrylate, or isopropyl methacrylate and alkyl acrylate such as methyl acrylate, ethyl acrylate, butyl acrylate, 2-ethylhexyl acrylate, or cyclohexyl acrylate; an unsaturated carboxylic acid monomer such as methacrylic acid, acrylic acid, itaconic acid, maleic acid, fumaric acid, or cinnamic acid; an unsaturated dicarboxylic acid anhydride monomer which is an anhydride of maleic acid anhydride, itaconic acid, ethyl maleic acid, methyl itaconic acid, or chloromaleic acid; an unsaturated nitrile monomer such as acrylonitrile or methacrylonitrile; and a conjugated diene such as 1,3-butadiene, 2-methyl-1,3-butadiene (isoprene), 2,3-dimethyl-1,3-butadiene, 1,3-pentadiene, or 1,3-hexadiene. Further, two or more kinds of these monomers can be copolymerized. A commercially available product can also be used as the styrene-based resin. Examples of the commercially available product include AS-70 (acrylonitrile/styrene copolymer resin, manufactured by Nippon Steel & Sumikin Chemical Co., Ltd.) and SMA2000P (styrene/maleic acid copolymer, manufactured by Kawahara Petrochemical Co., Ltd.).

As the styrene-based resin, a plurality of resins with different compositions, different molecular weights can be used in combination.

The styrene-based resin can be obtained by a known anion, bulk, suspension, emulsification, or solution polymerization method. Further, in the polystyrene-based resin, an unsaturated double bond of a conjugated diene or a benzene ring of a styrene-based monomer may be hydrogenated. The hydrogenation rate can be measured by a nuclear magnetic resonance device (NMR).

The weight-average molecular weight (Mw) of the resin is preferably in a range of 3000 to 2000000. The upper limit thereof is preferably 1000000 or less and more preferably 500000 or less. The lower limit thereof is preferably 4000 or greater and more preferably 5000 or greater.

The total light transmittance of the resin is preferably 80% or greater, more preferably 85% or greater, and still more preferably 90% or greater. In the present specification, the total light transmittance of the resin is a value measured based on the contents described in "The Fourth Series of Experimental Chemistry 29 Polymer Material" (Maruzen, 1992), pp. 225 to 232, edited by the Chemical Society of Japan.

The content of the resin in the total solid content of the resin composition is preferably in a range of 1% to 99.9% by mass. The lower limit thereof is preferably 70% by mass or greater. The upper limit thereof is preferably 95% by mass or less and more preferably 90% by mass or less. The resin composition may contain only one or two or more kinds of resins.

In a case where the resin composition contains two or more kinds of resins, it is preferable that the total amount thereof is in the above-described range.

<<Curable compound>>

The resin composition can contain a curable compound. Examples of the curable compound include a polymerizable compound and a compound having an -O-Si-O- structure.

As the polymerizable compound, a compound that can be polymerized and cured by applying energy can be used without limitation. Examples of the polymerizable compound include a compound containing an ethylenically unsaturated bond-containing group, a compound containing an epoxy group, and a compound containing a methylol group. Among these, a compound containing an ethylenically unsaturated bond-containing group is preferable, and a compound containing two or more of ethylenically unsaturated bond-containing groups is more preferable. Examples of the ethylenically unsaturated bond-containing group include a vinyl group, an allyl group, and a (meth)acryloyl group.

The polymerizable compound may be, for example, any one of a monomer, a prepolymer (that is, a dimer, a trimer, or an oligomer), and a mixture thereof, and a (co)polymer of a compound selected from the monomer and the prepolymer.

Examples of the compound containing an ethylenically unsaturated bond-containing group used as a polymerizable compound include an unsaturated carboxylic acid (for example, acrylic acid, methacrylic acid, itaconic acid, crotonic acid, isocrotonic acid, or maleic acid), an ester of an unsaturated carboxylic acid, an amide of an unsaturated carboxylic acid, and a (co)polymer of the unsaturated carboxylic acid, the ester thereof, or the amide thereof. Among these, esters of an unsaturated carboxylic acid and an aliphatic polyhydric alcohol, amides of an unsaturated carboxylic acid and an aliphatic polyvalent amine, and homopolymers or copolymers thereof are preferable.

Further, examples of the compound containing an ethylenically unsaturated bond-containing group used as a polymerizable compound include an addition reactant of an unsaturated carboxylic acid ester or an unsaturated carboxylic acid amide containing a nucleophilic substituent (such as a hydroxy group, an amino group, or a mercapto group) and a monofunctional or polyfunctional isocyanate compound or an epoxy compound; a dehydration condensation reactant of an unsaturated carboxylic acid ester or an unsaturated carboxylic acid amide containing a nucleophilic substituent and a monofunctional or polyfunctional carboxylic acid; an addition reactant of an unsaturated carboxylic acid ester or an unsaturated carboxylic acid amide of a electrophilic substituent (such as an isocyanate group or an epoxy group), a monofunctional or polyfunctional alcohol, and an amine or thiol; and a substitution reactant of an unsaturated carboxylic acid ester or an unsaturated carboxylic acid amide containing a releasable substituent (such as a halogen group or a tosyloxy group), a monofunctional or polyfunctional alcohol, and an amine or thiol. Further, a compound obtained by substituting the above-described unsaturated carboxylic acid with an unsaturated phosphonic acid, or styrene, vinyl ether can also be exemplified.

Further, a plurality of compounds with different numbers of functional groups or a plurality of compounds with different kinds of polymerizable groups (for example, acrylic acid ester, methacrylic acid ester, a styrene-based compound, or a vinyl ether-based compound) may be used in combination with the compound containing an ethylenically unsaturated bond-containing group used as a polymerizable compound.

Examples of commercially available products of the compound containing an ethylenically unsaturated bond-containing group include KYARAD (registered trademark) Series (for example, PET-30, TPA-330, manufactured by Nippon Kayaku Co., Ltd.), POLYVEST (registered trademark) 110M (manufactured by Evonik Industries AG), and a polyfunctional (meth)acrylate compound of NK Ester Series (for example, NK Ester A-9300, manufactured by Shin Nakamura Chemical Industry Co., Ltd.).

Examples of the compound containing an epoxy group (hereinafter, also referred to as an epoxy compound) used as a polymerizable compound include a monofunctional or polyfunctional glycidyl ether compound and a polyfunctional aliphatic glycidyl ether compound. Further, as the epoxy compound, a compound containing an alicyclic epoxy group can also be used. Examples of the epoxy compound include a compound containing one or more epoxy groups in one molecule. It is preferable that the epoxy compound is a compound containing 1 to 100 epoxy groups in one molecule. The upper limit of the number of epoxy groups can be set to, for example, 10 or less or 5 or less. The lower limit of the epoxy group is preferably two or greater. Specific examples of the monofunctional epoxy compound include 2-ethylhexyl glycidyl ether. Specific examples of the polyfunctional epoxy compound include 1,4-cyclohexanedimethanol diglycidyl ether and 3',4'-epoxycyclohexylmethyl 3,4-epoxycyclohexane carboxylate.

The epoxy compound may be a low-molecular-weight compound (for example, a molecular weight of less than 1000) or a polymer (macromolecule) compound (for example, a molecular weight of 1000 or greater, and in a case of a polymer, a weight-average molecular weight of 1000 or greater). The weight-average molecular weight of the epoxy compound is preferably in a range of 2000 to 100000. The upper limit of the weight-average molecular weight is preferably 10000 or less, more preferably 5000 or less, and still more preferably 3000 or less. Examples of commercially available products of the epoxy compound include polyfunctional epoxy compounds such as CELLOXIDE 2021P (manufactured by

Daicel Corporation) (trade name: 3',4'-epoxycyclohexylmethyl 3,4-epoxycyclohexanecarboxylate) and RIKARESIN DME-100 (trade name, containing 1,4-cyclohexanedimethanol diglycidyl ether as a main component, manufactured by New Japan Chemical Co., Ltd.).

Examples of the compound containing a methylol group (hereinafter, also referred to as a methylol compound) include a compound in which the methylol group is bonded to a nitrogen atom or a carbon atom forming an aromatic ring. Examples of the compound in which the methylol group is bonded to a nitrogen atom include alkoxymethylated melamine, methylolated melamine, alkoxymethylated benzoguanamine, methylolated benzoguanamine, alkoxymethylated glycoluril, methylolated glycoluril, alkoxymethylated urea, methylolated urea, and a trimethylolpropane adduct of tolylene diisocyanate.

As the polymerizable compound, a polymer compound can also be used. Examples of the polymerizable compound of the polymer include a (meth)acrylic resin, an ester resin, a urethane resin, and a fluororesin. Examples of commercially available products thereof on the market include DIANAL BR Series (polymethyl methacrylate (PMMA), for example, DIANAL BR-80, BR-83, and BR-87; manufactured by Mitsubishi Chemical Corporation); Photomer 6173 (COOH-containing polyurethane acrylic oligomer, manufactured by Diamond Shamrock Co., Ltd.); VISCOAT R-264, KS Resist 106 (both manufactured by Osaka Organic Chemical Industry Ltd.); CYCLOMER P Series (for example, ACA230AA), PLACCEL CF200 Series (all manufactured by Dycel Corporation); Ebecryl 3800 (manufactured by Dycel UCB Co.); and Acrylic-RD-F8 (manufactured by Nippon Shokubai Co., Ltd.). The polymerizable compound of the polymer is a component that also corresponds to a resin.

From the viewpoint of improving the strength after curing, it is preferable that the polymerizable compound is a compound capable of forming a crosslinked structure. The formation of the crosslinked structure is not particularly limited, and examples of a method of forming the crosslinked structure include a method of using a polymerizable compound of a polymer and a polyfunctional (meth)acrylate monomer in combination, and a method of using a polymerizable compound of a polymer to which a reactive group has been introduced and a crosslinking agent containing a crosslinkable group that can react with the above-described reactive group in combination.

Examples of the reactive group include a group containing active hydrogen, and specific examples thereof include a group selected from the group consisting of a hydroxyl group, a primary amino group, and a secondary amino group. Examples of the polymerizable compound of the polymer to which the reactive group has been introduced include a (meth)acrylic resin which has a structural unit derived from a (meth)acrylate monomer and contains two or more groups having active hydrogen.

Examples of the crosslinking agent include a polyisocyanate containing two or more isocyanate groups as the crosslinkable group, and examples of commercially available products thereof on the market include AD-TMP and A-9550 (both trade names, manufactured by Shin Nakamura Chemical Industry Co., Ltd.). Among these, it is preferable to use a (meth)acrylic resin containing two or more (preferably three or more) groups having active hydrogen and a crosslinking agent containing two or more isocyanate groups (preferably a polyisocyanate containing two or more isocyanate groups and more preferably a polyisocyanate containing three or more isocyanate groups) in combination. In this manner, a crosslinked structure can be formed by reacting a group having active hydrogen with an isocyanate group.

As the compound having an -O-Si-O- structure, a hydrolyzable silicon compound is preferable, a hydrolyzable alkoxysilane is more preferable, and a trifunctional or tetrafunctional alkoxysilane is still more preferable. Specific examples of the compound having an -O-Si-O- structure include tetramethoxysilane, tetraethoxysilane, tetra-n-propoxysilane, tetra-i-propoxysilane, tetra-n-butoxysilane, methyltrimethoxysilane, methyltriethoxysilane, ethyltrimethoxysilane, ethyltriethoxysilane, n-propyltrimethoxysilane, n-propyltriethoxysilane, phenyltrimethoxysilane, phenyltriethoxysilane, $\gamma$-glycidyloxypropyltrimethoxysilane, $\gamma$-glysidyloxypropyltriethoxysilane, $\gamma$-glycidyloxypropylmethyldimethoxysilane, $\gamma$-glycidyloxypropylmethyldiethoxysilane, $\gamma$-methacryloyloxypropyltrimethoxysilane, $\gamma$-methacryloyloxypropyltriethoxysilane, $\gamma$-methacryloyloxypropylmethyldimethoxysilane, vinyl trimethoxysilane, vinyltriethoxysilane, 3,4-epoxycyclohexylethyltrimethoxysilane, 3,4-epoxycyclohexylethyltriethoxysilane, tris-(trimethoxysilylpropyl)isocyanurate, 4-trimethoxysilylstyrene, 3,3,3-trifluoropropyltrimethoxysilane, dimethyldimethoxysilane, dimethyldiethoxysilane, diethyldimethoxysilane, diethyldiethoxysilane, di-n-propyldimethoxysilane, di-n-propyldiethoxysilane, diphenyldimethoxysilane, divinyldiethoxysilane, bis(triethoxysilylpropyl)tetrasulfide, 3-(trimethoxysilyl)propyl isocyanate, and 3-(triethoxysilyl)propylisocyanate.

In a case where the resin composition contains a curable compound, the content of the curable compound in the total solid content of the resin composition is preferably in a range of 0.1% to 90% by mass. The lower limit thereof is preferably 1% by mass or greater and more preferably 5% by mass or greater. The upper limit thereof is preferably 80% by mass or less and more preferably 70% by mass or less. The resin composition may contain only one or two or more kinds of curable compounds. In a case where the resin composition contains two or more kinds of curable compounds, the total amount thereof is preferably in the above-described range.

<<Polymerization Initiator>>

The resin composition can contain a polymerization initiator. Particularly in a case where a polymerizable compound is used as the curable compound, it is preferable that the resin composition contains a polymerization initiator. In a case where the resin composition contains a polymerization initiator, the polymerization reaction of the polymerizable compound can be satisfactorily initiated. As the polymerization initiator, a compound capable of generating an initiating species required for the polymerization reaction by applying energy can be used. The polymerization initiator can be appropriately selected from a photopolymerization initiator and a thermal polymerization initiator, and a photopolymerization initiator is preferable.

As the photopolymerization initiator, for example, a compound having light absorption from an ultraviolet region to a visible region (for example, in a range of 280 nm to 400 nm) is preferable, and examples thereof include a photoradical initiator that generates an active radical to initiate photoradical polymerization and a cation initiator that initiates photocationic polymerization.

Examples of the photopolymerization initiator include a halogenated hydrocarbon derivative (such as a compound having a triazine skeleton or a compound having an oxadiazole skeleton), an acylphosphine compound, hexaarylbiimidazole, an oxime compound, an organic peroxide, a thio compound, a ketone compound, an aromatic onium salt, an aminoacetophenone compound, and a hydroxyacetophenone compound. Examples of the aminoacetophenone compound include aminoacetophenone-based initiators described in JP2009-191179A and JP1998-291969A (JP-H10-291969A). Examples of the acylphosphine compound include the acylphosphine-based initiator described in JP4225898B. As the photopolymerization initiator, a synthetic product may be used, or a commercially available product on the market may be used.

Examples of commercially available products of the hydroxyacetophenone compound include Omnirad 184, Omnirad 1173, Omnirad 2959, and Omnirad 127 (all manufactured by IGM Resins B. V.). Examples of commercially available products of the aminoacetophenone compound include Omnirad 907, Omnirad 369, Omnirad 369E, and Omnirad 379EG (all manufactured by IGM Resins B. V.). Examples of commercially available products of the acylphosphine compound include Omnirad 819 and Omnirad TPO (both manufactured by IGM Resins B. V.).

As the photopolymerization initiator, an oxime compound is preferable. Specific examples of the oxime compound include the compounds described in JP2001-233842A, the compounds described in JP2000-080068A, the compounds described in JP2006-342166A, and the compounds described in paragraphs 0073 to 0075 of JP2016-006475A. Among the examples of the oxime compound, an oxime ester compound is preferable. Examples of commercially available products of the oxime compound include Irgacure OXE01, Irgacure OXE02 (manufactured by BASF SE), and Irgacure OXE03 (manufactured by BASF SE).

Examples of the cationic polymerization initiator include an initiator that initiates photocationic polymerization, a light-decoloring agent of a dye compound, a photochromic agent, a known acid generator used for a microresist, and a mixture thereof. Specific examples of the cationic polymerization initiator include an onium compound, an organic halogen compound, and a disulfone compound.

Examples of the onium compound include a diazonium salt, an ammonium salt, an iminium salt, a phosphonium salt, an iodonium salt, a sulfonium salt, an arsonium salt, and a selenonium salt. Specific examples of the onium compound include the compounds described in paragraphs 0058 and 0059 of JP2002-029162A.

In a case where the resin composition contains a polymerization initiator, the content of the polymerization initiator in the total solid content of the resin composition is preferably in a range of 0.1% to 20% by mass. The lower limit thereof is preferably 0.3% by mass or greater and more preferably 0.4% by mass or greater. The upper limit thereof is preferably 15% by mass or less and more preferably 10% by mass or less. The resin composition may contain only one or two or more kinds of polymerization initiators. In a case where the resin composition contains two or more kinds of polymerization initiators, it is preferable that the total amount thereof is in the above-described range.

<<Acid generator>>

The resin composition according to the embodiment of the present invention can contain an acid generator. Particularly in a case where a cationically polymerizable compound such as a compound containing an epoxy group is used as the polymerizable compound, it is preferable that the resin composition contains an acid generator. The acid generator may be a photoacid generator or a thermal acid generator. In the present specification, an acid generator denotes a compound which generates an acid by applying energy such as heat or light. Further, the thermal acid generator denotes a compound that generates an acid by thermal decomposition. Further, the photoacid generator denotes a compound that generates an acid by light irradiation. Examples of the kind of acid generator, specific compounds, and preferred examples thereof include the compounds described in paragraphs 0066 to 0122 of JP2008-013646A, and these compounds can also be applied to the present invention.

As the thermal acid generator, a compound having a thermal decomposition temperature of 130°C to 250°C is

preferable, and a compound having a thermal decomposition temperature of 150°C to 220°C is more preferable. Examples of the thermal acid generator include compounds that generate low nucleophilic acids such as a sulfonic acid, a carboxylic acid, and disulfonylimide by heating. As the acid generated by the thermal acid generator, an acid having a pKa of 4 or less is preferable, an acid having a pKa of 3 or less is more preferable, and an acid having a pKa of 2 or less is still more preferable. For example, a sulfonic acid, an alkylcarboxylic acid substituted with an electron withdrawing group, an arylcarboxylic acid, or disulfonylimide is preferable. Examples of the electron-withdrawing group include a halogen atom such as a fluorine atom, a haloalkyl group such as a trifluoromethyl group, a nitro group, and a cyano group.

Examples of the photoacid generator include an onium salt compound such as a diazonium salt, a phosphonium salt, a sulfonium salt, or an iodonium salt, which are decomposed by light irradiation to generate an acid, and a sulfonate compound such as imide sulfonate, oxime sulfonate, diazodisulfone, disulfone, or ortho-nitrobenzyl sulfonate. Examples of commercially available products of the photoacid generator include WPAG-469 (manufactured by FUJIFIILM Wako Pure Chemical Corporation), CPI-100P (manufactured by San-Apro Ltd.), and Irgacure 290 (manufactured by BASF SE). Further, 2-isopropylthioxanthone can also be used as the photoacid generator.

In a case where the resin composition contains an acid generator, the content of the acid generator is preferably in a range of 0.1 to 100 parts by mass, more preferably in a range of 0.1 to 50 parts by mass, and still more preferably in a range of 0.1 to 20 parts by mass with respect to 100 parts by mass of the curable compound. The resin composition may contain only one or two or more kinds of acid generators. In a case where the resin composition contains two or more kinds of acid generators, it is preferable that the total amount thereof is in the above-described range.

<<Catalyst>>

The resin composition can contain a catalyst. Particularly in a case where a compound having an -O-Si-O- structure is used as the curable compound, it is preferable that the resin composition contains a catalyst. According to this aspect, the sol-gel reaction is promoted, and a stronger film is likely to be obtained. Examples of the catalyst include an acid catalyst such as hydrochloric acid, sulfuric acid, acetic acid, or propionic acid and a base catalyst such as sodium hydroxide, potassium hydroxide, or triethylamine. In a case where the resin composition contains a catalyst, the content of the catalyst is preferably in a range of 0.1 to 100 parts by mass, more preferably in a range of 0.1 to 50 parts by mass, and still more preferably in a range of 0.1 to 20 parts by mass with respect to 100 parts by mass of the curable compound. The resin composition may contain only one or two or more kinds of catalysts. In a case where the resin composition contains two or more kinds of catalysts, it is preferable that the total amount thereof is in the above-described range.

<<Silane coupling agent>>

The resin composition according to the embodiment of the present invention may contain a silane coupling agent. According to this aspect, the adhesiveness of the film to be obtained to the support can be further improved. In the present invention, the silane coupling agent denotes a silane compound containing a hydrolyzable group and other functional groups. Further, the hydrolyzable group denotes a substituent that is directly bonded to a silicon atom and can form a siloxane bond by at least one of a hydrolysis reaction or a condensation reaction. Examples of the hydrolyzable group include a halogen atom, an alkoxy group, and an acyloxy group. Among these, an alkoxy group is preferable. That is, it is preferable that the silane coupling agent is a compound containing an alkoxysilyl group. Examples of the functional group other than the hydrolyzable group include a vinyl group, a (meth)allyl group, a (meth)acryloyl group, a mercapto group, an epoxy group, an oxetanyl group, an amino group, a ureido group, a sulfide group, and an isocyanate group, and a phenyl group. Among these, an amino group, a (meth)acryloyl group, and an epoxy group are preferable. Specific examples of the silane coupling agent include the compounds described in paragraphs 0018 to 0036 of JP2009-288703A and the compounds described in paragraphs 0056 to 0066 of JP2009-242604A. Examples of commercially available products of the silane coupling agent include A-50 (organosilane) (manufactured by Soken Chemical & Engineering Co., Ltd.). The content of the silane coupling agent in the total solid content of the resin composition is preferably in a range of 0.1% to 5% by mass. The upper limit thereof is preferably 3% by mass or less and more preferably 2% by mass or less. The lower limit thereof is preferably 0.5% by mass or greater and more preferably 1% by mass or greater. The silane coupling agent may be used alone or in combination of two or more kinds thereof. In a case where two or more kinds of silane coupling agents are used, it is preferable that the total amount is in the above-described range.

<<Surfactant>>

The resin composition according to the embodiment of the present invention can contain a surfactant. As the surfactant, various surfactants such as a fluorine-based surfactant, a nonionic surfactant, a cationic surfactant, an anionic surfactant, and a silicon-based surfactant can be used. It is preferable that the surfactant is a fluorine-based surfactant. By allowing the resin composition to contain a fluorine-based surfactant, the liquid characteristics (particularly, fluidity) are

further improved, and a film having small thickness unevenness can be formed. The content of fluorine in the fluorine-based surfactant is suitably in a range of 3% to 40% by mass, more preferably in a range of 5% to 30% by mass, and particularly preferably in a range of 7% to 25% by mass. The fluorine-based surfactant in which the content of fluorine is in the above-described range is effective in terms of uniformity in the thickness of the coating film and liquid saving properties and has satisfactory solubility in the resin composition.

Examples of commercially available products of the fluorine-based surfactants include MEGAFAX F-171, F-172, F-173, F-176, F-177, F-141, F-142, F-143, F-144., F-437, F-475, F-477, F-479, F-482, F-554, F-555-A, F-556, F-557, F-558, F-559, F-560, F-561, F-565, F-563, F-568, F-575, F-780, EXP, MFS-330, R-01, R-40, R-40-LM, R-41, R-41-LM, RS-43, TF-1956, RS-90, R-94, RS-72-K, and DS-21 (all manufactured by DIC Corporation), FLUORARD FC430, FC431, and FC171 (all manufactured by Sumitomo 3M Ltd.), SURFLON S-382, SC-101, SC-103, SC-104, SC-105, SC-1068, SC-381, SC-383, S-393, and KH-40 (all manufactured by AGC Inc.), PolyFox PF636, PF656, PF6320, PF6520, and PF7002 (all manufactured by OMNOVA Solutions Inc.), and FTERGENT 208G, 215M, 245F, 601AD, 601ADH2, 602A, 610FM, 710FL, 710FM, 710FS, and FTX-218 (all manufactured by NEOS Company Limited). The following compounds are also exemplified as the fluorine-based surfactant used in the present invention.

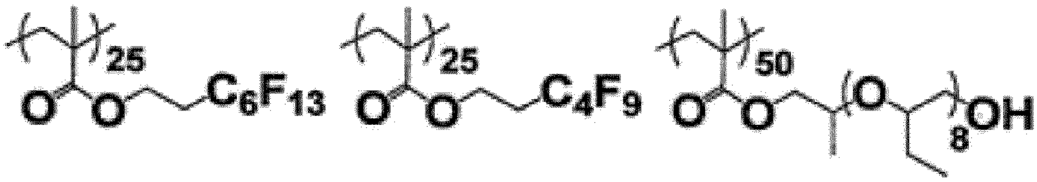

Compositional ratio numbers are in units of wt%

Examples of the nonionic surfactant include glycerol, trimethylolpropane, trimethylolethane, ethoxylates and propoxylates thereof (such as glycerol propoxylate and glycerol ethoxylate), polyoxyethylene lauryl ether, polyoxyethylene stearyl ether, polyoxyethylene oleyl ether, polyoxyethylene octyl phenyl ether, polyoxyethylene nonyl phenyl ether, polyethylene glycol dilaurate, polyethylene glycol distearate, sorbitan fatty acid ester, PLURONIC L10, L31, L61, L62, 10R5, 17R2, and 25R2 (manufactured by BASF SE), TETRONIC 304, 701, 704, 901, 904, and 150R1 (manufactured by BASF SE), SOLSPERSE 20000 (manufactured by Lubrizol Corporation), NCW-101, NCW-1001, and NCW-1002 (FUJIFILM Wako Pure Chemical Corporation), PIONIN D-6112, D-6112-W, and D-6315 (Takemoto Oil & Fat Co., Ltd.), and OLFINE E1010, SURFINOL 104, 400, and 440 (Nissin Chemical Industry Co., Ltd.).

Examples of the silicone-based surfactant include Toray Silicone DC3PA, Toray Silicone SH7PA, Toray Silicone DC11PA, Toray Silicone SH21PA, Toray Silicone SH28PA, Toray Silicone SH29PA, Toray Silicone SH30PA, and Toray Silicone SH8400 (all manufactured by Dow Toray Co., Ltd.), TSF-4440, TSF-4300, TSF-4445, TSF-4460, and TSF-4452 (all manufactured by Momentive Performance Materials Inc.), KP-341, KF-6001, and KF-6002 (all manufactured by Shin-Etsu Chemical Co., Ltd.), and BYK-307, BYK-323, BYK-330, BYK-3760, and BYK-UV3510 (all manufactured by BYK-Chemie GmbH).

In recent years, it was clarified that compounds containing a linear perfluoroalkyl group having 7 or more carbon atoms have high toxicity and ecological accumulation, and thus the use of perfluorooctanoic acid and perfluorooctanesulfonic acid is restricted. Therefore, it is preferable to use a surfactant using an alternative material for perfluorooctanoic acid or perfluorooctanesulfonic acid. From the viewpoint of improving environmental suitability, a surfactant derived from an alternative material for the compound containing a linear perfluoroalkyl group having 7 or more carbon atoms such as perfluorooctanoic acid (PFOA) or perfluorooctanesulfonic acid (PFOS) is preferably used as the fluorine-based surfactant.

The content of the surfactant in the total solid content of the resin composition is preferably in a range of 0.001% by mass to 5.0% by mass and more preferably in a range of 0.005 to 3.0% by mass. The surfactant may be used alone or two or more kinds thereof. In a case where two or more kinds of silane coupling agents are used, it is preferable that the total amount is in the above-described range.

<<Solvent>>

It is preferable that the resin composition further contains a solvent. The solvent is not particularly limited, and examples thereof include water and an organic solvent. Examples of the organic solvent include an alcohol-based solvent, an ester-based solvent, a ketone-based solvent, an amide-based solvent, an ether-based solvent, a hydrocarbon-based solvent, and a halogen-based solvent. Specific examples of the organic solvent include methanol, ethanol, 1-propanol, 2-propanol, 1-butanol, 2-butanol, 2-methyl-1-propanol, 1-methoxy-2-propanol, 2-ethoxyethanol, 2-butoxyethanol, polyethylene glycol monoalkyl ether, polypropylene glycol monoalkyl ether, ethylene glycol, propylene glycol, polyethylene glycol, polypropylene glycol, glycerin, ethylene carbonate, N-methylpyrrolidone, dioxane, tetrahydrofuran, ethylene glycol

dialkyl ether, propylene glycol dialkyl ether, polyethylene glycol dialkyl ether, polypropylene glycol dialkyl ether, acetonitrile, propionitrile, benzonitrile, carboxylic acid ester, phosphoric acid ester, phosphonic acid ester, dimethyl sulfoxide, sulfolane, dimethylformamide, dimethylacetamide, ethyl acetate, chloroform, methylene chloride, and methyl acetate. The solvent may be used alone or in combination of two or more kinds thereof. The content of the solvent is preferably in a range of 10% to 90% by mass with respect to the total amount of the resin composition.

<<Other additives>>

The resin composition may appropriately contain optional additives such as an antioxidant, a light stabilizer, a processing stabilizer, an anti-aging agent, and a compatibilizer as necessary. By allowing the resin composition to appropriately contain these components, various characteristics of the cured substance to be obtained can be appropriately adjusted.

<<Applications>>

The resin composition according to the embodiment of the present invention can also be suitably used for applications in a case where the resin composition may be exposed to light including sunlight and ultraviolet rays. Specific examples include coating materials or films for window glass of houses, facilities, and transportation equipment; interior/exterior materials and interior/exterior paints of houses, facilities, and transportation equipment; members for light sources that emit ultraviolet rays, such as a fluorescent lamp and a mercury lamp; solar cells, precision machineries, electronic and electrical equipment, and members for a display device; containers or packaging materials for food, chemicals, and drugs; agricultural and industrial sheets; clothing textile products and fibers such as sportswear, stockings, and hats; lenses such as plastics lenses, contact lenses, glasses, and artificial eyes, or coating materials thereof; optical supplies such as optical filters, prisms, mirrors, and photographic materials; stationery such as tapes and inks; and marking boards, marking devices, and the surface coating materials thereof. For the details thereof, the description in paragraphs 0158 to 0218 of JP2009-263617A can be referred to.

The resin composition according to the embodiment of the present invention is preferably used for an ultraviolet cut filter, a lens, or a protective material. The form of the protective material is not particularly limited, and examples thereof include a coating film, a film, and a sheet. Further, the resin composition according to the embodiment of the present invention can also be used as a pressure sensitive adhesive or an adhesive.

Further, the resin composition according to the embodiment of the present invention can also be used for various members of a display device. For example, in a case of a liquid crystal display device, the resin composition can be used for each member constituting the liquid crystal display device such as an antireflection film, a polarizing plate protective film, an optical film, a phase difference film, a pressure sensitive adhesive, and an adhesive. Further, in a case of an organic electroluminescence display device, the resin composition can be used for each member constituting the organic electroluminescence display device such as an optical film, a polarizing plate protective film in a circular polarizing plate, a phase difference film such as a quarter wave plate, and an adhesive or a pressure sensitive adhesive.

<Cured substance and applications>

The cured substance according to the embodiment of the present invention is obtained by using the above-described resin composition according to the embodiment of the present invention. The "cured substance" in the present specification includes a dried product obtained by drying and solidifying the resin composition and a cured substance cured by performing a curing reaction on the resin composition in a case where the resin composition undergoes a curing reaction.

The cured substance according to the embodiment of the present invention may be obtained as a molded product formed by molding the resin composition into a desired shape. The shape of the molded product can be appropriately selected according to the intended use and the purpose. Examples of the shape thereof include a coating film, a film, a sheet, a plate, a lens, a tube, and a fiber.

The cured substance according to the embodiment of the present invention is preferably used as an optical member. Examples of the optical member include an ultraviolet cut filter, a lens, and a protective material. Further, the optical member can also be used as a polarizing plate.

The ultraviolet cut filter can be used for an article such as an optical filter, a display device, a solar cell, or window glass. The kind of display device is not particularly limited, and examples thereof include a liquid crystal display device and an organic electroluminescence display device.

In a case where the cured substance according to the embodiment of the present invention is used for a lens, the cured substance according to the embodiment of the present invention may be formed into a lens shape and used. Further, the cured substance according to the embodiment of the present invention may be used for a coating film on a surface of a

lens, or an interlayer (adhesive layer) of a cemented lens. Examples of the cemented lens include those described in paragraphs 0094 to 0102 of WO2019/131572A.

The kind of the protective material is not particularly limited, and examples thereof include a protective material for a display device, a protective material for a solar cell, a protective material for window glass, and an organic electroluminescence display device. The shape of the protective material is not particularly limited, and examples thereof include a coating film, a film, and a sheet.

<Ultraviolet absorbing agent>

An ultraviolet absorbing agent according to the embodiment of the present invention contains a compound represented by Formula (1) (compound (1)). The compound (1) has the same definition as that for the compound described in the section of the resin composition above, and the preferable ranges thereof are the same as described above. It is preferable that the compound (1) used for the ultraviolet absorbing agent is the compound represented by Formula (1a) described above.

The ultraviolet absorbing agent according to the embodiment of the present invention can also be used by being added to a pressure sensitive adhesive or an adhesive. Examples of the pressure sensitive adhesive include an acrylic pressure sensitive adhesive, a rubber-based pressure sensitive adhesive, and a silicone-based pressure sensitive adhesive. The acrylic pressure sensitive adhesive denotes a pressure sensitive adhesive containing a polymer of a (meth) acrylic monomer ((meth)acrylic polymer). Examples of the adhesive include a urethane resin adhesive, a polyester adhesive, an acrylic resin adhesive, an ethylene vinyl acetate resin adhesive, a polyvinyl alcohol adhesive, a polyamide adhesive, and a silicone adhesive. Among these, from the viewpoint of excellent adhesive strength, a urethane resin adhesive or a silicone adhesive is preferable as the adhesive. As the adhesive, a commercially available product on the market may be used, and examples of the commercially available product thereof include a urethane resin adhesive (LIS-073-50U: trade name, manufactured by of Toyo Ink Co., Ltd.) and an acrylic pressure sensitive adhesive (SK Dyne-SF2147: trade name, manufactured by Soken Chemical & Engineering Co., Ltd.). A curing agent may be further used in combination with the adhesive. Examples of commercially available products of the curing agent include CR-001 (trade name, manufactured by Toyo Ink Co., Ltd.).

The content of the compound (1) in the ultraviolet absorbing agent is preferably in a range of 1% to 100% by mass, more preferably in a range of 10% to 100% by mass, and still more preferably in a range of 20% to 100% by mass. The ultraviolet absorbing agent may contain only one or two or more kinds of the compounds (1). In a case where the resin composition contains two or more kinds of the compounds (1), it is preferable that the total amount thereof is in the above-described range.

It is preferable that the ultraviolet absorbing agent according to the embodiment of the present invention further contains the compound represented by Formula (2) (compound (2)) described above. The compound (2) has the same definition as that for the compound described in the section of the resin composition above, and the preferable ranges are the same as described above.

In a case where the ultraviolet absorbing agent contains the compound (2), the content of the compound (2) is preferably 5 parts by mass or less, more preferably 3 parts by mass or less, and still more preferably 1 part by mass with respect to 100 parts by mass of the compound (1). The lower limit thereof is preferably 0.1 part by mass or greater. The ultraviolet absorbing agent may contain only one or two or more kinds of the compounds (2). In a case where the resin composition contains two or more kinds of the compounds (2), it is preferable that the total amount thereof is in the above-described range.

<Optical member>

An optical member according to the embodiment of the present invention contains the ultraviolet absorbing agent according to the embodiment of the present invention. It is also preferable that the optical member according to the embodiment of the present invention contains a cured substance formed of the resin composition according to the embodiment of the present invention. The cured substance according to the embodiment of the present invention may be obtained as a molded product formed by molding the above-described resin composition according to the embodiment of the present invention into a desired shape. The shape of the molded product can be appropriately selected according to the intended use and the purpose. Examples of the shape thereof include a coating film, a film, a sheet, a plate, a lens, a tube, and a fiber.

Further, the optical member according to the embodiment of the present invention may be obtained by using a pressure sensitive adhesive or an adhesive containing the ultraviolet absorbing agent according to the embodiment of the present invention. For example, the optical member may be a member in which a polarizing plate and a polarizing plate protective film are attached to each other using a pressure sensitive adhesive or an adhesive containing the ultraviolet absorbing agent.

Examples of the optical member include an ultraviolet cut filter, a lens, and a protective material.
The ultraviolet cut filter can be used for an article such as an optical filter, a display device, a solar cell, or window glass. The kind of display device is not particularly limited, and examples thereof include a liquid crystal display device and an organic electroluminescence display device.
Examples of the lens include those obtained by forming the cured substance according to the embodiment of the present invention into a lens shape and those obtained by allowing a coating film on a surface of a lens, an interlayer (an adhesive layer or a pressure sensitive adhesive layer) of a cemented lens to contain the ultraviolet absorbing agent according to the embodiment of the present invention.
The kind of the protective material is not particularly limited, and examples thereof include a protective material for a display device, a protective material for a solar cell, and a protective material for window glass. The shape of the protective material is not particularly limited, and examples thereof include a coating film, a film, and a sheet.
Further, a resin film is exemplified as one form of the optical member. The resin film can be formed of the above-described resin composition according to the embodiment of the present invention. Examples of the resin used in the resin composition for forming a resin film include the above-described resins. Among these, a (meth)acrylic resin, a polyester fiber, a cyclic olefin resin, and a cellulose acylate resin are preferable, and a cellulose acylate resin is more preferable. The resin composition containing the cellulose acylate resin can contain the additives described in paragraphs 0022 to 0067 of JP2012-215689A. Examples of such additives include sugar esters. By adding a sugar ester compound to the resin composition containing a cellulose acylate resin, the total haze and the internal haze can be decreased without impairing the expression of optical properties even in a case where a heat treatment is not performed before a stretching step. Examples of the sugar ester include a sugar ester 1 and a sugar ester 2 described in examples below. Further, the resin film formed of the resin composition containing the cellulose acylate resin (cellulose acylate film) can be produced by the method described in paragraphs 0068 to 0096 of JP2012-215689. Further, the hard coat layer described in paragraphs 0097 to 0113 of JP2012-215689A may be further laminated on the resin film.
Further, examples of other forms of the optical member include an optical member having a laminate of a transparent support base material and a resin layer. In this case, at least one of the support base material or the resin layer is obtained by using the above-described resin composition according to the embodiment of the present invention or contains the above-described ultraviolet absorbing agent according to the embodiment of the present invention. Such an optical member is preferably used as a film-like or sheet-like ultraviolet cut filter or a protective material.
It is preferable that the support base material has transparency within a range where the optical performance is not impaired. The expression "the support base material has transparency" denotes that the support base material is optically transparent and specifically, the total light transmittance of the support base material is 85% or greater. The total light transmittance of the support base material is preferably 90% or greater and more preferably 95% or greater. The total light transmittance of the support base material can be measured by the following method. The total light transmittance is a value obtained by measuring the spectral spectrum of the support base material using a UV/vis spectrum meter (for example, a UV/vis spectrum meter UV3400, manufactured by Shimadzu Corporation) and acquired based on the measured values.
Suitable examples of the support base material include a resin film. Examples of the resin forming the support base material include an ester resin (such as polyethylene terephthalate (PET), polyethylene naphthalate (PEN), polybutylene terephthalate (PBT), or polycyclohexane dimethylene terephthalate (PCT)), an olefin resin (such as polypropylene (PP), polyethylene (PE)), polyvinyl chloride (PVA), and tricellulose acetate (TAC). Among these, PET is preferable in terms of general purpose properties.
The support base material is obtained by molding the above-described resin into a plate shape by a method of the related art. Further, as the support base material, a commercially available resin film on the market may be used. Further, the support base material may be obtained by using the above-described resin composition according to the embodiment of the present invention.
The thickness of the support base material can be appropriately selected according to the intended use, and the purpose. In general, the thickness thereof is preferably in a range of 5 μm to 2500 μm and more preferably in a range of 20 μm to 500 μm.
The resin layer is a layer formed of the resin composition. As the resin composition, the above-described resin composition according to the embodiment of the present invention can be used. The resin layer may be a layer that has been dried and solidified or may be a cured layer obtained by a curing reaction.
The thickness of the resin layer is not particularly limited and can be optionally selected from the viewpoint of a desired visible light transmittance. The thickness of the resin layer can be set to be, for example, in a range of 5 μm to 2500 μm. Particularly from the viewpoints of easily ensuring the function of blocking or suppressing ultraviolet rays and blue light, easily ensuring the visible light transmittance, and handleability, the thickness of the resin layer is preferably in a range of 5 μm to 500 μm and more preferably in a range of 5 μm to 100 μm.
Further, as the transparent support base material, a peelable support base material (peelable laminated film) can also be used. An optical member for which such a support base material is used is preferably used for a polarizing plate.

Examples of the peelable laminated film include a peelable laminated film having a configuration in which a support containing polyethylene terephthalate and a resin layer containing the ultraviolet absorbing agent according to the embodiment of the present invention are in direct contact with each other.

The support of the peelable laminated film is a material that can be peeled from the resin layer. The stress in a case of peeling the support from the resin layer is preferably in a range of 0.05 N/25 mm or greater and 2.00 N/25 mm or less, more preferably 0.08 N/25 mm or greater and 0.50 N/25 mm or less, and still more preferably 0.11 N/25 mm or greater and 0.20 N/25 mm or less. It is preferable that the stress is 0.05 N/25 mm or greater because the support is unlikely to be peeled off during the polarizing plate processing process, and it is preferable that the stress is 2.00 N/25 mm or less because the polarizing plate is not broken in a case of peeling the support. The stress in a case of peeling the support of the peelable laminated film from the resin layer is evaluated by bonding and fixing the surface of the optical film of the peelable laminated film cut to have a size of a width of 25 mm and a length of 80 mm to a glass base material via an acrylic pressure sensitive adhesive sheet, grasping one end (one side with a width of 25 mm) of the base material film in the length direction of the test piece using a tension tester (RTF-1210, manufactured by A & D Co., Ltd.), and performing a 90° peeling test (in conformity with Japanese Industrial Standards (JIS) K 6854-1: 1999 "Adhesive-Determination of peel strength of bonded assemblies-Part 1: 90° peel") in an atmosphere of a temperature of 23°C, a relative humidity of 60%, and a closshead speed (grasping movement speed) of 200 mm/min.

The support of the peelable laminated film will be described. As the support of the peelable laminated film, a support containing polyethylene terephthalate (PET) is used. It is preferable that the main component of the support (the component having the highest content in terms of mass among the components constituting the support) is polyethylene terephthalate (PET). From the viewpoint of mechanical strength, the weight-average molecular weight of PET is preferably 20000 or greater, more preferably 30000 or greater, and still more preferably 40000 or greater. The weight-average molecular weight of PET can be determined by dissolving the support in hexafluoroisopropanol (HFIP) using the above-described GPC method. The thickness of the support is not particularly limited, but is preferably in a range of 0.1 to 100 μm, more preferably in a range of 0.1 to 75 μm, still more preferably in a range of 0.1 to 55 μm, and particularly preferably in a range of 0.1 to 10 μm. Further, the support may be subjected to a corona treatment, a glow discharge treatment, undercoating, as a known surface treatment.

The peelable laminated film can be produced by coating the support with a solution containing the ultraviolet absorbing agent according to the embodiment of the present invention, the resin, and the solvent and drying the solution to form a resin layer. The solvent can be appropriately selected from the viewpoints of being able to dissolve or disperse the resin, easily forming a uniform surface in the coating steps and the drying step, ensuring liquid preservability, and having an appropriate saturated vapor pressure.

Further, examples of other forms of the optical member include a laminate obtained by laminating a hard coat layer, a transparent support base material, and a pressure sensitive adhesive layer or an adhesive layer in this order. Such a laminate is preferably used as an ultraviolet cut filter or a protective material (a protective film or a protective sheet).

In the optical member in this form, any of the support base material, the hard coat layer, and the pressure sensitive adhesive layer or the adhesive layer may contain the above-described ultraviolet absorbing agent according to the embodiment of the present invention. Examples of the transparent support base material include those described in the first form.

The optical member in this form has a hard coat layer on the support base material. The hard coat layer is provided on the outermost surface layer of the optical member, and thus the scratch resistance of the optical member can be improved. The hard coat layer may be formed by any of a wet coating method or a dry coating method (vacuum film formation). From the viewpoint of excellent productivity, the wet coating method is preferable. In a case where the hard coat layer is formed of the resin composition according to the embodiment of the present invention, it is preferable that the hard coat layer is formed by the wet coating method.

In a case where the hard coat layer is not a cured substance of the resin composition according to the embodiment of the present invention, the hard coat layers described in JP2013-045045A, JP2013-043352A, JP2012-232459A, JP2012-128157A, JP2011-131409A, JP2011-131404A, JP2011-126162A, JP2011-075705A, JP2009-286981A, JP2009-263567A, JP2009-075248A, JP2007-164206A, JP2006-096811A, JP2004-075970A, JP2002-156505A, JP2001-272503A, WO2012/018087A, WO2012/098967A, WO2012/086659A, and WO2011/105594A can be employed as the hard coat layer.

The thickness of the hard coat layer is preferably in a range of 5 μm to 100 μm from the viewpoint of further improving the scratch resistance.

The optical member in this form has a pressure sensitive adhesive layer or an adhesive layer on a side of the support base material opposite to a side where the hard coat layer is provided. The kind of the pressure sensitive adhesive or the adhesive used for the pressure sensitive adhesive layer or the adhesive layer is not particularly limited. Examples thereof include the above-described pressure sensitive adhesives or adhesives. Further, as the pressure sensitive adhesive or the adhesive, those to which the above-described ultraviolet absorbing agent according to the embodiment of the present invention is added can also be used. Further, the resin composition according to the embodiment of the present

invention described above can be used as the pressure sensitive adhesive or the adhesive. As the pressure sensitive adhesive or the adhesive, those containing the acrylic resin described in paragraphs 0056 to 0076 of JP2017-142412A and the crosslinking agent described in paragraphs 0077 to 0082 of JP2017-142412A are also preferably used. Further, the pressure sensitive adhesive or the adhesive may contain an adhesiveness improver (silane-based compound) described in paragraphs 0088 to 0097 of JP2017-142412A and the additives described in paragraph 0098 of JP2017-142412A. Further, the pressure sensitive adhesive layer or the adhesive layer can be formed by the method described in paragraphs 0099 and 0100 of JP2017-142412A.

The thickness of the pressure sensitive adhesive layer or the adhesive layer is preferably in a range of 5 μm to 100 μm from the viewpoint of achieving both adhesive strength and handleability.

<Disposition of optical member for display applications>

The optical member according to the embodiment of the present invention can be preferably used as a constituent member of a display such as a liquid crystal display device (LCD) or an organic electroluminescence display device (OLED).

<<Liquid crystal display device>>

Examples of the liquid crystal display device include a liquid crystal display device containing the ultraviolet absorbing agent according to the embodiment of the present invention in a member such as an antireflection film, a polarizing plate protective film, an optical film, a phase difference film, a pressure sensitive adhesive, or an adhesive. The optical member containing the ultraviolet absorbing agent according to the embodiment of the present invention may be disposed on any of a viewer side (front side) or a backlight side with respect to the liquid crystal cell and any of a side far from the liquid crystal cell (outer) or a side close to the liquid crystal cell (inner) with respect to the polarizer. Figs. 1 to 10 illustrate preferable configurations of a liquid crystal display device including the optical member containing the ultraviolet absorbing agent according to the embodiment of the present invention. Further, Figs. 1 to 10 are schematic views, and the relationship between the thicknesses of respective layers and the positional relationship do not necessarily match the actual relationships. Fig. 1 is a schematic view illustrating a configuration in which the ultraviolet absorbing agent according to the embodiment of the present invention is added to a polarizing plate protective film on a front side. Fig. 2 is a schematic view illustrating a configuration in which the ultraviolet absorbing agent according to the embodiment of the present invention is added to the polarizing plate protective film on a backlight side. Fig. 3 is a schematic view illustrating a configuration in which the ultraviolet absorbing agent according to the embodiment of the present invention is added to an inner protective layer on a front side. Fig. 4 is a schematic view illustrating a configuration in which the ultraviolet absorbing agent according to the embodiment of the present invention is added to the inner protective film on a backlight side. The inner protective film can also serve as a phase difference film. Fig. 5 is a schematic view illustrating a configuration in which an optical film containing the ultraviolet absorbing agent according to the embodiment of the present invention is bonded to a phase difference film on a front side via an adhesive or a pressure sensitive adhesive. Fig. 6 is a schematic view illustrating a configuration in which an optical film containing the ultraviolet absorbing agent according to the embodiment of the present invention is bonded to the phase difference film on a backlight side via an adhesive or a pressure sensitive adhesive. Fig. 7 is a schematic view illustrating a configuration in which the ultraviolet absorbing agent according to the embodiment of the present invention is added to an adhesive or a pressure sensitive adhesive on a front side. Fig. 8 is a schematic view illustrating a configuration in which the ultraviolet absorbing agent according to the embodiment of the present invention is added to an adhesive or a pressure sensitive adhesive on a backlight side. Fig. 9 is a schematic view illustrating a configuration in which the ultraviolet absorbing agent according to the embodiment of the present invention is added to a functional layer on a front side. Fig. 10 is a schematic view illustrating a configuration in which the ultraviolet absorbing agent according to the embodiment of the present invention is added to a functional layer on a backlight side. Examples of the functional layer include an antireflection layer and a hard coat layer.

**In** a case where the optical member containing the ultraviolet absorbing agent according to the embodiment of the present invention is disposed on the viewer side (front side), deterioration of the material in the liquid crystal cell due to external light can be prevented.

**In** a case where the ultraviolet absorbing agent according to the embodiment of the present invention is added to the polarizing plate protective film, it is preferable that the above-described cellulose acylate film or the resin layer in the above-described peelable laminated film is used as the polarizing plate protective film. In a case where the ultraviolet absorbing agent according to the embodiment of the present invention is added to the polarizing plate protective film, the ultraviolet absorbing agent according to the embodiment of the present invention may be contained on any one or both of a side closer to the liquid crystal cell side and a side far from the liquid crystal cell.

A cellulose acylate film is used as the polarizing plate used in the liquid crystal display device. The polarizing plate having a cellulose acylate film can be produced by the method described in paragraphs 0114 to 0117 of JP2012-215689A.

In a case where the resin layer in the peelable laminated film is used as a polarizing plate protective film, a polarizing plate having a polarizer and a resin layer containing the ultraviolet absorbing agent according to the embodiment of the present invention can be obtained by bonding the surface of the peelable laminated film on a side opposite to the interface of the support to the polarizer via an adhesive and peeling off the support. The depth of penetration into the resin layer containing the ultraviolet absorbing agent changes depending on a difference in SP (solubility parameter) value between the adhesive and the resin layer containing the ultraviolet absorbing agent and aging conditions (such as the temperature and the time) after the adhesive and the resin layer containing the ultraviolet absorbing agent come into contact with each other, and for example, conditions of a low temperature and a short time are suitable for suppressing penetration. Further, in regard to the depth of penetration, the permeation rate can be decreased by temporarily applying active energy rays, and thus additional irradiation after the depth of penetration is suppressed by temporary irradiation is also effective for the purpose of promoting curing to ensure the durability of the polarizing plate.
The surface of the resin layer containing the ultraviolet absorbing agent of the peelable laminated film opposite to the interface on the support side may be subjected to a hydrophilic treatment by performing a glow discharge treatment, a corona treatment, an alkali saponification treatment as necessary.
The support can be peeled off by the same method as the separator (peeling film) peeling step performed by a typical polarizing plate with a pressure sensitive adhesive. The support may be peeled off immediately after a step of laminating a resin layer containing an ultraviolet absorbing agent and a polarizer via an adhesive and drying the laminate or may be separately peeled off in a subsequent step after temporarily being wound in a roll shape after the drying step.

<<Organic electroluminescence display device>>

Examples of the organic electroluminescence display device include an organic electroluminescence display device in which a member such as an optical film, a polarizing plate protective film in a circular polarizing plate, a phase difference film such as a quarter wave plate, an adhesive, or a pressure sensitive adhesive contains the ultraviolet absorbing agent according to the embodiment of the present invention. Further, a form in which the peelable laminated film is bonded to a circular polarizing plate via an adhesive or a pressure sensitive adhesive is also preferable as a method of introducing the ultraviolet absorbing agent according to the embodiment of the present invention. By adding the ultraviolet absorbing agent according to the embodiment of the present invention with the above configuration, deterioration of the organic electroluminescence display device due to external light can be suppressed.
Figs. 11 to 13 illustrate a preferable configuration of an organic electroluminescence display device including an optical member containing the ultraviolet absorbing agent according to the embodiment of the present invention. Further, Figs. 11 to 13 are schematic views, and the relationship between the thicknesses of respective layers and the positional relationship do not necessarily match the actual relationships. Fig. 11 is a schematic view illustrating a configuration in which the ultraviolet absorbing agent according to the embodiment of the present invention is added to a polarizing plate protective film. Fig. 12 is a schematic view illustrating a configuration in which the ultraviolet absorbing agent according to the embodiment of the present invention is added to an adhesive or a pressure sensitive adhesive. Fig. 13 is a schematic view illustrating a configuration in which an optical film containing the ultraviolet absorbing agent according to the embodiment of the present invention is bonded to a touch panel via an adhesive or a pressure sensitive adhesive.

<Compound and method of synthesizing compound>

A compound according to the embodiment of the present invention is a compound represented by Formula (1a). The compound represented by Formula (1a) has the same definition as that for the compound described in the section of the resin composition above, and the preferable ranges are the same as described above. The compound represented by Formula (1a) is preferably used as an ultraviolet absorbing agent.
The maximum absorption wavelength of the compound represented by Formula (1a) is present preferably in a wavelength range of 381 to 420 nm and more preferably in a wavelength range of 381 to 400 nm.
The molar absorption coefficient $\varepsilon_{405}$ of the compound represented by Formula (1a) calculated by the following formula at a wavelength of 405 nm is preferably 500 or greater, more preferably 1000 or greater, still more preferably 2000 or greater, and particularly preferably 3000 or greater.

$$\varepsilon_{405} = \varepsilon_{max} \times (A_{405}/A_{max})$$

$\varepsilon_{405}$ represents the molar absorption coefficient of the compound represented by Formula (1a) at a wavelength of 405 nm, $\varepsilon_{max}$ represents the molar absorption coefficient of the compound represented by Formula (1a) at the maximum absorption wavelength, $A_{405}$ represents the absorbance of the compound represented by Formula (1a) at a wavelength of 405 nm, and $A_{max}$ represents the absorbance of the compound represented by Formula (1a) at the maximum absorption

wavelength.

In the spectral absorption spectrum of the compound represented by Formula (1a) measured in ethyl acetate, the ratio of the absorbance $A_{430}$ at a wavelength of 430 nm to the absorbance $A_{405}$ at a wavelength of 405 nm ($A_{430}/A_{405}$) is preferably less than 0.13 and more preferably 0.10 or less. The lower limit of the ratio is not particularly limited, but can be set to 0 or greater.

The compound represented by Formula (1a) can be synthesized by performing a step of reacting a compound represented by Formula (2a) with an alkyl halide compound or a carboxylic acid halide.

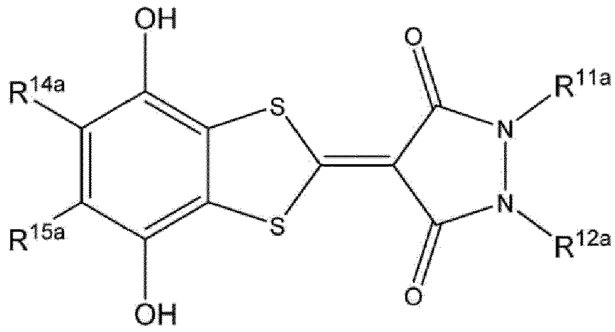

(2a)

in Formula (2a), $R^{11a}$ and $R^{12a}$ each independently represent an alkyl group,
$R^{14a}$ represents an alkyl group or an alkoxy group,
$R^{15a}$ represents a hydrogen atom, an alkyl group, or an alkoxy group, and
$R^{14a}$ and $R^{15a}$ may be bonded to each other to form a ring.

The groups represented by $R^{11a}$, $R^{12a}$, $R^{14a}$, and $R^{15a}$ in Formula (2a) each have the same definition as that for $R^{11}$, $R^{12}$, $R^{14}$, and $R^{15}$ in Formula (2), and the preferable ranges thereof are also the same as described above.

Examples of the alkyl halide compound that reacts with the compound represented by Formula (2a) include a compound represented by $R^{30}$-$X^{30}$. Examples of the carboxylic acid halide that reacts with the compound represented by Formula (2a) include a compound represented by $R^{30}$-C(=O)-$X^{30}$. $R^{30}$ represents an alkyl group, and $X^{30}$ represents a halogen atom. The number of carbon atoms of the alkyl group represented by $R^{30}$ is preferably in a range of 1 to 30, more preferably in a range of 1 to 20, still more preferably in a range of 1 to 15, particularly preferably in a range of 1 to 10, and most preferably in a range of 1 to 8. The alkyl group may be linear or branched. The alkyl group may have a substituent. Examples of the substituent include the groups described in the section of the substituent T above. Examples of the halogen atom represented by $X^{30}$ include a chlorine atom, a bromine atom, and an iodine atom.

Examples

Hereinafter, the present invention will be described in more detail based on the following examples. The materials, the used amounts, the ratios, the treatment contents, the treatment procedures described in the following examples can be appropriately changed without departing from the gist of the present invention. Therefore, the scope of the present invention is not limited to the following specific examples. Further, in the structural formulae shown below, Me represents a methyl group, Et represents an ethyl group, Bu represents a butyl group, tBu represents a tert-butyl group, Pr represents a propyl group, Ph represents a phenyl group, and Ac represents an acetyl group.

<Synthesis examples>

(Synthesis Example 1) (synthesis of compound (1)-1)

An intermediate 1-1 was synthesized according to the following scheme. In the following scheme, the synthesis from p-toluquinone to the intermediate 1-1 was performed using p-toluquinone in place of 2-tert-butyl-1,4-benzoquinone with reference to the method described in paragraph 0176 of JP2016-081035A.

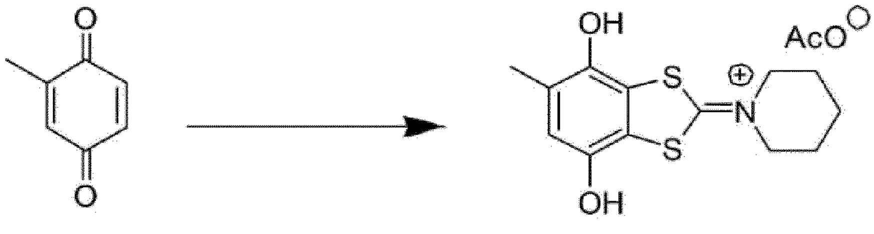

Intermediate 1-1

Next, a compound (2)-31 was synthesized according to the following synthesis scheme. The synthesis from the intermediate 1-1 to the compound (2)-31 was performed using the intermediate 1-1 in place of 1-(4,7-dihydroxybenzo[1,3] dithiol-2-ylidene)piperidinium acetate with reference to the method described in paragraphs 0154 to 0155 of JP5376885B.

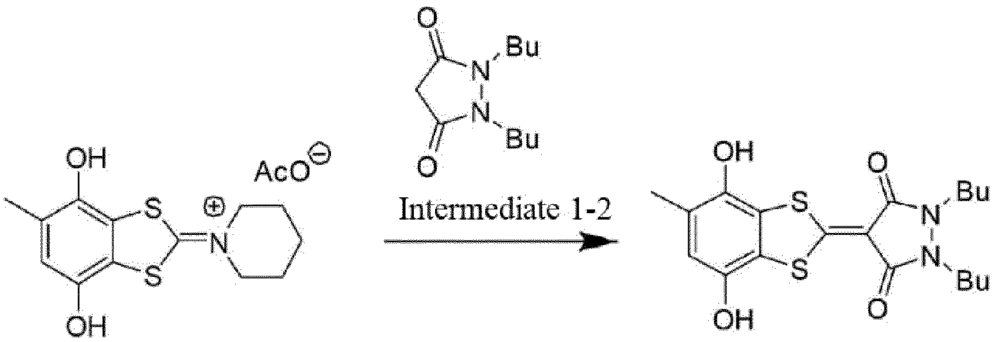


Intermediate 1-1 (2)-31

Next, 0.5 g of the compound (2)-31, 0.39 g of potassium carbonate, and 5 ml of N,N-dimethylacetamide were mixed and stirred at room temperature for 5 minutes. 0.46 g of 2-iodopropane was added to the mixture, and the mixture was stirred while being heated at 90°C for 4 hours. After completion of the reaction, the mixture was cooled to room temperature, 1.5 ml of water was added thereto, and the mixture was stirred for 20 minutes. After the precipitated solid was collected by filtration, 2.5 ml of methanol and 2.5 ml of acetonitrile were added thereto, and the mixture was heated under reflux in a nitrogen atmosphere for 1 hour. After the mixture was cooled to room temperature and stirred at room temperature for 1 hour, and the solid was collected by filtration and washed with a mixed solvent of 2.5 ml of methanol and 2.5 ml of acetonitrile, thereby obtaining 0.4 g of a compound (1)-1 (yield of 70%). $^1$H-NMR ($CDCl_3$): δ 6.70 (s, 1H), 4.62 (m, 1H), 4.42 (m, 1H), 3.66 (m, 4H), 2.34 (s, 3H), 1.54 (m, 4H), 1.4-1.2 (m, 16H), 0.91 (m, 6H)

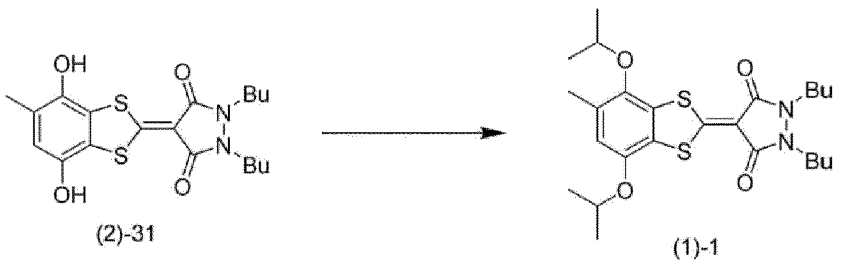

(2)-31 (1)-1

(Synthesis Example 2) (synthesis of compound (1)-2 to compound (1)-8)

Each of the compounds (1)-2 to (1)-8 was synthesized by the same method as in Synthesis Example 1 except that the corresponding alkylating agent was used in place of 2-iodopropane in Synthesis Example 1.

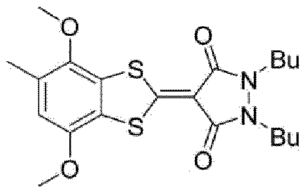
(1)-2

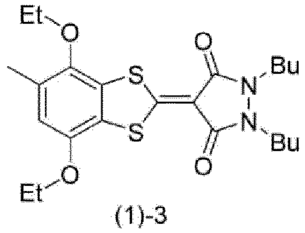
(1)-3

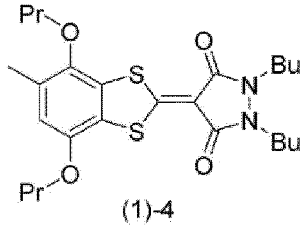
(1)-4

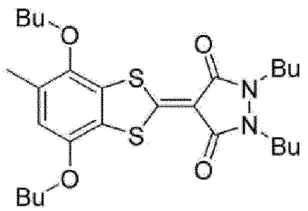
(1)-5

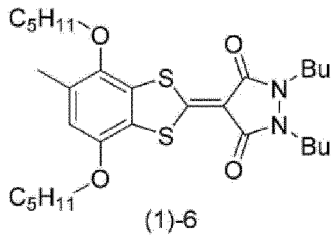
(1)-6

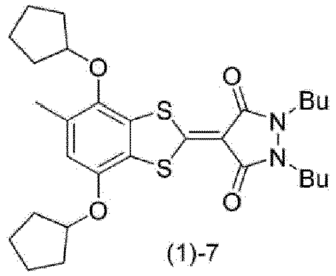
(1)-7

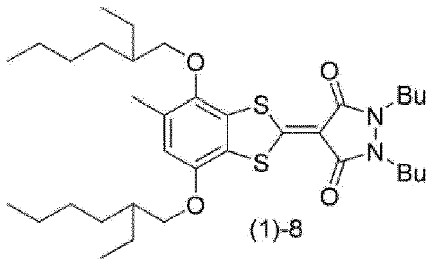
(1)-8

(Synthesis Example 3) (synthesis of compound (1)-9 and compound (1)-10)

Each of compounds (1)-9 and (1)-10 was synthesized by the same method as in Synthesis Example 1 except that 1,2-dimethyl-pyrazolidine-3,5-dione was used in place of the intermediate 1-2 in Synthesis Example 1.

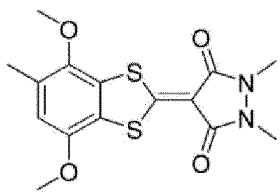
(1)-9

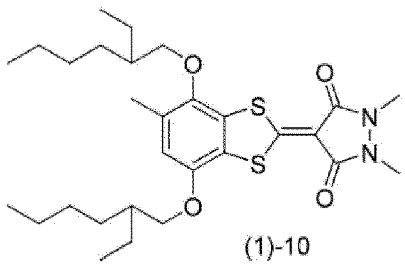
(1)-10

(Synthesis Example 4) (synthesis of compound (1)-11)

A compound (1)-11 was synthesized by the same method as in the same manner as in Synthesis Example 1 except that 2-tert-butyl-1,4-benzoquinone was used instead of p-toluquinone in Synthesis Example 1.

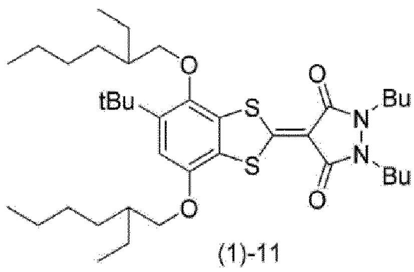
(1)-11

(Synthesis Example 5) (synthesis of compound (1)-12)

Compound (1) -12 was synthesized in the same manner as in Synthesis Example 1 except that 2-phenyl-1,4-benzoquinone was used in place of p-toluquinone in Synthesis Example 1.

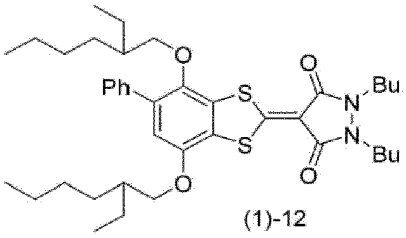

(1)-12

(Synthesis Example 6) (synthesis of compound (1)-13)

A compound (2)-40 was synthesized according to the following scheme by the same method as in Synthesis Example 1.

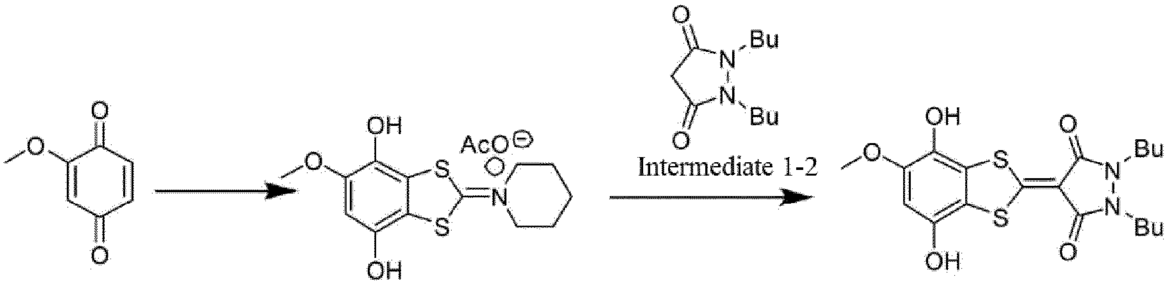


(2)-40

A compound (1)-13 was synthesized according to the following scheme by the same method as in Synthesis Example 1.

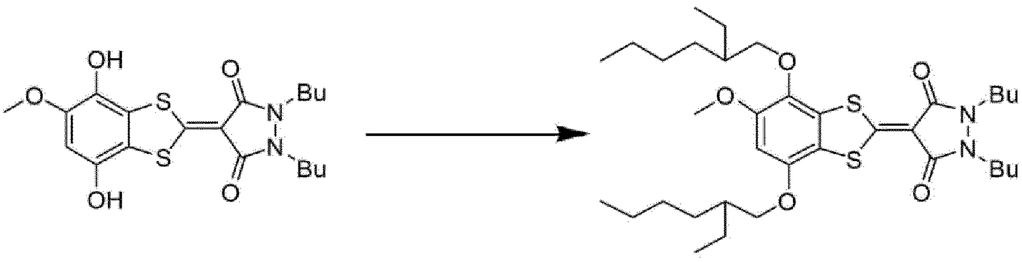

(2)-40

(1)-13

(Synthesis Example 7) (synthesis of compound (1)-14)

A compound (1)-14 was synthesized according to the following scheme with reference to the method described in paragraphs 0154 to 0155 of JP5376885B.

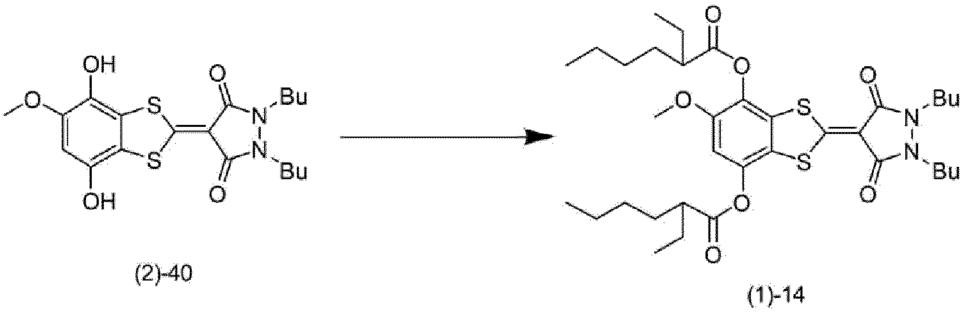

(2)-40 (1)-14

(Synthesis Example 8) (synthesis of compound (1)-15)

An intermediate 15-2 was synthesized according to the following scheme by the same method as in Synthesis Example 1. In the following scheme, the synthesis from the intermediate 1-1 to the intermediate 15-2 was performed using the intermediate 15-1 in place of the intermediate 1-2 used in Synthesis Example 1. Further, the intermediate 15-1 was synthesized by the method described in Journal of the American Chemical Society, 2009, Vol. 131, 33, pp. 11875 to 11881.

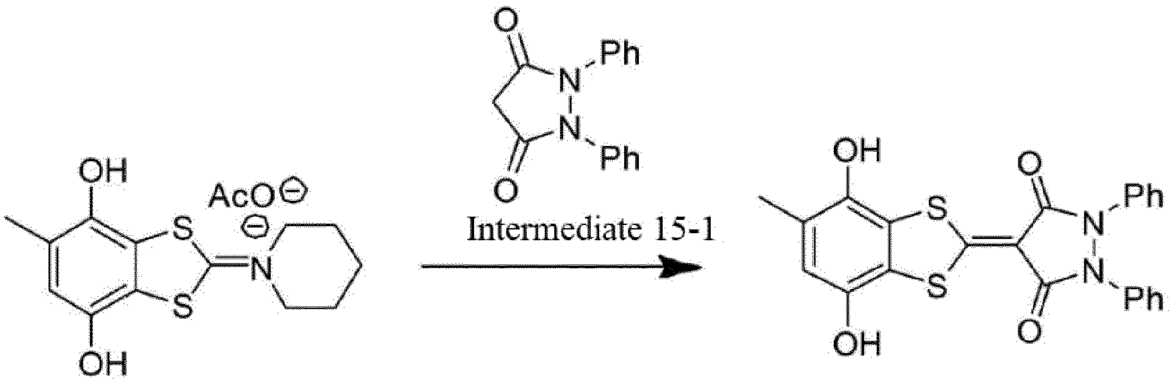


Intermediate 1-1 Intermediate 15-2

A compound (1)-15 was synthesized according to the following scheme by the same method as in Synthesis Example 1.

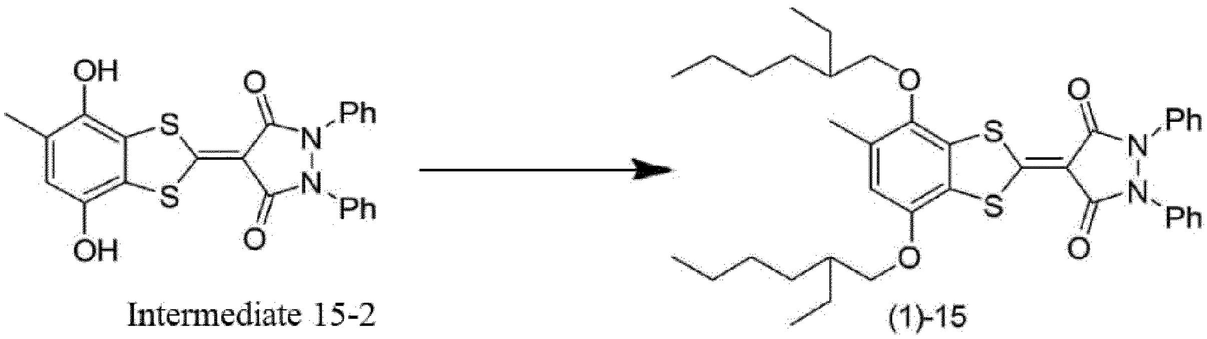

Intermediate 15-2 (1)-15

(Synthesis Example 9) (synthesis of compound (1)-16)

An intermediate 16-2 was synthesized according to the following scheme. In the following scheme, the synthesis from 1,4-naphthoquinone to the intermediate 16-2 was performed using 1,4-naphthoquinone in place of p-toluquinone used in Synthesis Example 1 and using the intermediate 16-1 in place of the intermediate 1-2.

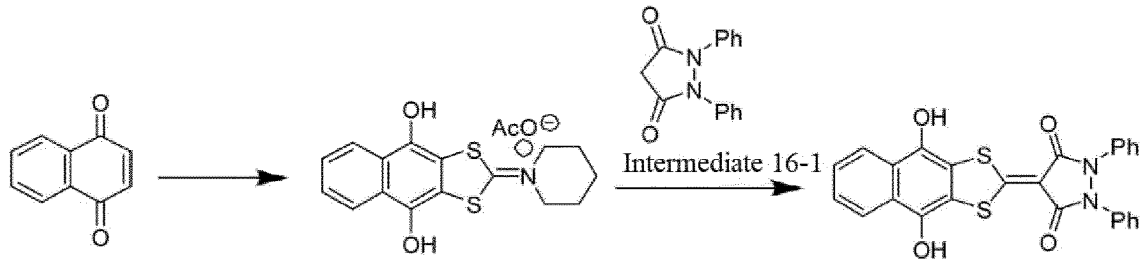


Intermediate 16-2

A compound (1)-16 was synthesized according to the following scheme by the same method as in Synthesis Example 1.

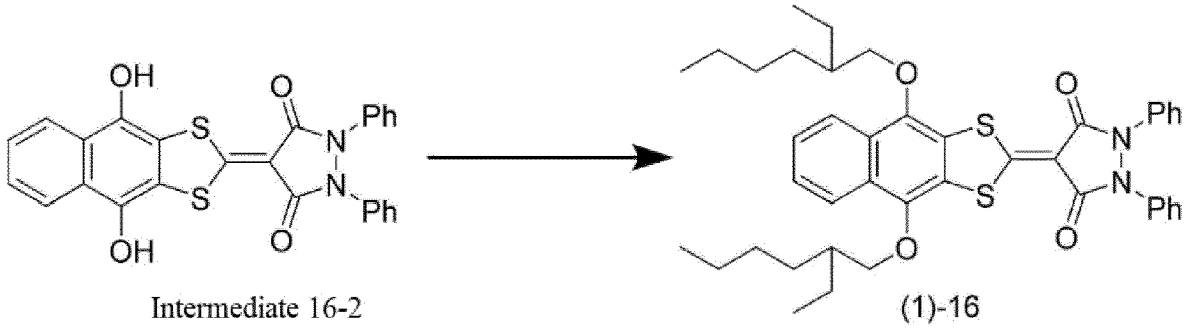

Intermediate 16-2 (1)-16

(Synthesis Example 10) (synthesis of compound (1)-37 and compound (1)-46)

A compound (2)-22 was synthesized according to the following scheme. In the following scheme, the synthesis from the intermediate 1-1 to the compound (2)-22 was performed using the intermediate 37-1 in place of the intermediate 1-2 used in Synthesis Example 1.

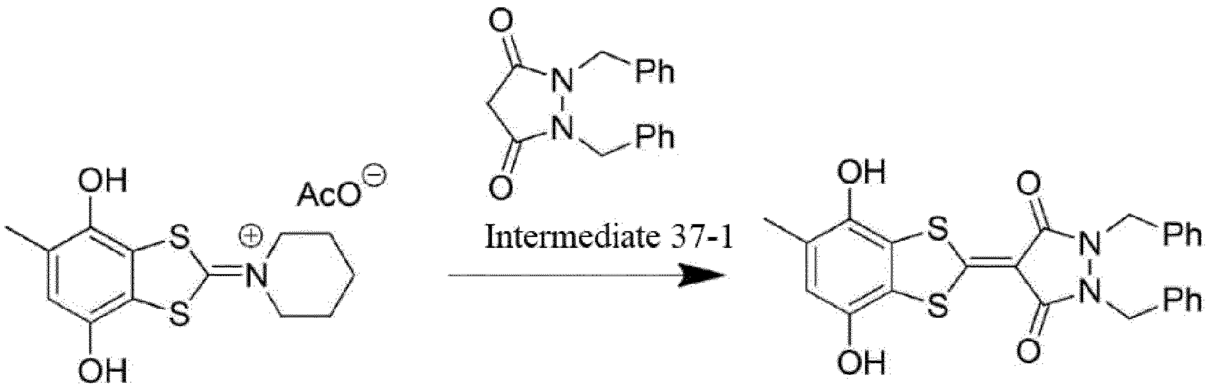


Intermediate 1-1 (2)-22

A compound (1)-37 and a compound (1)-46 were respectively synthesized by the same method as in Synthesis Example 1 except that the compound (2)-22 was used in place of the compound (2)-31 and the corresponding alkylating agent was used in place of 2-iodopropane in Synthesis Example 1.

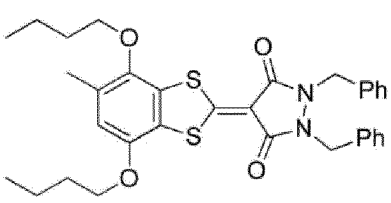

(1)-37

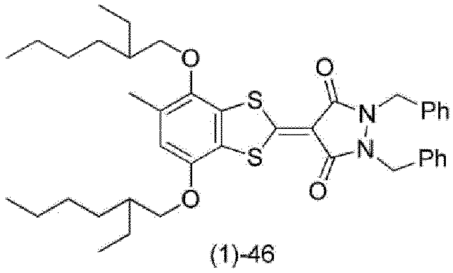

(1)-46

(Synthesis Example 11) (synthesis of compound (1)-47)

A compound (2)-1 was synthesized according to the following scheme. In the scheme below, the synthesis from 2,3-dimethyl-p-benzoquinone to the compound (2)-1 was performed using 2,3-dimethyl-p-benzoquinone in place of p-toluquinone used in Synthesis Example 1.

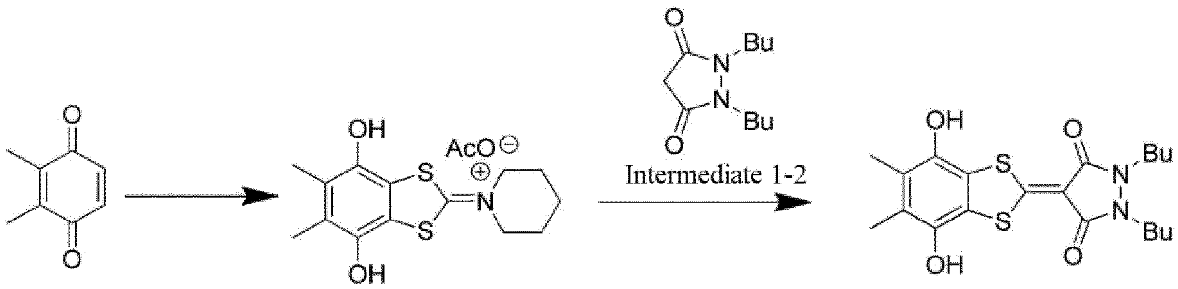


(2)-1

A compound (1)-47 was synthesized according to the following scheme by the same method as in Synthesis Example 1.

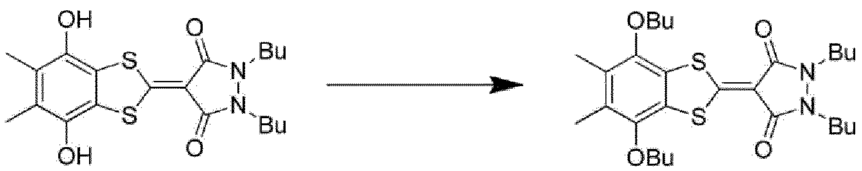

(2)-1 (1)-47

(Synthesis Example 12) (synthesis of compound (1)-48)

A compound (2)-54 was synthesized according to the following scheme. In the scheme below, the synthesis from 2,3-dimethyl-p-benzoquinone to the compound (2)-54 was performed using 2,3-dimethyl-p-benzoquinone in place of p-toluquinone used in Synthesis Example 1.

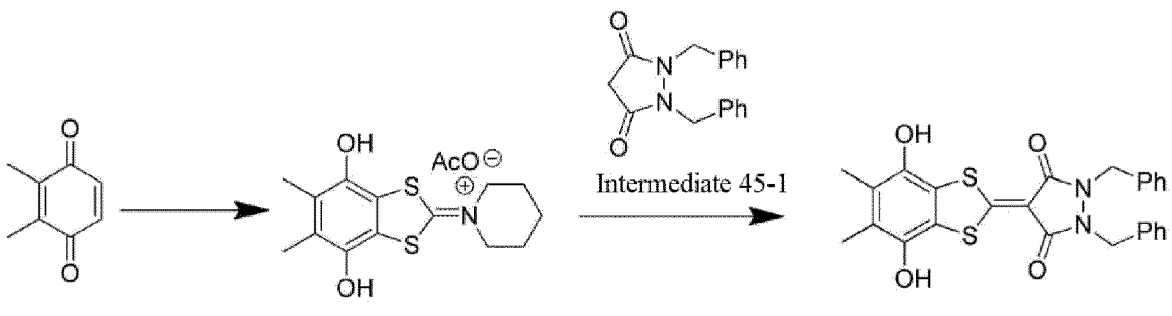


(2)-54

A compound (1)-48 was synthesized according to the following scheme by the same method as in Synthesis Example 1.

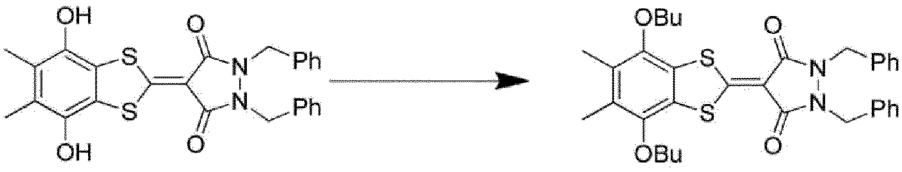

(2)-54 (1)-48

<Test Example 1>

Sample solutions 101 to 120 were prepared by dissolving 2 mg of the compounds listed in the table (the compound (1)-1 to the compound (1)-16, the compound (1)-37, the compound (1)-46, the compound (1)-47, the compound (1)-48, and the comparative compounds 1 to 4) in 100 mL of ethyl acetate and diluting the solution with ethyl acetate such that the absorbance of the solution was in a range of 0.6 to 1.2. Further, the compounds having the following structures were used as the comparative compounds 1 to 4.

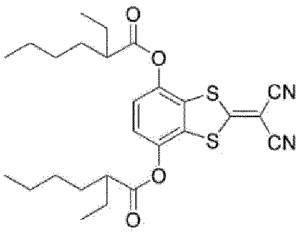

Comparative compound 1

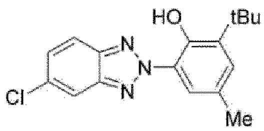

Comparative compound 2

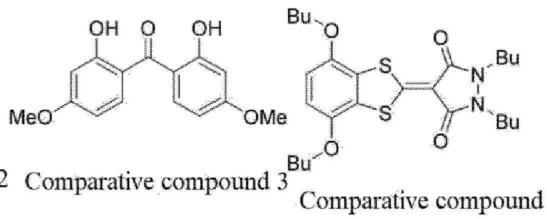

Comparative compound 3 Comparative compound 4

The absorbance of each of the sample solutions 101 to 120 was measured in a 1 cm quartz cell using a spectrophotometer UV-1800PC (manufactured by Shimadzu Corporation). The maximum absorption wavelength ($\lambda_{max}$) was measured from the spectral chart obtained for each sample solution. The values of $\lambda_{max}$ are listed in the table below.

Further, the molar absorption coefficient ($\varepsilon_{405}$) at a wavelength of 405 nm was calculated according to the following equation.

$$\varepsilon_{405} = \varepsilon_{max} \times (A_{405}/A_{max})$$

$\varepsilon_{405}$ represents the molar absorption coefficient of the sample solution at a wavelength of 405 nm, $\varepsilon_{max}$ represents the molar absorption coefficient of the sample solution at the maximum absorption wavelength, $A_{405}$ represents the absorbance of the sample solution at a wavelength of 405 nm, and $A_{max}$ represents the absorbance of the sample solution at the maximum absorption wavelength.

A case where the value of $\varepsilon_{405}$ was 3000 or greater was evaluated as A, a case where the value thereof was less than 3000 and 500 or greater was evaluated as B, and a case where the value thereof less than 500 was evaluated as C on a three level scale. The absorption ability at 405 nm increases as the value of $\varepsilon_{405}$ increases. The evaluation results are listed in the columns of $\varepsilon_{405}$ in Table 1.

Further, the value of the absorbance at a wavelength of 430 nm in a case where the absorbance of the sample solution at a wavelength of 405 nm was set to 1 was calculated, and the coloration was evaluated such that a case where the absorbance at a wavelength of 430 nm was less than 0.13 was evaluated as A and a case where the absorbance at a wavelength of 430 nm was 0.13 or greater was evaluated as B. The coloration is less as the value of the absorbance at a wavelength of 430 nm decreases. The results are listed in the columns of the coloration of Table 1.

[Table 1]

| Sample solution No. | Type of compound | $\lambda_{max}$ (nm) | $\varepsilon_{405}$ | Coloration |
|---|---|---|---|---|
| 101 | Compound (1)-1 | 383 | A | A |
| 102 | Compound (1)-2 | 381 | A | A |

(continued)

| Sample solution No. | Type of compound | $\lambda_{max}$ (nm) | $\varepsilon_{405}$ | Coloration |
|---|---|---|---|---|
| 103 | Compound (1)-3 | 383 | A | A |
| 104 | Compound (1)-4 | 383 | A | A |
| 105 | Compound (1)-5 | 383 | A | A |
| 106 | Compound (1)-6 | 383 | A | A |
| 107 | Compound (1)-7 | 384 | A | A |
| 108 | Compound (1)-8 | 383 | A | A |
| 109 | Compound (1)-9 | 381 | A | A |
| 110 | Compound (1)-10 | 382 | A | A |
| 111 | Compound (1)-11 | 383 | A | A |
| 112 | Compound (1)-12 | 383 | A | A |
| 113 | Compound (1)-13 | 388 | A | A |
| 114 | Compound (1)-14 | 382 | A | A |
| 115 | Compound (1)-15 | 393 | A | A |
| 116 | Compound (1)-16 | 388 | A | A |
| 117 | Compound (1)-37 | 386 | A | A |
| 118 | Compound (1)-46 | 387 | A | A |
| 119 | Compound (1)-47 | 385 | A | A |
| 120 | Compound (1)-48 | 388 | A | A |
| 121 | Comparative compound 1 | 350 | C | B |
| 122 | Comparative compound 2 | 352 | C | A |
| 123 | Comparative compound 3 | 350 | C | B |
| 124 | Comparative compound 4 | 380 | B | A |

As listed in the table, the compounds (1)-1 to (1)-16, the compound (1)-37, the compound (1)-46, the compound (1)-47, and the compound (1)-48 had a high molar absorption coefficient ($\varepsilon_{405}$) at a wavelength of 405 nm, and thus the coloration was less.

<Test Example 2-1>

The compounds listed in the table below, 7.6 g of chloroform and 1.1 g of a (meth)acrylic resin (DIANAL BR-80, manufactured by Mitsubishi Chemical Corporation, containing 60% by mass or greater of methyl methacrylate as a monomer unit, Mw of 95000) were mixed to prepare a resin composition. A glass substrate was spin-coated with the obtained resin composition to form a coating film, and the obtained coating film was dried at 110°C for 2 minutes, thereby preparing a resin film. In addition, the compound (1)-1, the compound (1)-2, the compound (1)-8, the compound (1)-11, the compound (1)-12, the compound (1)-13, the compound (1)-14, the compound (1)-5, the compound (1)-37, the compound (1)-46, the compound (1)-47, the compound (1)-48, and the comparative compounds 1 to 4 listed in the columns of the type of compound in Table 2 are respectively compounds having the above-described structures.

(Evaluation of surface unevenness)

The resin film prepared above was observed with an optical microscope (MX-61L, manufactured by Olympus Corporation) at a bright field of view of 200 magnifications, and the resin film was observed to confirm the presence of unevenness. In a case where the film was uniform with no unevenness confirmed by an optical microscope, it is determined that the film has excellent resistance to the thermal stress during film formation. The evaluation results of surface unevenness are listed in Table 2.

A: No unevenness was found with an optical microscope.
B: Slight unevenness was found with an optical microscope.
C: Significant unevenness was found with an optical microscope.

(Light resistance)

The light resistance was evaluated by performing a light resistance test on the prepared resin film under the following condition 1 and acquiring the retention rate of the absorbance at the maximum absorption wavelength ($\lambda_{max}$). Specifically, after measurement of the absorbance of the resin film at the maximum absorption wavelength ($\lambda_{max}$), the resin film was subjected to the light resistance test for one week under the condition 1, and the absorbance of the resin film after the light resistance test at the maximum absorption wavelength ($\lambda_{max}$) was measured.

(Condition 1)

Device: Xenon Weather Meter (XL75, manufactured by Suga Test Instruments Co., Ltd.)
Illuminance: 10 klx (40w/$m^2$)
Test period: 1 week
Environment: 23°C at relative humidity of 50%

The retention rate (%) of the absorbance was calculated from the value of the absorbance of the resin film before and after the light resistance test at the maximum absorption wavelength ($\lambda_{max}$) according to the following equation. The retention rate was calculated according to the following equation.

Retention rate (%) of absorbance = 100 × (absorbance of resin film after irradiation at $\lambda_{max}$)/(absorbance of resin film before irradiation at $\lambda_{max}$)

A: The retention rate of the absorbance was 90% or greater.
B: The retention rate of the absorbance was 80% or greater and less than 90%.
C: The retention rate of the absorbance was less than 80%.

In addition, the degree of a change in the coloration of the resin film after the light resistance test was visually confirmed. The evaluation results are listed in Table 2.

[Table 2]

| Resin film No. | Type of compound | Blending amount (mg) | Surface unevenness | Light resistance | |
|---|---|---|---|---|---|
| | | | | Retention rate of absorbance | Presence of coloration |
| 201a | Compound (1)-1 | 10 | A | A | Coloration was not found |
| 202a | Compound (1)-2 | 10 | A | A | Coloration was not found |
| 203a | Compound (1)-8 | 10 | A | A | Coloration was not found |
| 204a | Compound (1)-11 | 10 | A | A | Coloration was not found |
| 205a | Compound (1)-12 | 10 | A | A | Coloration was not found |
| 206a | Compound (1)-13 | 10 | A | A | Coloration was not found |
| 207a | Compound (1)-14 | 10 | A | A | Coloration was not found |
| 208a | Compound (1)-5 | 10 | A | A | Coloration was not found |
| 209a | Compound (1)-37 | 10 | A | A | Coloration was not found |
| 210a | Compound (1)-46 | 10 | A | A | Coloration was not found |
| 211a | Compound (1)-47 | 10 | A | A | Coloration was not found |
| 212a | Compound (1)-48 | 10 | A | A | Coloration was not found |

(continued)

| Resin film No. | Type of compound | Blending amount (mg) | Surface unevenness | Light resistance | |
|---|---|---|---|---|---|
| | | | | Retention rate of absorbance | Presence of coloration |
| 213a | Comparative compound 1 | 10 | B | B | Coloration was found |
| 214a | Comparative compound 2 | 10 | C | A | Coloration was found |
| 215a | Comparative compound 3 | 10 | C | C | Coloration was found |
| 216a | Comparative compound 4 | 10 | B | A | Coloration was found |

The resin films 201a to 212a had larger absorption in the vicinity of a wavelength of 400 nm than the resin films 213a to 216a and were excellent in absorbency of ultraviolet rays on a long wavelength side. Further, the resin films 201a to 212a were less colored. That is, the resin films 201a to 212a had large absorption in the vicinity of a wavelength of 400 nm and were less colored.

Further, as listed in the table, the resin films 201a to 212a had less surface unevenness and further had excellent light resistance.

Further, the storage stability of the resin composition was improved by allowing the resin composition used for forming the resin films 20a1 to 212a to contain the compound (2) (compound represented by Formula (2)) described in the present specification described above, and thus precipitation of the compound (1) was not found even in a case where a resin film was formed by using, for example, the resin composition after storage at 5°C for 2 weeks.

<Test Example 2-2>

The compounds listed in the table below, 7.6 g of chloroform and 1.1 g of a (meth)acrylic resin (DIANAL BR-80, manufactured by Mitsubishi Chemical Corporation, containing 60% by mass or greater of methyl methacrylate as a monomer unit, Mw of 95000) were mixed to prepare a resin composition. A glass substrate was spin-coated with the obtained resin composition to form a coating film, and the obtained coating film was dried at 110°C for 2 minutes, thereby preparing a resin film. Among the compounds listed in the columns of the type of the compounds of Table 3, the compound (1)-1, the compound (1)-5, and the compound (1)-8 are respectively compounds having the above-described structures. Further, UV-1 to UV-6 are compounds having the following structure.

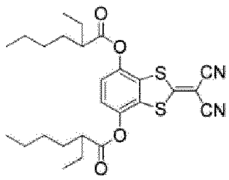
UV-1

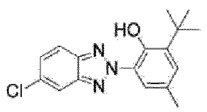
UV-2

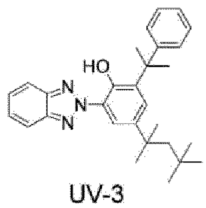
UV-3

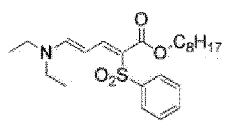
UV-4

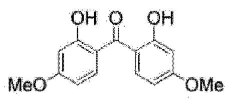
UV-5

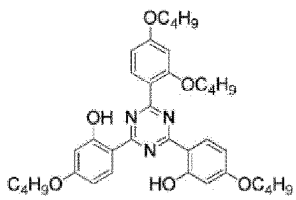
UV-6

(Evaluation of surface unevenness)

The surface unevenness of the resin film prepared above was evaluated by the same method according to the same evaluation standards as in Test Example 2-1.

(Light resistance 2 (retention rate of absorbance at 405 nm))

The resin film prepared above was subjected to the light resistance test under the condition 1 of Test Example 2-1 to acquire the retention rate of the absorbance at a wavelength of 405 nm, and the light resistance 2 was evaluated. Specifically, after measurement of the absorbance of the resin film at a wavelength of 405 nm, the resin film was subjected to the light resistance test for one week under the condition 1, and the absorbance of the resin film after the light resistance test at a wavelength of 405 nm was measured. The retention rate (%) of the absorbance was calculated from the value of the absorbance of the resin film at 405 nm before and after the light resistance test according to the following equation. The retention rate was calculated according to the following equation.

Retention rate (%) of absorbance = 100 × (absorbance of resin film after irradiation at wavelength of 405 nm)/(absorbance of resin film before irradiation at wavelength of 405 nm)

A: The retention rate of the absorbance was 90% or greater.
B: The retention rate of the absorbance was 80% or greater and less than 90%.
C: The retention rate of the absorbance was less than 80%.

[Table 3]

| Resin film No. | Type of compound | Blending amount (mg) | Surface unevenness | Light resistance 2 |
|---|---|---|---|---|
| 201b | Compound (1)-1 UV-6 | 20<br>20 | A | A |
| 202b | Compound (1)-1 UV-1 | 30<br>20 | A | A |
| 203b | Compound (1)-5 UV-2 | 10<br>20 | A | A |
| 204b | Compound (1)-5 UV-4 | 20<br>20 | A | A |
| 205b | Compound (1)-8 UV-3 | 30<br>40 | A | A |
| 206b | Compound (1)-8 UV-5 | 10<br>10 | A | A |

The resin films 201b to 206b had large absorption in the vicinity of a wavelength of 400 nm and were excellent in absorbency of ultraviolet rays on a long wavelength side. In addition, the retention rate of the absorbance at 405 nm after the light resistance test was also satisfactory, and the light resistance was excellent.

<Test Example 3>

The resin film 301 was prepared by the method described below.

(1) Preparation of raw material

(1-1) Preparation of cellulose acylate

Cellulose acylate with an acetyl substitution degree of 2.85 was prepared. Sulfuric acid (7.8 parts by mass with respect to 100 parts by mass of cellulose) was added as a catalyst, each carboxylic acid was added, and an acylation reaction was carried out at 40°C. Thereafter, the total degree of substitution and the degree of substitution at the 6-position were adjusted by adjusting the amount of the sulfuric acid catalyst, the amount of moisture, and the aging time. The aging was performed at a temperature of 40°C. Further, low-molecular-weight components of the cellulose acylate were washed with acetone to be removed.

(1-2) Preparation of sugar ester compound

A sugar ester compound was prepared by the following method.

First, the sugar ester compound 1 having the following structure was synthesized by the method described in the synthesis of the exemplary compound 3 in paragraph 0054 of WO2009/003164A. Further, a sugar ester compound 2 was also synthesized by the same method.

(2) Preparation of dope

The following composition was put into a mixing tank, stirred to dissolve each component, further heated to 80°C for approximately 180 minutes, and filtered through a filter paper having an average pore size of 34 μm and a sintered metal filter having an average pore size of 10 μm.

(Composition of dope)

Cellulose acylate (degree of substitution: 2.85): 100.0 parts by mass
Sugar ester 1: 7.5 parts by mass
Sugar ester 2: 2.5 parts by mass
Ultraviolet absorbing agent 1 (compound (1)-5): 0.1 parts by mass
Ultraviolet absorbing agent (comparative compound 5): 0.1 parts by mass
Methylene chloride: 475.3 parts by mass
Methanol: 103.9 parts by mass
Butanol: 4.6 parts by mass

The concentration of solid contents of the dope was 16.0% by mass, the addition amount of the plasticizer was the ratio to the cellulose ester, and the solvent of the dope was methylene chloride/methanol/butanol = 81/18/1 (mass ratio). The film thickness was 60 μm.

The specific structure of the sugar ester is shown below. A compound with the following structure in which the addition amount of the sugar ester 1 was 7.5% by mass and the average degree of substitution was 5.5 was used. The degree of substitution was calculated by performing measurement according to high performance liquid chromatography (HPLC). The addition amount of the sugar ester 2 was 2.5% by mass, and the amount of the compound (1)-5 was 0.1% by mass.

Sugar ester 1

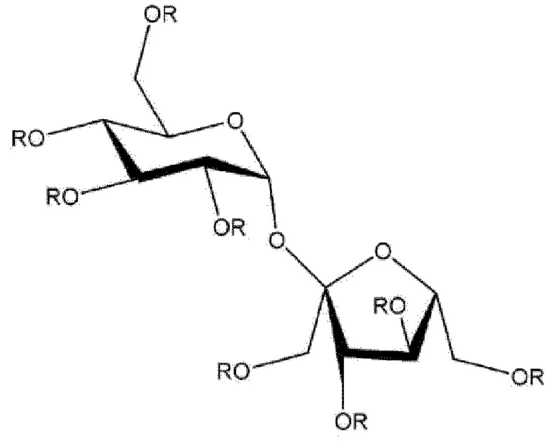

R= 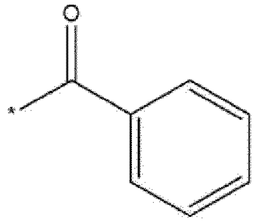 or H

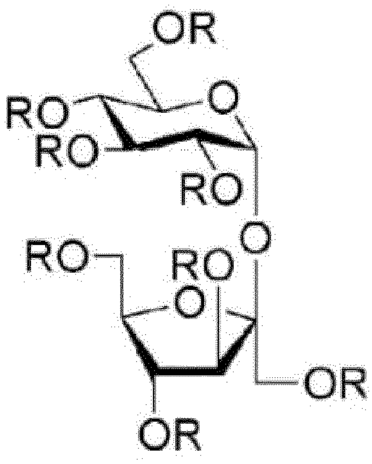

Sugar ester 2 R=Ac/i-Butylate(2/6)

As listed in the table below, each dope of the examples and the comparative examples was prepared in the same manner as that for the dope of the resin film 301A except that the addition amount of the ultraviolet absorbing agent 1 and the addition amount of the ultraviolet absorbing agent 2 were changed as listed in the table below.

(3) Casting

The above-described dope was cast using a drum film forming machine. The dope of the core layer was co-cast from the die so that the dope of the surface layer was on the dope of the core layer to be in contact with the metal support cooled to -10°C, gelled, and peeled off. The drum was made of stainless steel.

(4) Drying

The web (film) obtained by casting was peeled off from the drum and dried in a tenter device for 20 minutes using a tenter device such that both ends of the web were clipped with clips and transported at 30°C to 40°C during film transport. Thereafter, the dried web was framed and post-dried at 140°C. The drying temperature here denotes the film surface temperature of the film.

(5) Winding

A film having the composition listed in the table below was prepared, and at least 24 rolls having a roll width of 1280 mm and a roll length of 2600 mm were prepared under the above-described conditions for the purpose of determining

the manufacturing suitability thereof. For one of the 24 rolls produced continuously, the measurement was performed by cutting out a sample (width of 1280 mm) having a length of 1 m at intervals of 100 m. The obtained cellulose acylate films were used as resin films 301 to 309 of each example and each comparative example.

The absorbance of each of the resin films 301A to 309A prepared above was measured using a spectrophotometer UV3600 (manufactured by Shimadzu Corporation).

Further, the ultraviolet absorption ability on a long wavelength side was evaluated such that a case where the value obtained by dividing the absorbance at a wavelength of 405 nm by the addition amount of the ultraviolet absorbing agent (% by mass with respect to cellulose acylate) was 50 or greater was evaluated as A, a case where the value thereof was in a range of 25 to 50 was evaluated as B, and a case where the value thereof was 25 or less was evaluated as C. The ultraviolet absorption ability on a long wavelength side is higher as the value increases. The results are listed in the columns of the ultraviolet absorption ability on a long wavelength side in the table below.

[Table 4]

| Resin film No. | Ultraviolet absorbing agent 1 | Ultraviolet absorbing agent 2 | Addition amount of ultraviolet absorbing agent 1 (% by mass) | Addition amount of ultraviolet absorbing agent 2 (% by mass) | Ultraviolet absorption ability on long wavelength side |
|---|---|---|---|---|---|
| 301A | Compound (1)-5 | - | 0.1 | 0 | A |
| 302A | Compound (1)-5 | - | 0.3 | 0 | A |
| 303A | Compound (1)-5 | - | 0.6 | 0 | A |
| 304A | Compound (1)-5 | Comparative compound 5 | 0.6 | 1.9 | A |
| 305A | Compound (1)-5 | Comparative compound 5 | 0.9 | 1.9 | A |
| 306A | Compound (1)-5 | Comparative compound 5 | 1.3 | 1.9 | A |
| 307A | - | Comparative compound 5 | 0 | 0.2 | C |
| 308A | - | Comparative compound 5 | 0 | 0.6 | C |
| 309A | - | Comparative compound 5 | 0 | 1.9 | C |

The compound (1)-5 used as the ultraviolet absorbing agent 1 is a compound having the above-described structure. Further, the comparative compound 5 used as the ultraviolet absorbing agent 2 is a compound having the following structure.

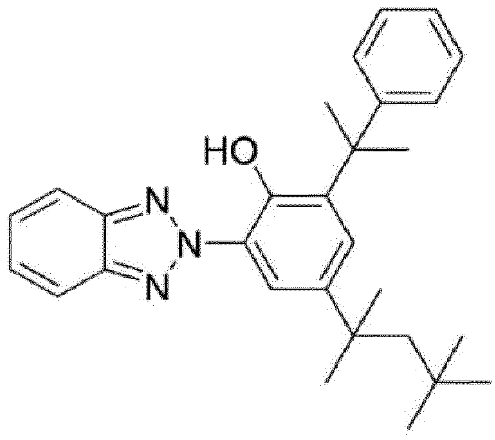

Comparative compound 5

The resin films 301A to 306A had larger light absorption in the vicinity of a wavelength of 400 nm than the resin films 307A to 309A and were excellent in absorbency of ultraviolet rays on a long wavelength side.

(6) Preparation of polarizing plate

A polarizing plate was prepared by the method described below.

(6-1) Saponification treatment of resin film

The prepared resin films 303A, 304A, and 308A were immersed in a 2.3 mol/L sodium hydroxide aqueous solution at 55°C for 3 minutes. The resin films were washed in a water washing bath at room temperature (25°C) and neutralized at 30°C with 0.05 mol/L sulfuric acid. The resin films were washed again in a water washing bath at room temperature and further dried with warm air at 100°C

(6-2) Preparation of polarizer

A polyvinyl alcohol (PVA) film having a thickness of 80 μm was immersed in an iodine aqueous solution having an iodine concentration of 0.05% by mass at 30°C for 60 seconds, dyed, longitudinally stretched to 5 times the original length during immersion in a boric acid aqueous solution having a boric acid concentration of 4% by mass for 60 seconds, and dried at 50°C for 4 minutes, thereby obtaining a polarizer having a thickness of 19 μm.

(6-3) Preparation of vertical alignment (VA) phase difference film

The following VA phase difference film 1 or VA phase difference film 2 was used as the phase difference film.

(6-3-1) Preparation of VA phase difference film 1

<Synthesis of cycloolefin polymer A>

A nitrogen-substituted reaction container was charged with 72.5 parts by mass of 8-methoxycarbonyl-8-methyltetracyclo[4.4.0.1$^{2,5}$.1$^{7,10}$]-3-dodecene, 27.5 parts by mass of dicyclopentadiene, 5.6 parts by mass of 1-hexene as a molecular weight adjuster, and 200 parts by mass of toluene and heated to 80°C. 0.18 ml of a toluene solution of triethylaluminum (0.6 mol/L) and 0.58 ml of a toluene solution of methanol-modified $WCl_6$ (0.025 mol/L) were added thereto, and the mixture was allowed to react at 80°C for 3 hours, thereby obtaining a ring-opening polymer. Thereafter, the obtained solution of the ring-opening polymer was put into an autoclave, and 200 parts by mass of toluene was further added thereto. 2500 ppm of $RuHCl(CO)[P(C_6H_5)]_3$, which is a hydrogenation catalyst, was added thereto with respect to the amount of monomer charged, and the hydrogen gas pressure was set to 9 to 10 MPa for reaction at 160°C to 165°C for 3 hours. After completion of the reaction, a hydrogenated material (cycloolefin-based polymer A) of a ring-opening polymer was obtained by precipitating in a large amount of the methanol solution. The hydrogenated material of the obtained ring-opening polymer had a weight-average molecular weight (Mw) of $119 \times 10^3$ and a molecular weight distribution (Mw/Mn) of 3.1.

<Preparation of fine particle dispersion liquid>

11 parts by mass of fine particles (AEROSIL R812, manufactured by Nippon Aerosil Co., Ltd.) and 89 parts by mass of ethanol were stirred and mixed with a dissolver for 50 minutes and dispersed with Manton Goulin, thereby preparing a fine particle dispersion liquid.

<Preparation of fine particle additive liquid>

4 parts by mass of the cycloolefin-based polymer A was added to a dissolution tank containing 99 parts by mass of methylene chloride and heated for complete dissolution, and 11 parts by mass of the fine particle dispersion liquid was slowly added thereto while the mixture was sufficiently stirred, and dispersed with an attritor. A fine particle additive liquid was prepared by filtering the mixture with a filter (FINEMET NF, Nippon Seisen Co., Ltd.).

<Preparation of VA phase difference film 1>

First, methylene chloride and methanol were added to the pressurization dissolution tank. The cycloolefin polymer A was put into the pressurization dissolution tank containing a solvent while being stirred. The mixture was heated and stirred for complete dissolution, thereby preparing a main dope liquid. 2 parts by mass of the fine particle additive liquid was added to 100 parts by mass of the main dope liquid, the mixture was sufficiently mixed with an in-line mixer (Toray

stationary in-pipe mixer Hi-Mixer, SWJ), and the mixture was uniformly cast on a stainless steel band support with a width of 2 m using a belt casting device. With respect to the obtained web (film), the solvent was evaporated until the amount of the residual solvent reached 110% by mass, and the web (film) was peeled off from the stainless steel band support. After the peeling, a tension was applied to stretch the film such that the longitudinal stretching ratio was set to 2%. Thereafter, the film was dried until the amount of the residual solvent in the film was less than 1% by mass and further stretched by 35% at 165°C in a direction orthogonal to the film transport direction using a tenter. The amount of the residual solvent was calculated according to the following equation.

$$\text{Amount of residual solvent (\% by mass)} = \{(M - N)/N\} \times 100$$

Here, M represents the mass of the web at an optional time point, and N represents the mass of the web used for measuring M after drying the web at 120°C for 2 hours.

As described above, a VA phase difference film 1 having a film thickness of 35 μm and an Rth of 121 nm, with knurling having a width of 1.5 m, a width of 1 cm at an end portion, and a height of 8 μm was prepared.

(6-3-2) Preparation of VA phase difference film 2

<Creation of cellulose acylate>

Cellulose acylate was synthesized by the method described in JP1998-045804A (JP-H10-405804A) and JP1996-231761A (JP-H08-231761A), and the acyl group substitution degree was measured. Specifically, sulfuric acid (7.8 parts by mass with respect to 100 parts by mass of cellulose) was added as a catalyst, carboxylic acid (acetic acid) as a raw material for an acyl group was added, and an acylation reaction was carried out at 40°C. Here, the degree of substitution of the acyl group (acetyl group) was adjusted by adjusting the amount of the carboxylic acid. After acylation, aging was performed at 40°C. Further, low-molecular-weight components of the cellulose acylate (cellulose acetate) were washed with acetone to be removed, thereby obtaining cellulose acylates having various average acyl group substitution degrees.

<Synthesis of additive A>

An additive A was synthesized by a method similar to or in conformity with the method described in JP6095766B. The structural formula of the synthesized compound is shown below.

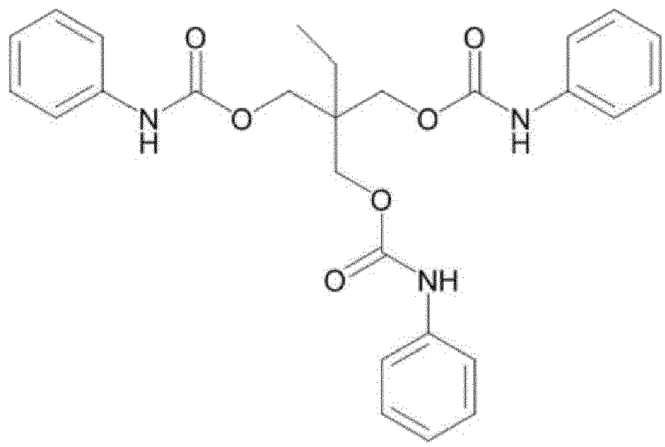

<Synthesis of additive B>

An additive B was synthesized by a method similar to or in conformity with the method described in WO2015/005398A. The structural formula of the synthesized compound is shown below.

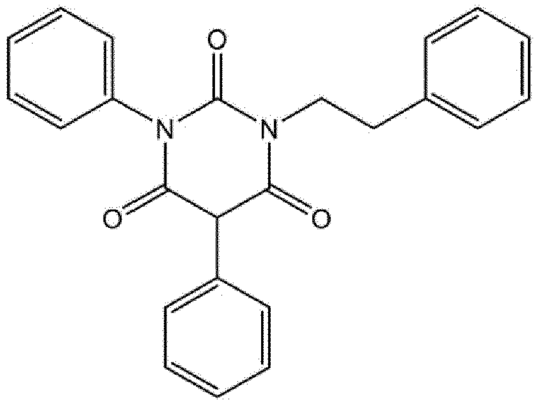

<Synthesis of additive C>

An additive C was synthesized by a method similar to or in conformity with the method described in JP4260332B. The structural formula of the synthesized compound is shown below.

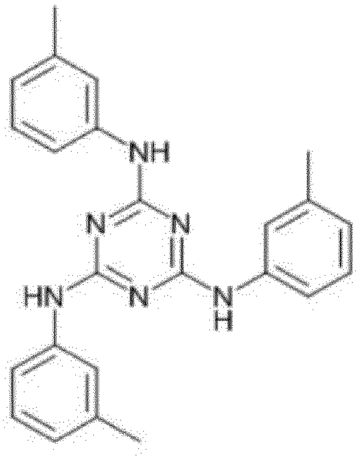

<Preparation of dope for forming core layer>

The following composition was put into a mixing tank and stirred to dissolve each component, thereby preparing a dope for forming a core layer.

(Composition of dope for forming core layer)

Cellulose acetate (degree of substitution: 2.4): 100.0 parts by mass
Additive A: 12.0 parts by mass
Additive B: 3.5 parts by mass
Additive C: 1.0 part by mass
Methylene chloride: 392.0 parts by mass
Methanol: 58.5 parts by mass

<Preparation of dope for forming skin layer>

The following composition was put into a mixing tank and stirred to dissolve cellulose acetate, thereby preparing a dope for forming a skin layer.

(Composition of dope for forming skin layer)

Cellulose acetate (degree of substitution: 2.8): 100 parts by mass
Methylene chloride: 440 parts by mass
Methanol: 65.8 parts by mass

<Casting of VA phase difference film 2>

The prepared dope for forming a core layer and the prepared dope for forming a skin layer were co-cast such that three layers of a skin layer, a core layer, and a skin layer were laminated in this order using a band casting device. The film thickness of the core layer after drying was 39 μm, and the film thickness of each skin layer was 1 μm. The obtained film (web) was peeled off from the band, sandwiched between clips, and laterally stretched (stretched in the width direction) at a stretching ratio of 1.1 times at 140°C using a tenter in a state where the amount of the residual solvent was in a range of 5% to 20% by mass with respect to the total mass of the film. Thereafter, the clips were removed from the film and dried at 140°C for 20 minutes, and the film was further laterally stretched at a glass transition temperature (Tg) of -3°C and a stretching ratio of 1.2 times using a tenter, thereby preparing a VA phase difference film 2. The film thickness of the obtained VA phase difference film 2 was 40 μm. In addition, "stretching ratio (%)" denotes a ratio obtained by the following equation.

$$\text{Stretching ratio (\%)} = 100 \times \{(\text{length after stretch}) - (\text{length before stretch})\}/\text{length before stretch}$$

Further, Tg is a temperature at which the loss tangent δ acquired by dynamic viscoelasticity measurement is a maximum value. The loss direct contact tan δ is obtained by measuring E" (loss elastic modulus) and E' (storage elastic modulus) with respect to a film sample whose humidity was adjusted in advance at 25°C and a relative humidity of 60% for 2 hours or longer using a dynamic viscoelasticity measuring device (DVA-200, manufactured by IT Measurement Control Co., Ltd.) under the following conditions, the tan δ (= E"/E') and the maximum value were acquired, and the Tg was measured.

Device: DVA-200, manufactured by IT Measurement Control Co., Ltd.
Sample: 5 mm, length of 50 mm (gap of 20 mm)
Measurement conditions: tension mode
Measurement temperature: -25°C to 220°C
Heating conditions: 5°C/min
Frequency: 1 Hz

Further, the amount of the residual solvent was acquired according to the following equation.

$$\text{Amount of residual solvent (\% by mass)} = \{(M - N)/N\} \times 100$$

M represents the mass of the web at any time point, and N represents the mass of the web used for measuring M after drying the web at 120°C for 2 hours.

(6-4) Preparation of VA phase difference film 1 with polarizer

<Preparation of ultraviolet curable adhesive 1>

An ultraviolet curable adhesive was prepared with the composition shown below.

(Composition of ultraviolet curable adhesive 1)

CELLOXIDE 2021P (manufactured by Daicel Corporation, polyfunctional epoxy compound): 100.0 parts by mass
RIKARESIN DME-100 (New Japan Chemical Co., Ltd., polyfunctional epoxy compound): 28.6 parts by mass
2-Ethylhexyl glycidyl ether (monofunctional epoxy compound): 14.3 parts by mass
CPI-100P (photoacid generator, manufactured by San-Apro Ltd.): 2.9 parts by mass

Irgacure 290 (photoacid generator, manufactured by BASF SE): 5.7 parts by mass
2-Isopropylthioxanthone (photoacid generator): 1.4 parts by mass

<Adhesion of VA phase difference film 1 to polarizer>

The above-described VA phase difference film 1 was attached to one side of the polarizer using the ultraviolet curable adhesive 1 and irradiated with ultraviolet rays at an intensity of 200 mJ from the VA phase different film 1 side 5 seconds after attachment, and the ultraviolet curable adhesive 1 was cured, thereby obtaining a VA phase difference film 1 with a polarizer.

(6-5-1) Production of polarizing plate 303B1

The resin film 303A subjected to the saponification treatment was attached to the VA phase difference film 1 with a polarizer on the polarizer side using a polyvinyl alcohol-based adhesive, thereby preparing a polarizing plate 303B1.

(6-5-2) Production of polarizing plate 303B2

The resin film 303A subjected to the saponification treatment was attached to one side of the polarizer using a polyvinyl alcohol-based adhesive, and the VA phase difference film 2 was attached to the surface of the polarizer opposite to the side where the resin film 303A was attached, thereby preparing a polarizing plate 303B2.

(6-6-1) Production of liquid crystal display devices 303C1, 304C1, and 308C1

A liquid crystal panel of a commercially available liquid crystal display device FlexScan 19-inch color liquid crystal monitor S1923-HBK (trade name, manufactured by EIZO Corporation) was taken out, the polarizing plate on the front side was peeled off, and the polarizing plate 303B1 on the VA phase difference film side was attached thereto in place of the peeled polarizing plate via a pressure sensitive adhesive (SK-2057, manufactured by Soken Chemical & Engineering Co., Ltd.). In this manner, a liquid crystal display device 303C1 was produced. Liquid crystal displays 304C1 and 308C1 were produced in the same manner except that the kind of the resin film was changed as listed in the table below.

(6-6-2) Production of liquid crystal display devices 303C2, 304C2, and 308C2

A liquid crystal panel of a commercially available liquid crystal display device FlexScan 19-inch color liquid crystal monitor S1923-HBK (trade name, manufactured by EIZO Corporation) was taken out, the polarizing plate on the front side was peeled off, and the polarizing plate 303B2 on the VA phase difference film side was attached thereto in place of the peeled polarizing plate via a pressure sensitive adhesive (SK-2057, manufactured by Soken Chemical & Engineering Co., Ltd.). In this manner, the liquid crystal display device 303C2 was produced. Liquid crystal displays 304C2 and 308C2 were produced in the same manner except that the kind of the resin film was changed as listed in the table below.

[Table 5]

| Liquid crystal display device No. | Polarizing plate No. | Resin film No. | VA phase difference film No. | Fig. No. |
|---|---|---|---|---|
| 303C1 | 303B1 | 303A | 1 | Fig. 1 |
| 304C1 | 304B1 | 304A | 1 | Fig. 1 |
| 308C1 | 308B1 | 308A | 1 | Fig. 1 |
| 303C2 | 303B2 | 303A | 2 | Fig. 1 |
| 304C2 | 304B2 | 304A | 2 | Fig. 1 |
| 308C2 | 308B2 | 308A | 2 | Fig. 1 |

The liquid crystal display devices 303C1, 303C2, 304C1, and 304C2 containing the ultraviolet absorbing agent according to the embodiment of the present invention were not preferable because a change in image quality was small even in a case of a display for a long time as compared with the liquid crystal display devices 308C1 and 308C2.

(7) Preparation of organic electroluminescence display device

An organic electroluminescence display device was prepared by the method described below.

(7-1) Preparation of optically anisotropic layer A

(Preparation of cellulose acylate solution)

The following composition was put into a mixing tank and stirred while being heated to dissolve each component, thereby preparing a cellulose acylate solution.

(Composition of cellulose acylate solution)

Cellulose acetate (degree of acetylation: 2.86): 100 parts by mass
Methylene chloride (first solvent): 320 parts by mass
Methanol (second solvent): 83 parts by mass
1-Butanol (third solvent): 3 parts by mass
Triphenyl phosphate: 7.6 parts by mass
Biphenyl diphenyl phosphate: 3.8 parts by mass

(Preparation of matting agent dispersion liquid)

The following composition was put into a disperser and stirred to dissolve each component, thereby preparing a matting agent dispersion liquid.

(Composition of matting agent dispersion liquid)

Silica particle dispersion liquid (average particle diameter of 16 nm, AEROSIL R972, manufactured by Nippon Aerosil Co., Ltd.): 10.0 parts by mass
Methylene chloride: 72.8 parts by mass
Methanol: 3.9 parts by mass
Butanol: 0.5 parts by mass
Cellulose acylate solution: 0.3 parts by mass

(Preparation of ultraviolet absorbing agent solution)

The following composition was put into a mixing tank and stirred while being heated to dissolve each component, thereby preparing an ultraviolet absorbing agent solution.

(Composition of ultraviolet absorbing agent solution)

Ultraviolet absorbing agent (compound having structure represented by Formula (UV-11)): 10.0 parts by mass
Ultraviolet absorbing agent (compound having structure represented by Formula (UV-12)): 10.0 parts by mass
Methylene chloride: 55.7 parts by mass
Methanol: 10 parts by mass
Methanol: 1.3 parts by mass
Cellulose acylate solution: 12.9 parts by mass

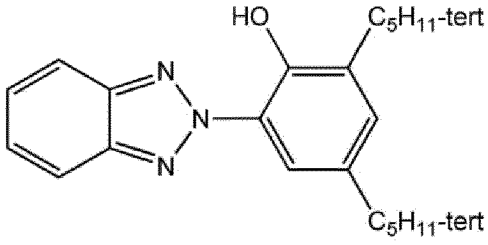

(UV-11)

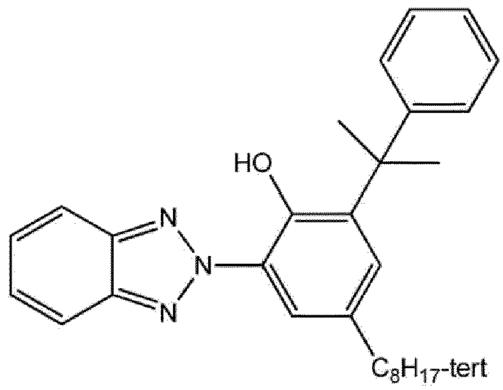

(UV-12)

(Preparation of cellulose acylate film)

The ultraviolet absorbing agent solution was added to a mixing solution obtained by mixing 94.6 parts by mass of the cellulose acylate solution and 1.3 parts by mass of the matting agent dispersion liquid such that the amount of the ultraviolet absorbing agent (UV-1) and the amount of the ultraviolet absorbing agent (UV-2) respectively reached 1.0 parts by mass with respect to 100 parts by mass of cellulose acylate, and the solution was sufficiently stirred while being heated to dissolve each component, thereby preparing a dope. The obtained dope was heated to 30°C and cast on a mirror surface stainless steel support, serving as a drum having a diameter of 3 m, through a casting geeser. The surface temperature of the mirror surface stainless steel support was set to -5°C, and the coating width was set to 1470 mm. The cast dope film was dried by applying dry air at 34°C on the drum at 150 $m^3$/min, and the dope film was peeled off from the drum in a state where the amount of the residual solvent was 150%. During the peeling, the film was stretched by 15% in the transport direction (longitudinal direction). Thereafter, both ends of the film in the width direction (direction orthogonal to the casting direction) were transported while being grasped by a pin tenter (pin tenter shown in Fig. 3 of JP1992-001009A (JP-H04-001009A), and the film was not subjected to a stretching treatment in the width direction. Further, the film was further dried by being transported between rolls of a heat treatment device, thereby producing a cellulose acylate film (T1). The amount of the residual solvent of the prepared long cellulose acylate film (T1) was 0.2%, the thickness thereof was 60 μm, and the Re (in-plane retardation) and the Rth (retardation in the thickness direction) at a wavelength of 550 nm were respectively 0.8 nm and 40 nm.

(Alkali saponification treatment)

The above-described cellulose acylate film (T1) was allowed to pass through a dielectric heating roll at a temperature of 60°C, the temperature of the film surface was increased to 40°C, the band surface of the film was coated with an alkaline solution having the composition described below with a coating amount of 14 ml/$m^2$ using a bar coater. Thereafter, the cellulose acylate film coated with the alkaline solution was transported under a steam-type far-infrared heater (manufactured by Noritake Co., Ltd.) which was heated to 110°C for 10 seconds. Subsequently, the obtained film was coated with pure water such that the coating amount thereof reached 3 ml/$m^2$ using a bar coater in the same manner as described above. Next, the process of washing the obtained film with water using a fountain coater and draining the film using an air knife was repeated three times, and the film was transported to a drying zone at 70°C for 10 seconds and dried, thereby preparing a cellulose acylate film which had been subjected to an alkali saponification treatment.

(Composition of alkaline solution)

Potassium hydroxide: 4.7 parts by mass
Water: 15.8 parts by mass
Isopropanol: 63.7 parts by mass
Surfactant SF-1 ($C_{14}H_{29}O(CH_2CH_2O)_{20}H$): 1.0 parts by mass
Propylene glycol: 14.8 parts by mass

(Formation of alignment film)

The surface of the cellulose acylate film (T1) on which the alkali saponification treatment had been performed was continuously coated with an alignment film coating solution having the following composition using a #14 wire bar. The film coated with the alignment film coating solution was dried with warm air at 60°C for 60 seconds and further dried with warm air at 100°C for 120 seconds, thereby forming an alignment film. The degree of saponification of the used modified polyvinyl alcohol was 88%.

(Composition of alignment film coating solution)

Modified polyvinyl alcohol having structure shown below: 10 parts by mass
Water: 308 parts by mass
Methanol: 70 parts by mass
Isopropanol: 29 parts by mass
Photopolymerization initiator (Omnirad 2959, manufactured by IGM Resins B. V.): 0.8 parts by mass

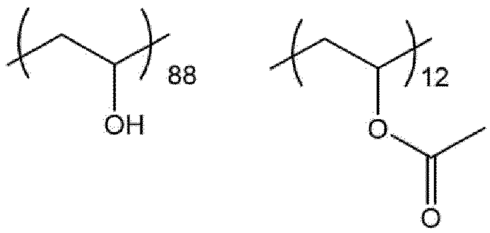

(Formation of optically anisotropic layer A)

The alignment film prepared above was continuously subjected to a rubbing treatment. Here, the longitudinal direction and the transport direction of the long film are parallel with each other, and the angle between the film longitudinal direction (transport direction) and the rotation axis of the rubbing roller is set to 72.5° (in a case where the film longitudinal direction (transport direction) is set to 90° and the counterclockwise direction is represented by a positive value with respect to the film width direction as a reference (0°) as observed from the alignment film side, the rotation axis of the rubbing roller is -17.5°, that is, the position of the rotation axis of the rubbing roller corresponds to a position rotated by 72.5 ° counterclockwise with respect to the film longitudinal direction).

The prepared alignment film was continuously coated with an optically anisotropic layer coating solution (A) containing a discotic liquid crystal (DLC) compound with the following composition using a #5.0 wire bar. The transport speed (V) of the film was set to 26 m/min. The film was heated with warm air at 115°C for 90 seconds and further heated with warm air at 80°C for 60 seconds in order to dry the solvent of the coating solution and to align and age the discotic liquid crystal compound (DLC compound), and the obtained coating film was irradiated with ultraviolet rays (UV) (exposure dose: 70 $mJ/cm^2$) at 80°C so that the alignment of the liquid crystal compound was fixed. The thickness of the optically anisotropic layer A was 2.0 μm. It was confirmed that the average tilt angle of the disc plane of the DLC compound with respect to the film surface was 90° and the DLC compound was aligned perpendicularly to the film surface. Further, in a case where the angle of the slow axis is parallel to the rotation axis of the rubbing roller, and the film longitudinal direction (transport direction) is set to 90° (in a case where the film width direction is set to 0° and the counterclockwise direction is represented by a positive value with respect to the film width direction as a reference (0°) as observed from the alignment film side), the angle is -17.5°. The obtained optically anisotropic layer A corresponds to a λ/2 plate, and the Re and Rth at a wavelength of 550 nm were respectively Re (550): 238 nm and Rth (550): -119 nm.

(Composition of optically anisotropic layer coating solution (A))

Discotic liquid crystal compound (A) shown below: 80 parts by mass
Discotic liquid crystal compound (B) shown below: 20 parts by mass
Ethylene oxide-modified trimethylolpropane triacrylate (V #360, manufactured by Osaka Organic Chemical Industry Ltd.): 5 parts by mass

Photopolymerization initiator (Omnirad 907, manufactured by IGM Resins B. V.): 4 parts by mass
Pyridinium salt (A) shown below: 2 parts by mass
Polymer (A) shown below: 0.2 parts by mass
Polymer (B) shown below: 0.1 parts by mass
Polymer (C) shown below: 0.1 parts by mass
Methyl ethyl ketone: 211 parts by mass

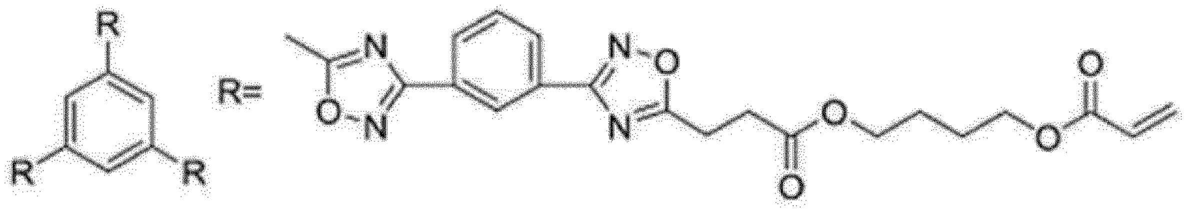

Discotic liquid crystal compound (A)

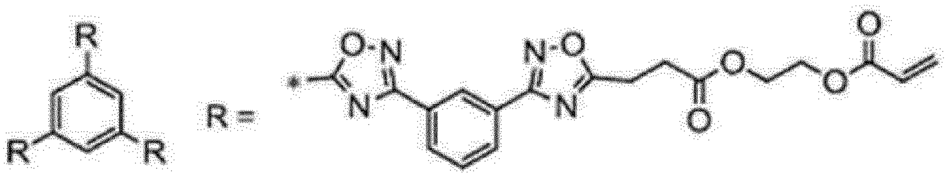

Discotic liquid crystal compound (B)

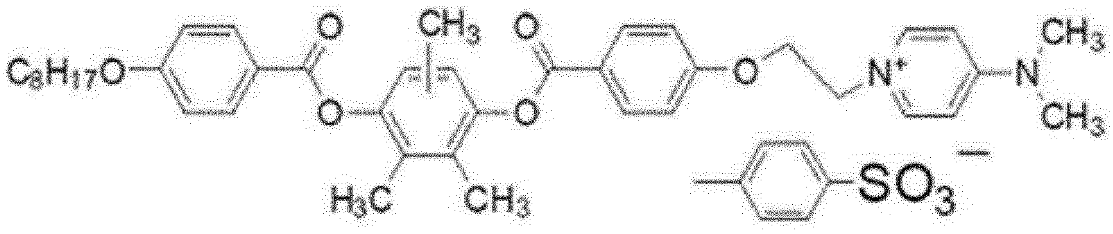

Pyridinium salt (A)

Polymer A: resin having structure shown below

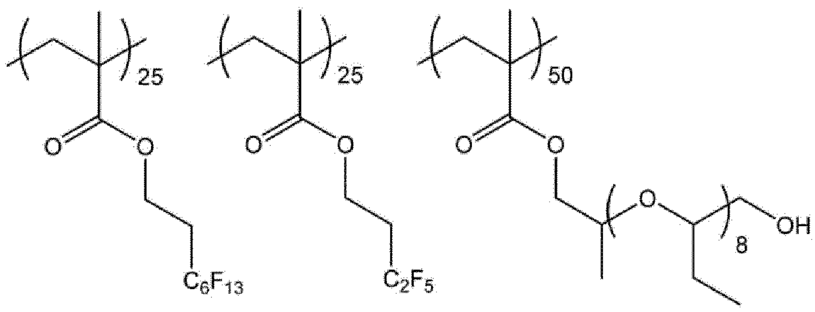

Polymer B: resin having structure shown below (in the structural formula, a represents 90 and b represents 10).

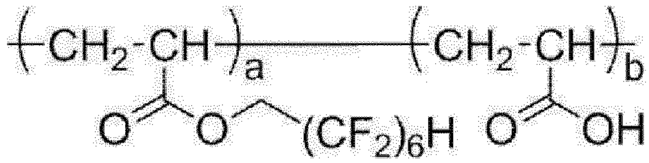

Polymer C: resin having structure shown below

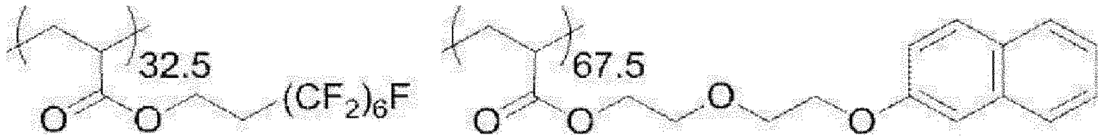

(7-2) Preparation of optically anisotropic layer B

(Formation of second optically anisotropic layer B)

An alignment film was formed on the cellulose acylate film (T1) according to the same procedure as above (preparation of the optically anisotropic layer A), and the alignment film was continuously subjected to a rubbing treatment. Here, the longitudinal direction and the transport direction of the long film are parallel with each other, and the angle between the film longitudinal direction (transport direction) and the rotation axis of the rubbing roller is set to 72.5° (in a case where the film longitudinal direction (transport direction) is set to 90° and the counterclockwise direction is represented by a positive value with respect to the film width direction as a reference (0°) as observed from the alignment film side, the rotation axis of the rubbing roller is -17.5°, that is, the position of the rotation axis of the rubbing roller corresponds to a position rotated by 102.5 ° counterclockwise with respect to the film longitudinal direction).

The alignment film after the rubbing treatment was continuously coated with an optically anisotropic layer coating solution (B) containing a discotic liquid crystal compound with the following composition using a #2.8 wire bar. The transport speed (V) of the film was set to 26 m/min. The film was heated with warm air at 60°C for 60 seconds in order to dry the solvent of the coating solution and to align and age the discotic liquid crystal compound, and the obtained coating film was irradiated with ultraviolet rays at 60°C so that the alignment of the discotic liquid crystal compound was fixed. The thickness of the optically anisotropic layer B was 0.8 $\mu$m. It was confirmed that the average tilt angle of the major axis of the discotic liquid crystal compound with respect to the film surface was 90° and the discotic liquid crystal compound was aligned perpendicularly to the film surface. Further, in a case where the angle of the slow axis is orthogonal to the rotation axis of the rubbing roller, and the film longitudinal direction is set to 90° (in a case where the film width direction is set to 0° and the counterclockwise direction is represented by a positive value with respect to the film width direction as a reference (0°) as observed from the alignment film side), the angle is 102.5° (-77.5°). The obtained optically anisotropic layer B corresponded to a $\lambda$/4 plate, and the Re (550) was 118 nm and the Rth (550) was -59 nm.

(Composition of optically anisotropic layer coating solution (B))

Discotic liquid crystal compound (A) shown above: 80 parts by mass
Discotic liquid crystal compound (B) shown above: 20 parts by mass
Ethylene oxide-modified trimethylolpropane triacrylate (V #360, manufactured by Osaka Organic Chemical Industry Ltd.): 10 parts by mass
Photopolymerization initiator (Omnirad 907, manufactured by IGM Resins B. V): 5 parts by mass
Pyridinium salt (A) shown above: 1 part by mass
Polymer (A) shown above: 0.2 parts by mass
Polymer (B) shown above: 0.1 parts by mass
Polymer (C) shown above: 0.1 parts by mass
Methyl ethyl ketone: 348 parts by mass

(7-3) Saponification treatment of resin film

The prepared resin films 303A, 304A, and 308A were immersed in a 2.3 mol/L sodium hydroxide aqueous solution at 55°C for 3 minutes. The resin films were washed in a water washing bath at room temperature (25°C) and

neutralized at 30°C with 0.05 mol/L sulfuric acid. The resin films were washed again in a water washing bath at room temperature and further dried with warm air at 100°C

(7-4) Preparation of polarizer

A polyvinyl alcohol (PVA) film having a thickness of 80 μm was immersed in an iodine aqueous solution having an iodine concentration of 0.05% by mass at 30°C for 60 seconds, dyed, longitudinally stretched to 5 times the original length during immersion in a boric acid aqueous solution having a boric acid concentration of 4% by mass for 60 seconds, and dried at 50°C for 4 minutes, thereby obtaining a polarizer having a thickness of 19 μm.

The resin film 303A subjected to the saponification treatment was attached to one surface of the polarizer prepared above using a polyvinyl alcohol-based adhesive, thereby preparing a polarizing plate.

(7-5) Production of circular polarizing plate 303D

A polarizer (which was not the resin film 303A) in the polarizing plate prepared above was coated with an adhesive (SK-2057, manufactured by Soken Chemical & Engineering Co., Ltd.) to form a pressure sensitive adhesive layer, the cellulose acylate film prepared above and the film having the alignment film and the optically anisotropic layer A were attached to each other such that the pressure sensitive adhesive layer and the optically anisotropic layer A were in close contact with each other. Thereafter, the cellulose acylate film and the alignment film were peeled off to obtain a laminate. Next, the optically anisotropic layer A in the obtained laminate was coated with a pressure sensitive adhesive (SK-2057, manufactured by Soken Chemical & Engineering Co., Ltd.) to form a pressure sensitive adhesive layer. Next, the laminate on which the pressure sensitive adhesive layer was disposed and the film having the cellulose acylate film, the alignment film, and the optically anisotropic layer B prepared above were attached to each other such that the pressure sensitive adhesive layer and the optically anisotropic layer B were in close contact with each other. Thereafter, the cellulose acylate film and the alignment film were peeled off. A circular polarizing plate 303D in which the polarizer, the optically anisotropic layer A (λ/2 plate), and the optically anisotropic layer B (λ/4 plate) were disposed in this order was prepared according to the above-described procedures. Further, in a case where the counterclockwise direction was represented by a positive value with respect to the transmission axis of the polarizer as a reference (0°) as observed from the polarizer side, the angle of the slow axis of the λ/2 plate was -17.5°, and the angle of the slow axis of the λ/4 plate was -77.5°. That is, the angle between the slow axis of the optically anisotropic layer A (λ/2 plate) and the transmission axis of the polarizer was 17.5°, and the angle between the slow axis of the optically anisotropic layer A (λ/2 plate) and the slow axis of the optically anisotropic layer B (λ/4 plate) was 60°.

(7-6) Production of organic electroluminescence display devices 303E, 304E, and 308E

A touch panel with a circular polarizing plate was peeled off from a commercially available organic electroluminescence display device GALAXY S5 (trade name, manufactured by Samsung Electronics Co., Ltd.), the circular polarizing plate was further peeled off from the touch panel, and the organic electroluminescence display element, the touch panel, and the circular polarizing plate were isolated from each other. Subsequently, the isolated touch panel was bonded again to the organic electroluminescence display element, and the circular polarizing plate 303E prepared above was bonded onto the touch panel so as not to allow air to enter, thereby preparing an organic electroluminescence display device. Liquid crystal display devices 304E and 308E were prepared in the same manner except that the kind of the resin film was changed as listed in Table 3.

[Table 6]

| Organic electroluminescence display device No. | Circular polarizing plate No. | Resin film No. | Fig. No. |
|---|---|---|---|
| 303E | 303D | 303A | Fig. 11 |
| 304E | 304D | 304A | Fig. 11 |
| 308E | 308D | 308A | Fig. 11 |

The organic electroluminescence display devices 303E and 304E containing the ultraviolet absorbing agent according to the embodiment of the present invention were not preferable because a change in image quality was small even in a case of a display for a long time as compared with the organic electroluminescence display device 308E.

<Test Example 4>

An optical film 401A was formed on the base material film by the method described below to prepare a peelable laminated film 401B.

(1) Preparation of coating solution

A coating solution 1 for forming the optical film 401A was prepared with the composition shown below. The obtained coating solution was filtered with a filter having an absolute filtration accuracy of 5 μm.

(Composition of coating solution 1)

AS-70 (acrylonitrile/styrene copolymer resin, manufactured by Nippon Steel & Sumikin Chemical Co., Ltd.): 100.0 parts by mass
VYLON 500 (polyester resin, manufactured by Toyobo Co., Ltd.): 0.9 parts by mass
SMA2000P (styrene/maleic acid copolymer, manufactured by Kawahara Petrochemical Co., Ltd.): 4.2 parts by mass
Surfactant 1: 0.1 parts by mass
Ultraviolet absorbing agent (compound (1)-5): 3.1 parts by mass
Ultraviolet absorbing agent (comparative compound 5): 0.0 parts by mass
Methyl acetate: 255.1 parts by mass
Acetonitrile: 229.6 parts by mass
Ethanol: 25.5 parts by mass

Surfactant 1: Compound having structure shown below

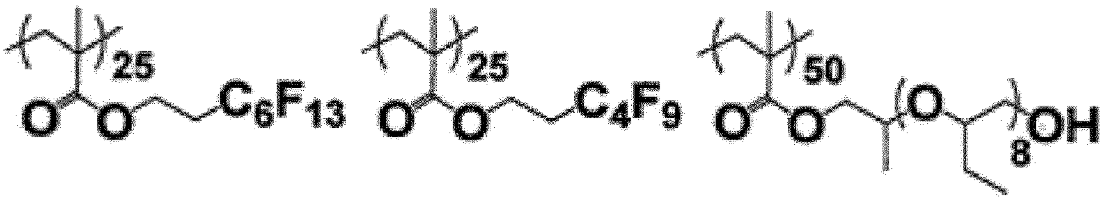

Compositional ratio numbers are in units of wt%

The compounds (1)-5 and the comparative compound 5 used as the ultraviolet absorbing agents are respectively compounds having the above-described structures.

Coating solutions 2 to 6 were prepared in the same manner as that for the coating solution 1 except that the addition amounts of the compounds (1)-5 and the comparative compound 5 were changed as listed in the table below.

(2) Coating of peelable laminated film

A commercially available polyethylene terephthalate film (EMBLET S38, film thickness of 38 μm, absorption edge wavelength of 310 nm on short wavelength side, manufactured by Unitika Ltd.) was used as a base material film, and optical films 401A to 406A were prepared such that the film thickness reached 5 μm using the coating solutions 1 to 6, thereby preparing a peelable laminated film (film thickness of 43 μm, absorption edge wavelength of 310 nm on short wave side). Specifically, the base material film was coated with the coating solution 1 under the condition of a transport speed of 30 m/min by the die coating method using the slot die described in Example 1 of JP2006-122889A, and the film was dried at 105°C for 30 seconds. Thereafter, the film was wound up. The obtained peelable laminated films were set as peelable laminated films 401B to 406B of each example and each comparative example.

The absorbance of each of the peelable laminated films 401B to 406B prepared above was measured using a spectrophotometer UV3600 (manufactured by Shimadzu Corporation). Further, the ultraviolet absorption ability on a long wavelength side was evaluated such that a case where the value obtained by dividing the absorbance at a wavelength of 405 nm by the addition amount of the ultraviolet absorbing agent (% by mass with respect to AS-70) was 50 or greater was evaluated as A, a case where the value thereof was in a range of 25 to 50 was evaluated as B, and a case where the value thereof was 25 or less was evaluated as C. The ultraviolet absorption ability on a long wavelength side is higher as the value

increases. The results are listed in the columns of the ultraviolet absorption ability on a long wavelength side in the table below.

[Table 7]

| Peelable laminate film No. | Optical film No. | Coating solution No. | Addition amount of compound (1)-5 (% by mass) | Addition amount of comparative compound 5 (% by mass) | Ultraviolet absorption ability on long wavelength side |
|---|---|---|---|---|---|
| 401B | 401A | 1 | 3.1 | 0 | A |
| 402B | 402A | 2 | 7.9 | 0 | A |
| 403B | 403A | 3 | 12.8 | 0 | A |
| 404B | 404A | 4 | 0 | 0.9 | C |
| 405B | 405A | 5 | 0 | 3.1 | C |
| 406B | 406A | 6 | 0 | 7.9 | C |

The peelable laminated films 401B to 403B had larger absorption of light in the vicinity of the wavelength of 400 nm than the peelable laminated films 404B to 406B and were excellent in absorbency of ultraviolet rays on a long wavelength side.

(3) Preparation of polarizing plate

A polarizing plate was prepared by the method described below.

(3-1) Saponification treatment of polarizing plate protective film

A 60 μm triacetyl cellulose film (FUJITAC TG60, manufactured by FUJIFILM Corporation) was immersed in a 2.3 mol/L sodium hydroxide aqueous solution at 55°C for 3 minutes. The resin films were washed in a water washing bath at room temperature (25°C) and neutralized at 30°C with 0.05 mol/L sulfuric acid. The resin films were washed again in a water washing bath at room temperature and further dried with warm air at 100°C

(3-2-1) Production of polarizing plate 4C1

A polarizing plate 4C1 was prepared in the same manner as that for the polarizing plate 303B1 except that the triacetyl cellulose film having a film thickness of 60 μm was used in place of the resin film 303A.

(3-2-2) Production of polarizing plate 4C2

A polarizing plate 4C2 was prepared in the same manner as that for the polarizing plate 303B2 except that the triacetyl cellulose film having a film thickness of 60 μm was used in place of the resin film 303A.

(3-3-1) Production of polarizing plate 402D1 with peelable laminated film

The peelable laminated film 402 on the optical film side and the polarizing plate 4C-1 on the VA phase difference film side were attached to each other via the ultraviolet curable adhesive 1 and irradiated with ultraviolet rays at an intensity of 200 mJ to cure the ultraviolet curable adhesive 1, thereby obtaining the polarizing plate 402D1 with a peelable laminated film.

(3-3-2) Production of polarizing plate 402D2 with peelable laminated film

The peelable laminated film 402 on the optical film side and the polarizing plate 4C-2 on the VA phase difference film side were attached to each other via the ultraviolet curable adhesive 1 and irradiated with ultraviolet rays at an intensity of 200 mJ to cure the ultraviolet curable adhesive 1, thereby obtaining the polarizing plate 402D1 with a peelable laminated film.

(3-4) Production of liquid crystal display devices 402E1, 405E1, 402E2, and 405E2

The EMBLET S38 of the polarizing plate 402D1 was peeled off so that the optical film was exposed, a liquid crystal panel of a commercially available liquid crystal display device FlexScan 19-inch color liquid crystal monitor S1923-HBK (trade name, manufactured by EIZO Corporation) was taken out, the polarizing plate on the front side was peeled off, and the polarizing plate 402D1 on the optical film side was attached thereto in place of the peeled polarizing plate via a pressure sensitive adhesive (SK-2057, manufactured by Soken Chemical & Engineering Co., Ltd.). In this manner, a liquid crystal display device 402E1 was produced. Liquid crystal display devices 405E1, 402E2, and 405E2 were produced in the same manner except that the kind of the polarizing plate with the peelable laminated film was changed as listed in the table below.

[Table 8]

| Liquid crystal display device No. | Polarizing plate with peelable laminate film No. | Polarizing plate No. | Peelable laminated film No. | Fig. No. |
|---|---|---|---|---|
| 402E1 | 402D1 | 4C1 | 402A | Fig. 5 |
| 405E1 | 405D1 | 4C1 | 405A | Fig. 5 |
| 402E2 | 402D2 | 4C2 | 402A | Fig. 5 |
| 405E2 | 405D2 | 4C2 | 405A | Fig. 5 |

The liquid crystal display devices 402E1 and 402E2 containing the ultraviolet absorbing agent according to the embodiment of the present invention were not preferable because a change in image quality was small even in a case of a display for a long time as compared with the liquid crystal display devices 405E1 and 405E2.

<Test Example 5>

A pressure sensitive adhesive 501 was prepared by the method described below.

(1) Preparation of coating solution

(Composition of coating solution 1)

Acrylic resin (SK Dyne-SF2147): 100.0 parts by mass
Polymerizable compound (TD-75): 0.04 parts by mass
Silane coupling agent (A-50): 0.06 parts by mass
Ultraviolet absorbing agent (compound (1)-5): 0.2 parts by mass
Ultraviolet absorbing agent (comparative compound 5): 0.0 parts by mass

The used materials are shown below. The compound (1)-5 and the comparative compound 5 used as the ultraviolet absorbing agent are respectively compounds having the above-described structures.

SK Dyne-SF2147: acrylic acid ester copolymer Concentration of solid contents: 10% to 20% by mass, ethyl acetate, butyl acrylate solvent (Soken Chemical & Engineering Co., Ltd.)
TD-75: trimethylolpropane adduct of tolylene diisocyanate (Soken Chemical & Engineering Co., Ltd.)
A-50: organosilane (Soken Chemical & Engineering Co., Ltd.)

Coating solutions 2 to 6 were prepared in the same manner as that for the coating solution 1 except that the addition amounts of the compounds (1)-5 and the comparative compound 5 were changed as shown in the table below.

(2) Preparation of pressure sensitive adhesive sheet

A surface subjected to a release treatment of a polyethylene terephthalate film (MASTACK AS3-310, manufactured by Fujimori Kogyo Co., Ltd., hereinafter, also referred to as a separator) on which a release treatment had been performed was coated with the coating solutions 1 to 6 such that the thickness after drying reached 35 μm using an applicator and dried at 110°C for 3 minutes. Thereafter, the surface of the separator subjected to a release treatment was

bonded, and the humidity thereof was adjusted at a temperature of 25°C and a humidity of 60% for 24 hours, thereby preparing pressure sensitive adhesive sheets 501A to 506A.

(3) Preparation of glass-bonded pressure sensitive adhesive

The separators of the pressure sensitive adhesive sheets 501A to 506A were peeled off and bonded to eagle glass having a thickness of 1.1 mm, thereby preparing glass-bonded pressure sensitive adhesives 501B to 506B. The absorbances of the glass-bonded adhesives 501B to 506B prepared above were measured using a spectrophotometer UV3600 (manufactured by Shimadzu Corporation). Further, the ultraviolet absorption ability on a long wavelength side was evaluated such that a case where the value obtained by dividing the absorbance at a wavelength of 405 nm by the addition amount of the ultraviolet absorbing agent (% by mass with respect to KS Dyne-SF2147) was 50 or greater was evaluated as A, a case where the value thereof was in a range of 25 to 50 was evaluated as B, and a case where the value thereof was 25 or less was evaluated as C. The ultraviolet absorption ability on a long wavelength side is higher as the value increases. The results are listed in the columns of the ultraviolet absorption ability on a long wavelength side in the table below.

[Table 9]

| Glass-bonded pressure sensitive adhesive No. | Pressure sensitive adhesive sheet No. | Coating solution No. | Addition amount of compound (1)-5 | Addition amount of comparative compound 5 (% by mass) | Ultraviolet absorption ability on long wavelength side |
|---|---|---|---|---|---|
| 501B | 501A | 1 | 0.2 | 0 | A |
| 502B | 502A | 2 | 0.3 | 0 | A |
| 503B | 503A | 3 | 0.4 | 0 | A |
| 504B | 504A | 4 | 0 | 0.2 | c |
| 505B | 505A | 5 | 0 | 0.3 | c |
| 506B | 506A | 6 | 0 | 0.4 | C |

The peelable laminated films 501B to 503B absorbed more light in the vicinity of the wavelength of 400 nm than the peelable laminated films 504B to 506B, and were excellent in absorbency of ultraviolet rays on the long wavelength side.

(4) Production of liquid crystal display devices 502C1, 505C1, 502C2, and 505C2

A liquid crystal panel of a commercially available liquid crystal display device FlexScan 19-inch color liquid crystal monitor S1923-HBK (trade name, manufactured by EIZO Corporation) was taken out, the polarizing plate on the backlight side was peeled off, and the polarizing plate 4C1 on the VA phase difference film side was attached thereto in place of the peeled polarizing plate via a pressure sensitive adhesive sheet 502A. In this manner, the liquid crystal display device 502C1 was produced. Further, liquid crystal display devices 505C1, 502C2, and 505C2 were produced in the same manner except that the kind of the pressure sensitive adhesive sheet and the kind of the polarizing plate were changed.

[Table 10]

| Liquid crystal display device No. | Polarizing plate No. | Pressure sensitive adhesive No. | Fig. No. |
|---|---|---|---|
| 502C1 | 4C1 | 502A | Fig. 8 |
| 505C1 | 4C1 | 505A | Fig. 8 |
| 502C2 | 4C2 | 502A | Fig. 8 |
| 505C2 | 4C2 | 505A | Fig. 8 |

The liquid crystal display devices 502C1 and 502C2 containing the ultraviolet absorbing agent according to the embodiment of the present invention were not preferable because a change in image quality was small even in a case of a display for a long time as compared with the liquid crystal display devices 505C1 and 505C2.

Explanation of References

1 to 10: liquid crystal display device
11 to 13: organic electroluminescence display device
22, 49, 176: polarizing plate protective film containing ultraviolet absorbing agent according to embodiment of present invention
56, 75: inner protective film (phase difference film) containing ultraviolet absorbing agent according to embodiment of present invention
88, 107, 206: optical member (optical film) containing ultraviolet absorbing agent according to embodiment of present invention.
121, 138, 194: pressure sensitive adhesive or adhesive containing ultraviolet absorbing agent according to embodiment of present invention
145, 174: functional layer containing ultraviolet absorbing agent according to embodiment of present invention
21, 35, 36, 50, 51, 65, 66, 80, 81, 97, 98, 114, 115, 129, 130, 144, 159, 160, 175, 186, 197: functional layer
23, 25, 27, 29, 31, 33, 38, 40, 42, 44, 46, 48, 53, 55, 57, 59, 61, 63, 68, 70, 72, 74, 76, 78, 83, 85, 87, 89, 91, 93, 95, 100, 102, 104, 106, 108, 110, 112, 117, 119, 123, 125, 127, 132, 134, 136, 140, 142, 147, 149, 151, 153, 155, 157, 162, 164, 166, 168, 170, 172, 177, 179, 181, 183, 188, 190, 192, 199, 201, 203, 205, 207: adhesive or pressure sensitive adhesive
24, 32, 39, 47, 54, 62, 69, 77, 84, 94, 101, 111, 118, 126, 133, 141, 148, 156, 163, 171, 178, 189, 200: polarizer
26, 30, 41, 45, 56, 60, 71, 75, 86, 92, 103, 109, 120, 124, 135, 139, 150, 154, 165, 169: phase difference film
28, 43, 58, 73, 90, 105, 122, 137, 152, 167: liquid crystal cell
34, 37, 52, 64, 67, 79, 82, 96, 99, 113, 116, 128, 131, 143, 146, 158, 161, 173, 187, 198: polarizing plate protective film
180, 191, 202: 2/λ plate
182, 193, 204: 4/λ plate
184, 195, 208: touch panel
185, 196, 209: organic electroluminescence light emitting element

## Claims

**1.** A resin composition comprising:

a compound represented by Formula (1); and
a resin,

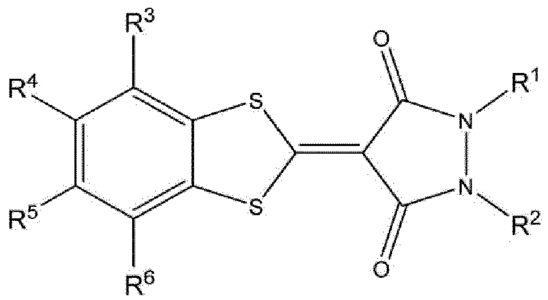

(1)

in Formula (1), $R^1$ and $R^2$ each independently represent an alkyl group, an aryl group or a heterocyclic group,
$R^3$ and $R^6$ each independently represent an alkoxy group, an acyloxy group, a carbamoyloxy group, or an alkoxycarbonyloxy group,
$R^4$ represents an alkyl group, an aryl group, an alkoxy group, or an aryloxy group,
$R^5$ represents a hydrogen atom, an alkyl group, an aryl group, an alkoxy group, or an aryloxy group,
$R^1$ and $R^2$ may be bonded to each other to form a ring,
$R^3$ and $R^4$ may be bonded to each other to form a ring,
$R^4$ and $R^5$ may be bonded to each other to form a ring, and
$R^5$ and $R^6$ may be bonded to each other to form a ring,

where in a case where $R^3$ and $R^6$ each independently represent an acyloxy group or a carbamoyloxy group, at least one of $R^4$ or $R^5$ represents an aryl group, an alkoxy group, or an aryloxy group.

**2.** The resin composition according to claim 1, wherein in Formula (1), at least one of $R^3$ or $R^6$ represents an alkoxy group.

**3.** The resin composition according to claim 1,

wherein the compound represented by Formula (1) is a compound represented by Formula (1a),

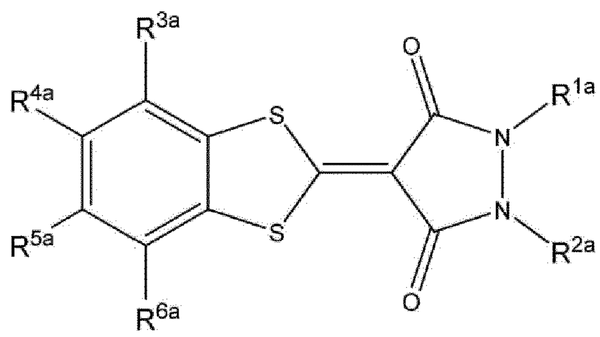

(1a)

in Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,
$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group.

**4.** The resin composition according to any one of claims 1 to 3, further comprising:

a compound represented by Formula (2),

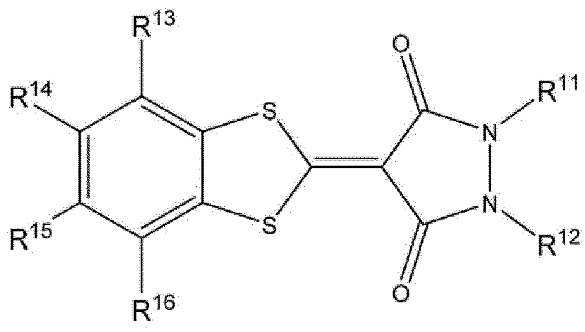

(2)

in Formula (2), $R^{11}$ and $R^{12}$ each independently represent an alkyl group, an aryl group, or a heterocyclic group,
$R^{13}$ and $R^{16}$ each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group,
$R^{14}$ represents an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,

$R^{15}$ represents a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,
$R^{11}$ and $R^{12}$ may be bonded to each other to form a ring,
$R^{13}$ and $R^{14}$ may be bonded to each other to form a ring,
$R^{14}$ and $R^{15}$ may be bonded to each other to form a ring, and
$R^{15}$ and $R^{16}$ may be bonded to each other to form a ring,
where at least one of $R^{13}$ or $R^{16}$ represents a hydroxy group.

**5.** The resin composition according to any one of claims 1 to 4, further comprising:
an ultraviolet absorbing agent other than the compound represented by Formula (1).

**6.** The resin composition according to any one of claims 1 to 5,
wherein the resin is at least one selected from a (meth)acrylic resin, a polystyrene resin, a polyester resin, a polyurethane resin, a polythiourethane resin, a polyimide resin, an epoxy resin, a polycarbonate resin, or a cellulose acylate resin.

**7.** A cured substance which is formed of the resin composition according to any one of claims 1 to 6.

**8.** An ultraviolet absorbing agent comprising:

a compound represented by Formula (1),

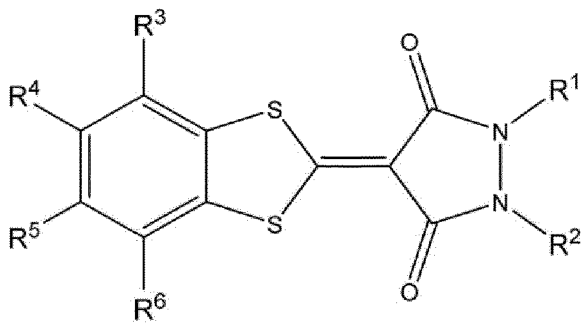

(1)

in Formula (1), $R^1$ and $R^2$ each independently represent an alkyl group, an aryl group or a heterocyclic group,
$R^3$ and $R^6$ each independently represent an alkoxy group, an acyloxy group, a carbamoyloxy group, or an alkoxycarbonyloxy group,
$R^4$ represents an alkyl group, an aryl group, an alkoxy group, or an aryloxy group,,
$R^5$ represents a hydrogen atom, an alkyl group, an aryl group, an alkoxy group, or an aryloxy group,
$R^1$ and $R^2$ may be bonded to each other to form a ring,
$R^3$ and $R^4$ may be bonded to each other to form a ring,
$R^4$ and $R^5$ may be bonded to each other to form a ring, and
$R^5$ and $R^6$ may be bonded to each other to form a ring,
where in a case where $R^3$ and $R^6$ each independently represent an acyloxy group or a carbamoyloxy group, at least one of $R^4$ or $R^5$ represents an aryl group, an alkoxy group, or an aryloxy group.

**9.** The ultraviolet absorbing agent according to claim 8, further comprising:

the compound represented by Formula (2),

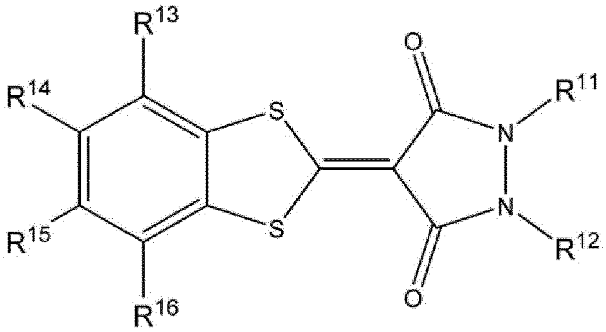

(2)

in Formula (2), $R^{11}$ and $R^{12}$ each independently represent an alkyl group, an aryl group, or a heterocyclic group, $R^{13}$ and $R^{16}$ each independently represent a hydroxy group, an alkoxy group, an aryloxy group, an acyloxy group, a carbamoyloxy group, an alkoxycarbonyloxy group, an aryloxycarbonyloxy group, a sulfinyloxy group, or a sulfonyloxy group,
$R^{14}$ represents an alkyl group, an aryl group, an alkoxy group, an aryloxy group, or an acyloxy group, an alkylamino group, an anilino group, an acylamino group, an alkylsulfonylamino group, an arylsulfonylamino group, an alkylthio group, or an arylthio group,
$R^{15}$ represents a hydrogen atom, an alkyl group, an aryl group, an alkoxy group, an aryloxy group, an acyloxy group, an alkylamino group, an anilino group, an acylamino group, an alkylsulfonylamino group, an arylsulfonylamino group, an alkylthio group, or an arylthio group,
$R^{11}$ and $R^{12}$ may be bonded to each other to form a ring,
$R^{13}$ and $R^{14}$ may be bonded to each other to form a ring,
$R^{14}$ and $R^{15}$ may be bonded to each other to form a ring, and
$R^{15}$ and $R^{16}$ may be bonded to each other to form a ring,
where at least one of $R^{13}$ or $R^{16}$ represents a hydroxy group.

**10.** An ultraviolet cut filter comprising:
the ultraviolet absorbing agent according to claim 8 or 9.

**11.** A lens comprising:
the ultraviolet absorbing agent according to claim 8 or 9.

**12.** A protective material comprising:
the ultraviolet absorbing agent according to claim 8 or 9.

**13.** A compound which is represented by Formula (1a),

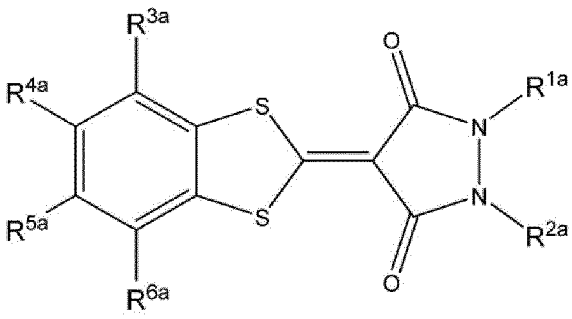

(1a)

in Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,
$R^{1a}$ and $R^{2a}$ may be bonded to each other to form a ring,

$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group.

**14.** A method of synthesizing a compound represented by Formula (1a), comprising:

reacting a compound represented by Formula (2a) with an alkyl halide compound or a carboxylic acid halide,

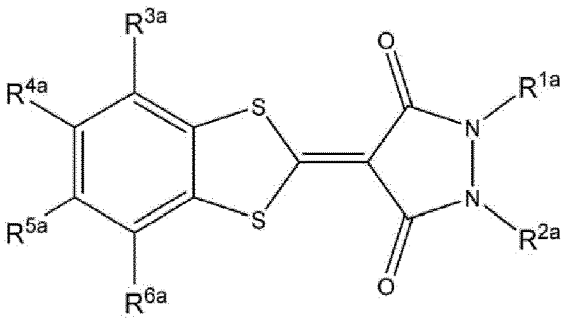

(1a)

in Formula (1a), $R^{1a}$ and $R^{2a}$ each independently represent an alkyl group,
$R^{3a}$ and $R^{6a}$ each independently represent an alkoxy group or an acyloxy group,
$R^{4a}$ represents an alkyl group or an alkoxy group,
$R^{5a}$ represents a hydrogen atom, an alkyl group, or an alkoxy group,
$R^{1a}$ and $R^{2a}$ may be bonded to each other to form a ring,
$R^{3a}$ and $R^{4a}$ may be bonded to each other to form a ring,
$R^{4a}$ and $R^{5a}$ may be bonded to each other to form a ring, and
$R^{5a}$ and $R^{6a}$ may be bonded to each other to form a ring,
where in a case where $R^{3a}$ and $R^{6a}$ represent an acyloxy group, at least one of $R^{4a}$ or $R^{5a}$ represents an alkoxy group,

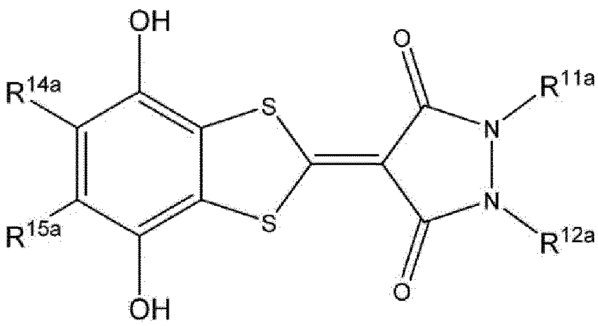

(2a)

in Formula (2a), $R^{11a}$ and $R^{12a}$ each independently represent an alkyl group,
$R^{14a}$ represents an alkyl group or an alkoxy group,
$R^{15a}$ represents a hydrogen atom, an alkyl group, or an alkoxy group, and
$R^{14a}$ and $R^{15a}$ may be bonded to each other to form a ring.

## Patentansprüche

**1.** Harzzusammensetzung, umfassend:

eine Verbindung, die durch die Formel (1) dargestellt wird; und
ein Harz,

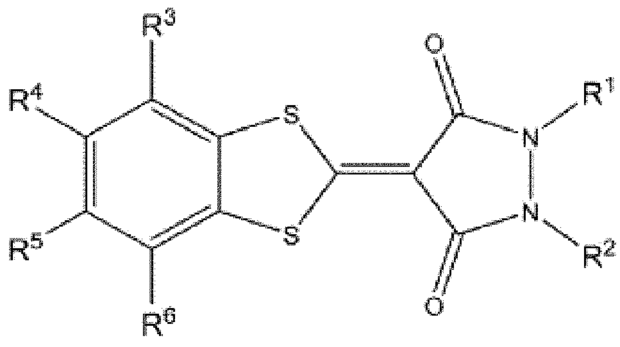

(1)

wobei in Formel (1) $R^1$ und $R^2$ jeweils unabhängig voneinander eine Alkylgruppe, eine Arylgruppe oder eine heterocyclische Gruppe darstellen,
$R^3$ und $R^6$ jeweils unabhängig voneinander eine Alkoxygruppe, eine Acyloxygruppe, eine Carbamoyloxygruppe oder eine Alkoxycarbonyloxygruppe darstellen,
$R^4$ eine Alkylgruppe, eine Arylgruppe, eine Alkoxygruppe oder eine Aryloxygruppe darstellt,
$R^5$ ein Wasserstoffatom, eine Alkylgruppe, eine Arylgruppe, eine Alkoxygruppe oder eine Aryloxygruppe darstellt,
$R^1$ und $R^2$ miteinander verbunden sein können, um einen Ring zu bilden,
$R^3$ und $R^4$ miteinander verbunden sein können, um einen Ring zu bilden,
$R^4$ und $R^5$ miteinander verbunden sein können, um einen Ring zu bilden, und
$R^5$ und $R^6$ miteinander verbunden sein können, um einen Ring zu bilden,
wobei in einem Fall, in dem $R^3$ und $R^6$ jeweils unabhängig voneinander eine Acyloxygruppe oder eine Carbamoyloxygruppe darstellen, mindestens eines von $R^4$ und $R^5$ eine Arylgruppe, eine Alkoxygruppe oder eine Aryloxygruppe darstellt.

**2.** Harzzusammensetzung nach Anspruch 1,
wobei in Formel (1) mindestens eines von $R^3$ und $R^6$ eine Alkoxygruppe darstellt.

**3.** Harzzusammensetzung nach Anspruch 1,

wobei die durch Formel (1) dargestellte Verbindung eine Verbindung ist, die durch Formel (1a) dargestellt wird,

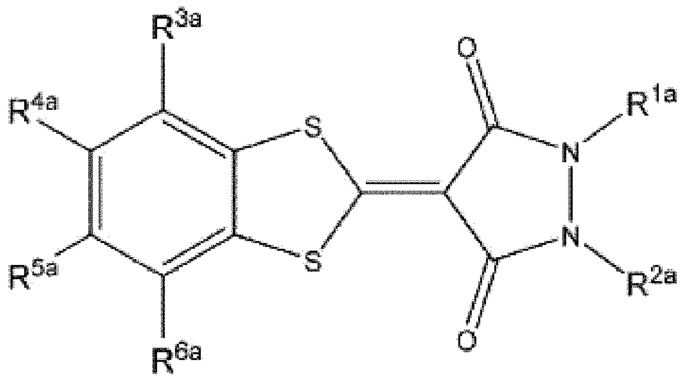

(1a)

in Formel (1a) $R^{1a}$ und $R^{2a}$ jeweils unabhängig voneinander eine Alkylgruppe darstellen,
$R^{3a}$ und $R^{6a}$ jeweils unabhängig voneinander eine Alkoxygruppe oder eine Acyloxygruppe darstellen,
$R^{4a}$ eine Alkylgruppe oder eine Alkoxygruppe darstellt,
$R^{5a}$ ein Wasserstoffatom, eine Alkylgruppe oder eine Alkoxygruppe darstellt,
$R^{1a}$ und $R^{2a}$ miteinander verbunden sein können, um einen Ring zu bilden,
$R^{3a}$ und $R^{4a}$ miteinander verbunden sein können, um einen Ring zu bilden,
$R^{4a}$ und $R^{5a}$ miteinander verbunden sein können, um einen Ring zu bilden, und
$R^{5a}$ und $R^{6a}$ miteinander verbunden sein können, um einen Ring zu bilden,

wobei in einem Fall, in dem $R^{3a}$ und $R^{6a}$ eine Acyloxygruppe darstellen, mindestens eines von $R^{4a}$ und $R^{5a}$ eine Alkoxygruppe darstellt.

**4.** Harzzusammensetzung nach einem der Ansprüche 1 bis 3, ferner umfassend:

eine Verbindung, die durch die Formel (2) dargestellt wird,

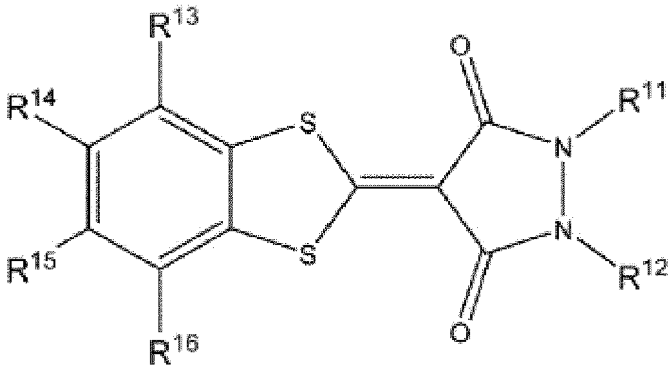

(2)

wobei in Formel (2) $R^{11}$ und $R^{12}$ jeweils unabhängig eine Alkylgruppe, eine Arylgruppe oder eine heterocyclische Gruppe darstellen,
$R^{13}$ und $R^{16}$ jeweils unabhängig voneinander eine Hydroxylgruppe, eine Alkoxygruppe, eine Aryloxygruppe, eine Acyloxygruppe, eine Carbamoyloxygruppe, eine Alkoxycarbonyloxygruppe, eine Aryloxycarbonyloxygruppe, eine Sulfinyloxygruppe oder eine Sulfonyloxygruppe darstellen,
$R^{14}$ eine Alkylgruppe, eine Arylgruppe, eine Alkoxygruppe, eine Aryloxygruppe, eine Acyloxygruppe, eine Alkylaminogruppe, eine Anilinogruppe, eine Acylaminogruppe, eine Alkylsulfonylaminogruppe, eine Arylsulfonylaminogruppe, eine Alkylthiogruppe oder eine Arylthiogruppe darstellt,
$R^{15}$ ein Wasserstoffatom, eine Alkylgruppe, eine Arylgruppe, eine Alkoxygruppe, eine Aryloxygruppe, eine Acyloxygruppe, eine Alkylaminogruppe, eine Anilinogruppe, eine Acylaminogruppe, eine Alkylsulfonylaminogruppe, eine Arylsulfonylaminogruppe, eine Alkylthiogruppe oder eine Arylthiogruppe darstellt,
$R^{11}$ und $R^{12}$ miteinander verbunden sein können, um einen Ring zu bilden,
$R^{13}$ und $R^{14}$ miteinander verbunden sein können, um einen Ring zu bilden,
$R^{14}$ und $R^{15}$ miteinander verbunden sein können, um einen Ring zu bilden, und
$R^{15}$ und $R^{16}$ miteinander verbunden sein können, um einen Ring zu bilden,
wobei mindestens eines von $R^{13}$ und $R^{16}$ eine Hydroxygruppe darstellt.

**5.** Harzzusammensetzung nach einem der Ansprüche 1 bis 4, ferner umfassend:
ein ultraviolettabsorbierendes Mittel, das nicht die Verbindung ist, die durch Formel (1) dargestellt wird.

**6.** Harzzusammensetzung nach einem der Ansprüche 1 bis 5,
wobei das Harz mindestens eines ausgewählt aus einem (Meth)acrylharz, einem Polystyrolharz, einem Polyesterharz, einem Polyurethanharz, einem Polythiourethanharz, einem Polyimidharz, einem Epoxidharz, einem Polycarbonatharz oder einem Celluloseacylatharz ausgewählt ist.

**7.** Ausgehärtete Substanz, die aus der Harzzusammensetzung nach einem der Ansprüche 1 bis 6 gebildet ist.

**8.** Ultraviolettabsorbierendes Mittel, umfassend:

eine Verbindung, die durch die Formel (1) dargestellt wird,

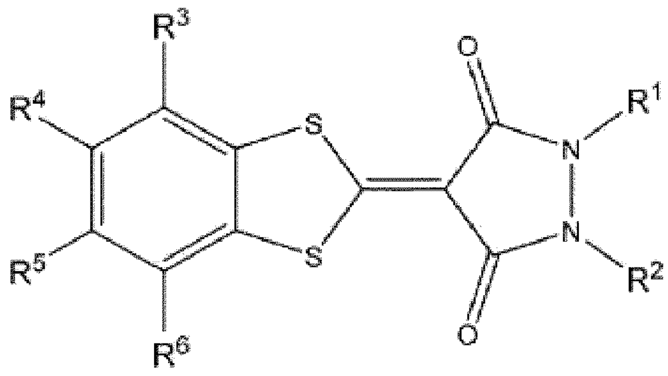

(1)

wobei in Formel (1) $R^1$ und $R^2$ jeweils unabhängig voneinander eine Alkylgruppe, eine Arylgruppe oder eine heterocyclische Gruppe darstellen,
$R^3$ und $R^6$ jeweils unabhängig voneinander eine Alkoxygruppe, eine Acyloxygruppe, eine Carbamoyloxygruppe oder eine Alkoxycarbonyloxygruppe darstellen,
$R^4$ eine Alkylgruppe, eine Arylgruppe, eine Alkoxygruppe oder eine Aryloxygruppe darstellt,
$R^5$ ein Wasserstoffatom, eine Alkylgruppe, eine Arylgruppe, eine Alkoxygruppe oder eine Aryloxygruppe darstellt,
$R^1$ und $R^2$ miteinander verbunden sein können, um einen Ring zu bilden,
$R^3$ und $R^4$ miteinander verbunden sein können, um einen Ring zu bilden,
$R^4$ und $R^5$ miteinander verbunden sein können, um einen Ring zu bilden, und
$R^5$ und $R^6$ miteinander verbunden sein können, um einen Ring zu bilden,
wobei in einem Fall, in dem $R^3$ und $R^6$ jeweils unabhängig voneinander eine Acyloxygruppe oder eine Carbamoyloxygruppe darstellen, mindestens eines von $R^4$ und $R^5$ eine Arylgruppe, eine Alkoxygruppe oder eine Aryloxygruppe darstellt.

**9.** Ultraviolettabsorbierendes Mittel nach Anspruch 8, ferner umfassend:

die Verbindung, die durch die Formel (2) dargestellt wird,

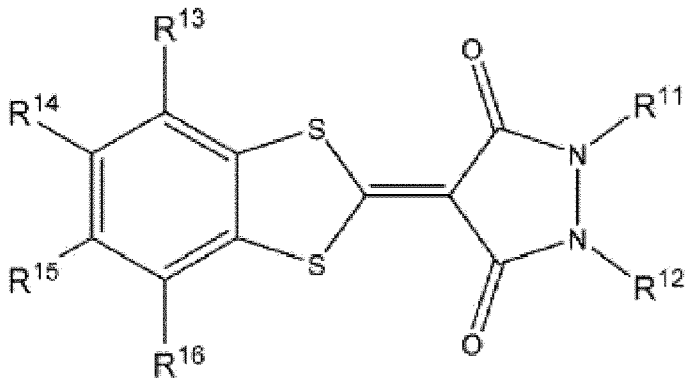

(2)

wobei in Formel (2) $R^{11}$ und $R^{12}$ jeweils unabhängig eine Alkylgruppe, eine Arylgruppe oder eine heterocyclische Gruppe darstellen,
$R^{13}$ und $R^{16}$ jeweils unabhängig voneinander eine Hydroxylgruppe, eine Alkoxygruppe, eine Aryloxygruppe, eine Acyloxygruppe, eine Carbamoyloxygruppe, eine Alkoxycarbonyloxygruppe, eine Aryloxycarbonyloxygruppe, eine Sulfinyloxygruppe oder eine Sulfonyloxygruppe darstellen,
$R^{14}$ eine Alkylgruppe, eine Arylgruppe, eine Alkoxygruppe, eine Aryloxygruppe oder eine Acyloxygruppe, eine Alkylaminogruppe, eine Anilinogruppe, eine Acylaminogruppe, eine Alkylsulfonylaminogruppe, eine Arylsulfonylaminogruppe, eine Alkylthiogruppe oder eine Arylthiogruppe darstellt,
$R^{15}$ ein Wasserstoffatom, eine Alkylgruppe, eine Arylgruppe, eine Alkoxygruppe, eine Aryloxygruppe, eine Acyloxygruppe, eine Alkylaminogruppe, eine Anilinogruppe, eine Acylaminogruppe, eine Alkylsulfonylaminogruppe, eine Arylsulfonylaminogruppe, eine Alkylthiogruppe oder eine Arylthiogruppe darstellt,
$R^{11}$ und $R^{12}$ miteinander verbunden sein können, um einen Ring zu bilden,
$R^{13}$ und $R^{14}$ miteinander verbunden sein können, um einen Ring zu bilden,
$R^{14}$ und $R^{15}$ miteinander verbunden sein können, um einen Ring zu bilden, und
$R^{15}$ und $R^{16}$ miteinander verbunden sein können, um einen Ring zu bilden,
wobei mindestens eines von $R^{13}$ und $R^{16}$ eine Hydroxygruppe darstellt.

**10.** Ultraviolett-Sperrfilter, umfassend:
das Ultraviolettabsorbierendes Mittel nach Anspruch 8 oder 9.

**11.** Linse, umfassend:
das Ultraviolettabsorbierendes Mittel nach Anspruch 8 oder 9.

**12.** Schutzmaterial, umfassend:
das Ultraviolettabsorbierendes Mittel nach Anspruch 8 oder 9.

**13.** Verbindung, die durch die Formel (1a) dargestellt wird,

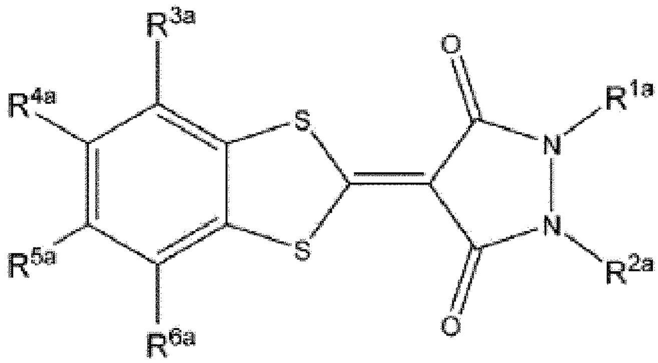

(1a)

wobei in Formel (1a) $R^{1a}$ und $R^{2a}$ jeweils unabhängig voneinander eine Alkylgruppe darstellen,
$R^{3a}$ und $R^{6a}$ jeweils unabhängig voneinander eine Alkoxygruppe oder eine Acyloxygruppe darstellen,
$R^{4a}$ eine Alkylgruppe oder eine Alkoxygruppe darstellt,
$R^{5a}$ ein Wasserstoffatom, eine Alkylgruppe oder eine Alkoxygruppe darstellt,
$R^{1a}$ und $R^{2a}$ miteinander verbunden sein können, um einen Ring zu bilden,
$R^{3a}$ und $R^{4a}$ miteinander verbunden sein können, um einen Ring zu bilden,
$R^{4a}$ und $R^{5a}$ miteinander verbunden sein können, um einen Ring zu bilden, und
$R^{5a}$ und $R^{6a}$ miteinander verbunden sein können, um einen Ring zu bilden,
wobei in einem Fall, in dem $R^{3a}$ und $R^{6a}$ eine Acyloxygruppe darstellen, mindestens eines von $R^{4a}$ und $R^{5a}$ eine Alkoxygruppe darstellt.

**14.** Verfahren des Synthetisierens einer Verbindung, die durch Formel (1a) dargestellt wird, umfassend:

Reagieren einer Verbindung, die durch Formel (2a) dargestellt wird, mit einer Alkylhalogenidverbindung oder einem Carbonsäurehalogenid,

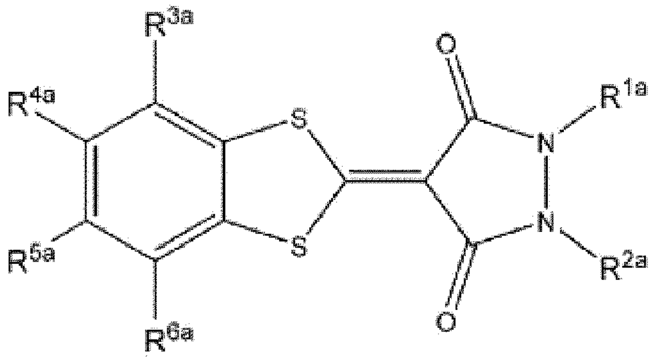

(1a)

wobei in Formel (1a) $R^{1a}$ und $R^{2a}$ jeweils unabhängig voneinander eine Alkylgruppe darstellen,
$R^{3a}$ und $R^{6a}$ jeweils unabhängig voneinander eine Alkoxygruppe oder eine Acyloxygruppe darstellen,
$R^{4a}$ eine Alkylgruppe oder eine Alkoxygruppe darstellt,
$R^{5a}$ ein Wasserstoffatom, eine Alkylgruppe oder eine Alkoxygruppe darstellt,
$R^{1a}$ und $R^{2a}$ miteinander verbunden sein können, um einen Ring zu bilden,
$R^{3a}$ und $R^{4a}$ miteinander verbunden sein können, um einen Ring zu bilden,
$R^{4a}$ und $R^{5a}$ miteinander verbunden sein können, um einen Ring zu bilden, und
$R^{5a}$ und $R^{6a}$ miteinander verbunden sein können, um einen Ring zu bilden,
wobei in einem Fall, in dem $R^{3a}$ und $R^{6a}$ eine Acyloxygruppe darstellen, mindestens eines von $R^{4a}$ und $R^{5a}$ eine Alkoxygruppe darstellt,

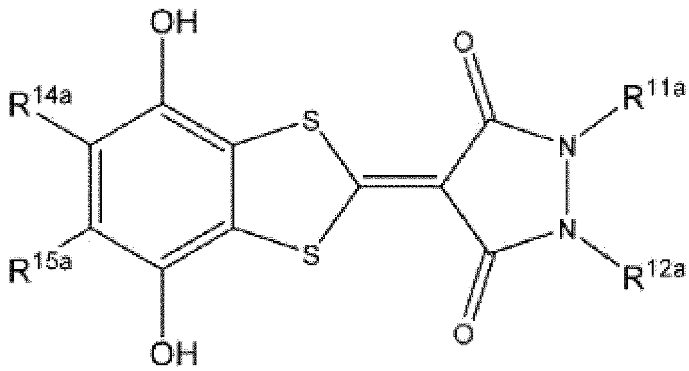

(2a)

in Formel (2a) $R^{11a}$ und $R^{12a}$ jeweils voneinander unabhängig eine Alkylgruppe darstellen,
$R^{14a}$ eine Alkylgruppe oder eine Alkoxygruppe darstellt,
$R^{15a}$ ein Wasserstoffatom, eine Alkylgruppe oder eine Alkoxygruppe darstellt, und
$R^{14a}$ und $R^{15a}$ miteinander verbunden sein können, um einen Ring zu bilden.

## Revendications

**1.** Composition de résine comprenant :

un composé représenté par Formule (1) ; et
une résine,

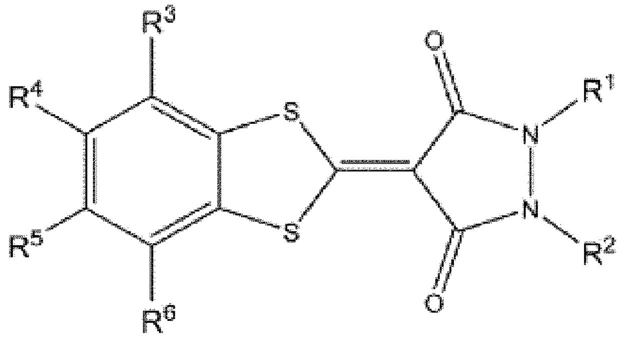

(1)

dans Formule (1), $R^1$ et $R^2$ représentent chacun indépendamment un groupe alkyle, un groupe aryle ou un groupe hétérocyclique,
$R^3$ et $R^6$ représentent chacun indépendamment un groupe alkoxy, un groupe acyloxy, un groupe carbamoyloxy ou un groupe alkoxycarbonyloxy,
$R^4$ représente un groupe alkyle, un groupe aryle, un groupe alkoxy ou un groupe aryloxy,
$R^5$ représente un atome d'hydrogène, un groupe alkyle, un groupe aryle, un groupe alkoxy ou un groupe aryloxy,
$R^1$ et $R^2$ peuvent être liés l'un à l'autre pour former un cycle,
$R^3$ et $R^4$ peuvent être liés l'un à l'autre pour former un cycle,
$R^4$ et $R^5$ peuvent être liés l'un à l'autre pour former un cycle, et
$R^5$ et $R^6$ peuvent être liés l'un à l'autre pour former un cycle,
où dans un cas où $R^3$ et $R^6$ représentent chacun indépendamment un groupe acyloxy ou un groupe carbamoyloxy, au moins l'un de $R^4$ et $R^5$ représente un groupe aryle, un groupe alkoxy ou un groupe aryloxy.

**2.** Composition de résine selon la revendication 1,
dans laquelle dans Formule (1), au moins l'un de $R^3$ et $R^6$ représente un groupe alkoxy.

**3.** Composition de résine selon la revendication 1,

dans laquelle le composé représenté par Formule (1) est un composé représenté par Formule (1a),

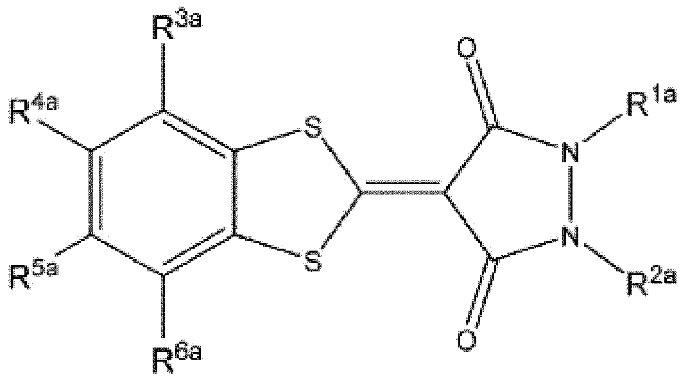

(1a)

dans Formule (1a), $R^{1a}$ et $R^{2a}$ représentent chacun indépendamment un groupe alkyle, $R^{3a}$ et $R^{6a}$ représentent chacun indépendamment un groupe alkoxy ou un groupe acyloxy,
$R^{4a}$ représente un groupe alkyle ou un groupe alkoxy,
$R^{5a}$ représente un atome d'hydrogène, un groupe alkyle ou un groupe alkoxy,
$R^{1a}$ et $R^{2a}$ peuvent être liés l'un à l'autre pour former un cycle,
$R^{3a}$ et $R^{4a}$ peuvent être liés l'un à l'autre pour former un cycle,
$R^{4a}$ et $R^{5a}$ peuvent être liés l'un à l'autre pour former un cycle, et
$R^{5a}$ et $R^{6a}$ peuvent être liés l'un à l'autre pour former un cycle,
où dans un cas où $R^{3a}$ et $R^{6a}$ représentent un groupe acyloxy, au moins l'un de $R^{4a}$ et $R^{5a}$ représente un groupe alkoxy.

**4.** Composition de résine selon l'une quelconque des revendications 1 à 3, comprenant en outre :

un composé représenté par Formule (2),

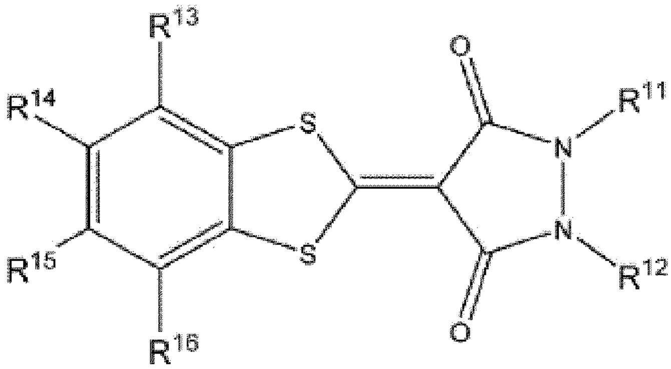

(2)

dans Formule (2), $R^{11}$ et $R^{12}$ représentent chacun indépendamment un groupe alkyle, un groupe aryle ou un groupe hétérocyclique,
$R^{13}$ et $R^{16}$ représentent chacun indépendamment un groupe hydroxy, un groupe alkoxy, un groupe aryloxy, un groupe acyloxy, un groupe carbamoyloxy, un groupe alkoxycarbonyloxy, un groupe aryloxycarbonyloxy, un groupe sulfinyloxy ou un groupe sulfonyloxy,
$R^{14}$ représente un groupe alkyle, un groupe aryle, un groupe alkoxy, un groupe aryloxy, un groupe acyloxy, un groupe alkylamino, un groupe anilino, un groupe acylamino, un groupe alkylsulfonylamino, un groupe arylsulfonylamino, un groupe alkylthio ou un groupe arylthio,
$R^{15}$ représente un atome d'hydrogène, un groupe alkyle, un groupe aryle, un groupe alkoxy, un groupe aryloxy, un groupe acyloxy, un groupe alkylamino, un groupe anilino, un groupe acylamino, un groupe alkylsulfonylamino, un groupe arylsulfonylamino, un groupe alkylthio ou un groupe arylthio,
$R^{11}$ et $R^{12}$ peuvent être liés l'un à l'autre pour former un cycle,
$R^{13}$ et $R^{14}$ peuvent être liés l'un à l'autre pour former un cycle,
$R^{14}$ et $R^{15}$ peuvent être liés l'un à l'autre pour former un cycle, et
$R^{15}$ et $R^{16}$ peuvent être liés l'un à l'autre pour former un cycle,
où au moins l'un de $R^{13}$ et $R^{16}$ représente un groupe hydroxy.

**5.** Composition de résine selon l'une quelconque des revendications 1 à 4, comprenant en outre :
un agent d'absorption d'ultraviolets autre que le composé représenté par Formule (1).

**6.** Composition de résine selon l'une quelconque des revendications 1 à 5,
dans laquelle la résine est au moins l'une choisie parmi une résine (méth)acrylique, une résine de polystyrène, une résine de polyester, une résine de polyuréthane, une résine de polythiourethane, une résine de polyimide, une résine époxy, une résine de polycarbonate ou une résine d'acylate de cellulose.

**7.** Substance durcie qui est formée de la composition de résine selon l'une quelconque des revendications 1 à 6.

**8.** Agent d'absorption d'ultraviolets comprenant :

un composé représenté par Formule (1),

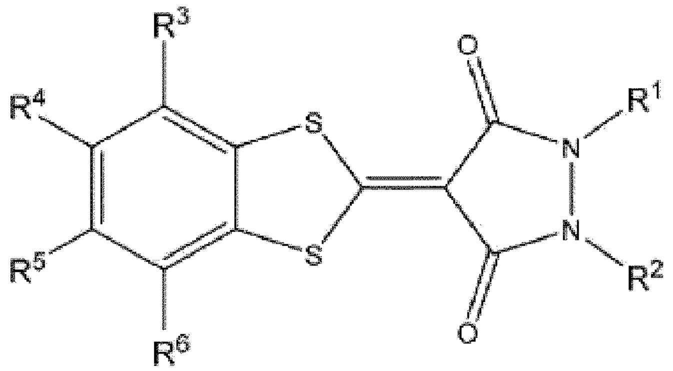

(1)

dans Formule (1), $R^1$ et $R^2$ représentent chacun indépendamment un groupe alkyle, un groupe aryle ou un groupe hétérocyclique,
$R^3$ et $R^6$ représentent chacun indépendamment un groupe alkoxy, un groupe acyloxy, un groupe carbamoyloxy ou un groupe alkoxycarbonyloxy,
$R^4$ représente un groupe alkyle, un groupe aryle, un groupe alkoxy ou un groupe aryloxy,
$R^5$ représente un atome d'hydrogène, un groupe alkyle, un groupe aryle, un groupe alkoxy ou un groupe aryloxy,
$R^1$ et $R^2$ peuvent être liés l'un à l'autre pour former un cycle,
$R^3$ et $R^4$ peuvent être liés l'un à l'autre pour former un cycle,
$R^4$ et $R^5$ peuvent être liés l'un à l'autre pour former un cycle, et
$R^5$ et $R^6$ peuvent être liés l'un à l'autre pour former un cycle,
où dans un cas où $R^3$ et $R^6$ représentent chacun indépendamment un groupe acyloxy ou un groupe carbamoyloxy, au moins l'un de $R^4$ et $R^5$ représente un groupe aryle, un groupe alkoxy ou un groupe aryloxy.

**9.** Agent d'absorption d'ultraviolets selon la revendication 8, comprenant en outre :

le composé représenté par Formule (2),

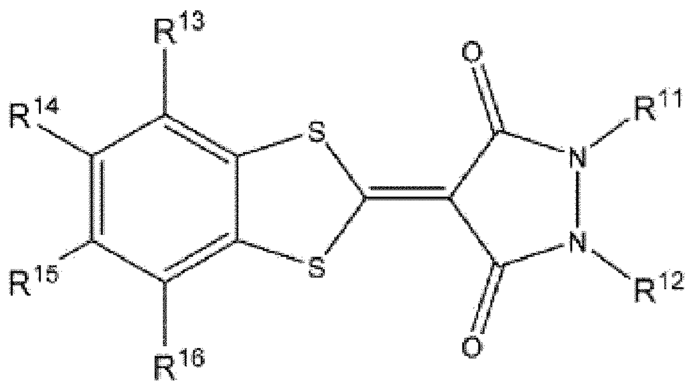

(2)

dans Formule (2), $R^{11}$ et $R^{12}$ représentent chacun indépendamment un groupe alkyle, un groupe aryle ou un groupe hétérocyclique,
$R^{13}$ et $R^{16}$ représentent chacun indépendamment un groupe hydroxy, un groupe alkoxy, un groupe aryloxy, un groupe acyloxy, un groupe carbamoyloxy, un groupe alkoxycarbonyloxy, un groupe aryloxycarbonyloxy, un groupe sulfinyloxy ou un groupe sulfonyloxy,
$R^{14}$ représente un groupe alkyle, un groupe aryle, un groupe alkoxy, un groupe aryloxy ou un groupe acyloxy, un groupe alkylamino, un groupe anilino, un groupe acylamino, un groupe alkylsulfonylamino, un groupe arylsulfonylamino, un groupe alkylthio ou un groupe arylthio,
$R^{15}$ représente un atome d'hydrogène, un groupe alkyle, un groupe aryle, un groupe alkoxy, un groupe aryloxy, un groupe acyloxy, un groupe alkylamino, un groupe anilino, un groupe acylamino, un groupe alkylsulfonylamino, un groupe arylsulfonylamino, un groupe alkylthio ou un groupe arylthio,
$R^{11}$ et $R^{12}$ peuvent être liés l'un à l'autre pour former un cycle,

$R^{13}$ et $R^{14}$ peuvent être liés l'un à l'autre pour former un cycle,
$R^{14}$ et $R^{15}$ peuvent être liés l'un à l'autre pour former un cycle, et
$R^{15}$ et $R^{16}$ peuvent être liés l'un à l'autre pour former un cycle,
où au moins l'un de $R^{13}$ et $R^{16}$ représente un groupe hydroxy.

**10.** Filtre coupe-ultraviolet comprenant :
l'agent d'absorption d'ultraviolets selon la revendication 8 ou la revendication 9.

**11.** Lentille comprenant :
l'agent d'absorption d'ultraviolets selon la revendication 8 ou la revendication 9.

**12.** Matériau protecteur comprenant :
l'agent d'absorption d'ultraviolets selon la revendication 8 ou la revendication 9.

**13.** Composé représenté par Formule (1a),

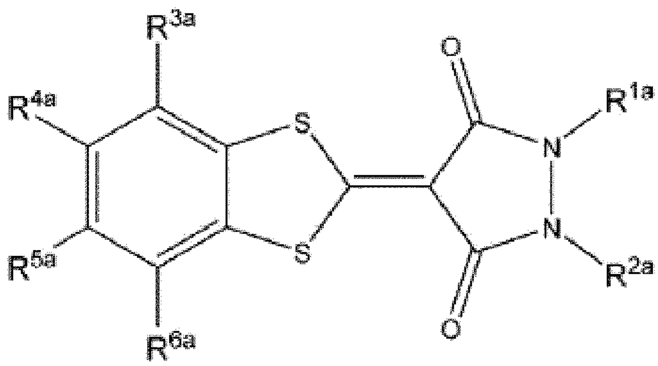

(1a)

dans Formule (1a), $R^{1a}$ et $R^{2a}$ représentent chacun indépendamment un groupe alkyle,
$R^{3a}$ et $R^{6a}$ représentent chacun indépendamment un groupe alkoxy ou un groupe acyloxy,
$R^{4a}$ représente un groupe alkyle ou un groupe alkoxy,
$R^{5a}$ représente un atome d'hydrogène, un groupe alkyle ou un groupe alkoxy,
$R^{1a}$ et $R^{2a}$ peuvent être liés l'un à l'autre pour former un cycle,
$R^{3a}$ et $R^{4a}$ peuvent être liés l'un à l'autre pour former un cycle,
$R^{4a}$ et $R^{5a}$ peuvent être liés l'un à l'autre pour former un cycle, et
$R^{5a}$ et $R^{6a}$ peuvent être liés l'un à l'autre pour former un cycle,
où dans un cas où $R^{3a}$ et $R^{6a}$ représentent un groupe acyloxy, au moins l'un de $R^{4a}$ et $R^{5a}$ représente un groupe alkoxy.

**14.** Procédé de synthèse d'un composé représenté par Formule (1a), comprenant :

réagir un composé représenté par Formule (2a) avec un composé d'halogénure d'alkyle ou un halogénure d'acide carboxylique,

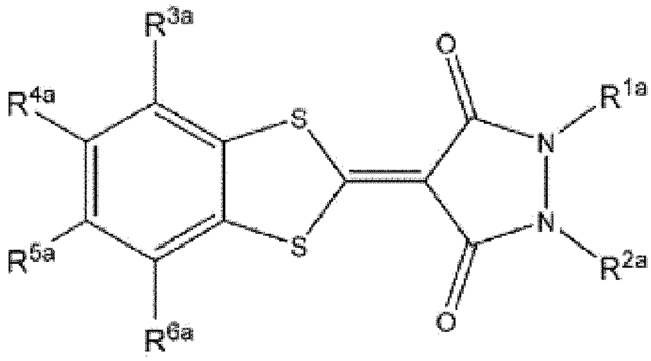

(1a)

dans Formule (1a), $R^{1a}$ et $R^{2a}$ représentent chacun indépendamment un groupe alkyle,
$R^{3a}$ et $R^{6a}$ représentent chacun indépendamment un groupe alkoxy ou un groupe acyloxy,
$R^{4a}$ représente un groupe alkyle ou un groupe alkoxy,
$R^{5a}$ représente un atome d'hydrogène, un groupe alkyle ou un groupe alkoxy,
$R^{1a}$ et $R^{2a}$ peuvent être liés l'un à l'autre pour former un cycle,
$R^{3a}$ et $R^{4a}$ peuvent être liés l'un à l'autre pour former un cycle,
$R^{4a}$ et $R^{5a}$ peuvent être liés l'un à l'autre pour former un cycle, et
$R^{5a}$ et $R^{6a}$ peuvent être liés l'un à l'autre pour former un cycle,
où dans un cas où $R^{3a}$ et $R^{6a}$ représentent un groupe acyloxy, au moins l'un de $R^{4a}$ et $R^{5a}$ représente un groupe alkoxy,

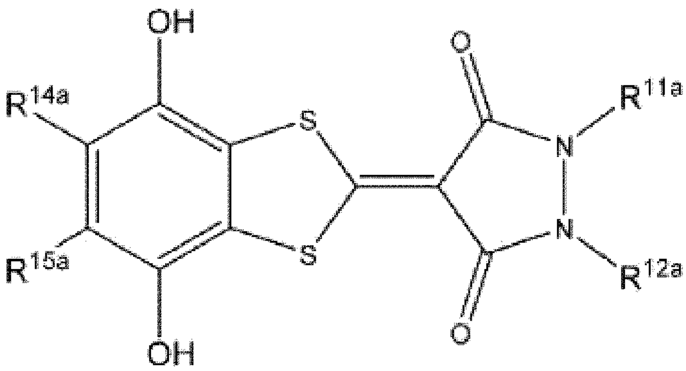

(2a)

dans Formule (2a), $R^{11a}$ et $R^{12a}$ représentent chacun indépendamment un groupe alkyle,
$R^{14a}$ représente un groupe alkyle ou un groupe alkoxy,
$R^{15a}$ représente un atome d'hydrogène, un groupe alkyle ou un groupe alkoxy, et
$R^{14a}$ et $R^{15a}$ peuvent être liés l'un à l'autre pour former un cycle.

FIG. 1

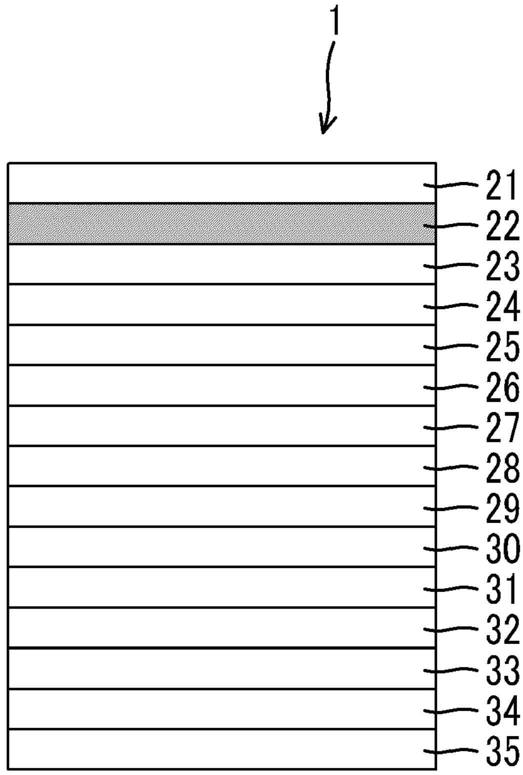
1
21
22
23
24
25
26
27
28
29
30
31
32
33
34
35

FIG. 2

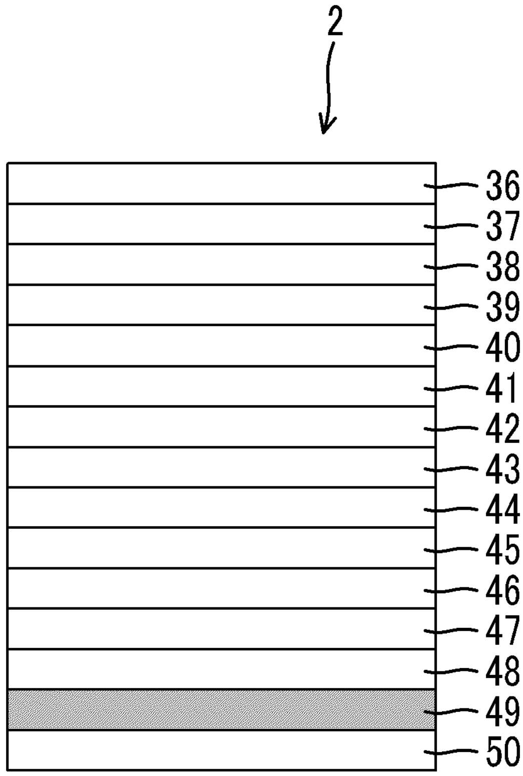
2
36
37
38
39
40
41
42
43
44
45
46
47
48
49
50

FIG. 3

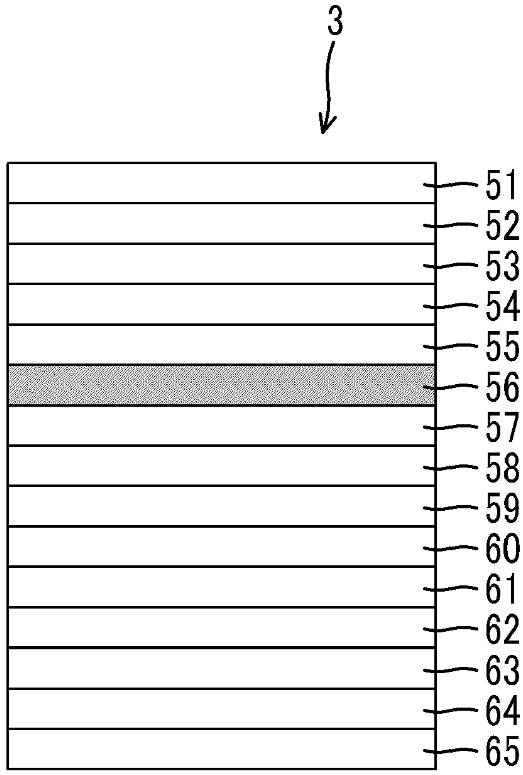
3
51
52
53
54
55
56
57
58
59
60
61
62
63
64
65

FIG. 4

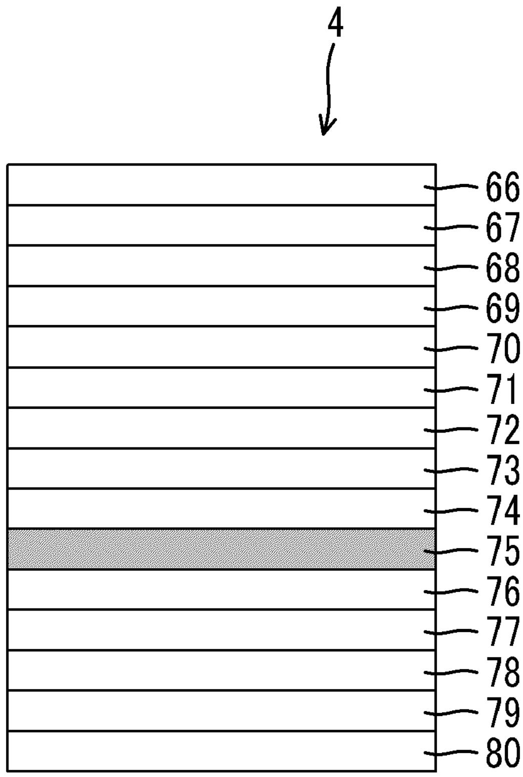
4
66
67
68
69
70
71
72
73
74
75
76
77
78
79
80

FIG. 5

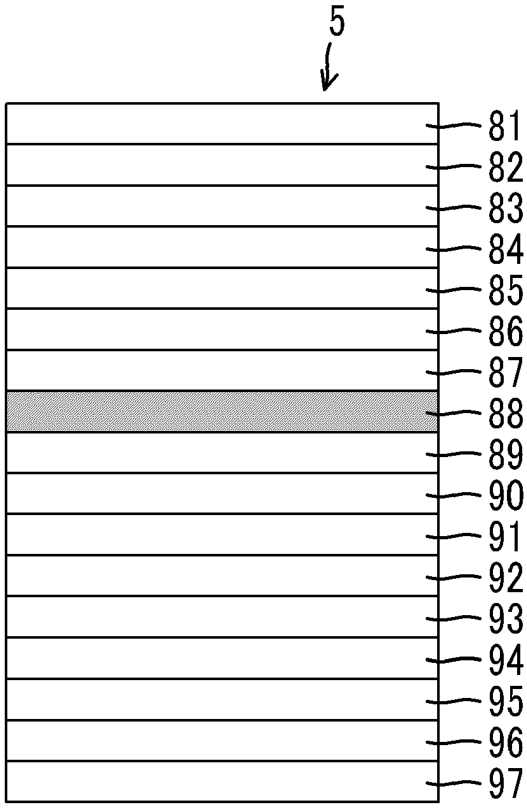
5
81
82
83
84
85
86
87
88
89
90
91
92
93
94
95
96
97

FIG. 6

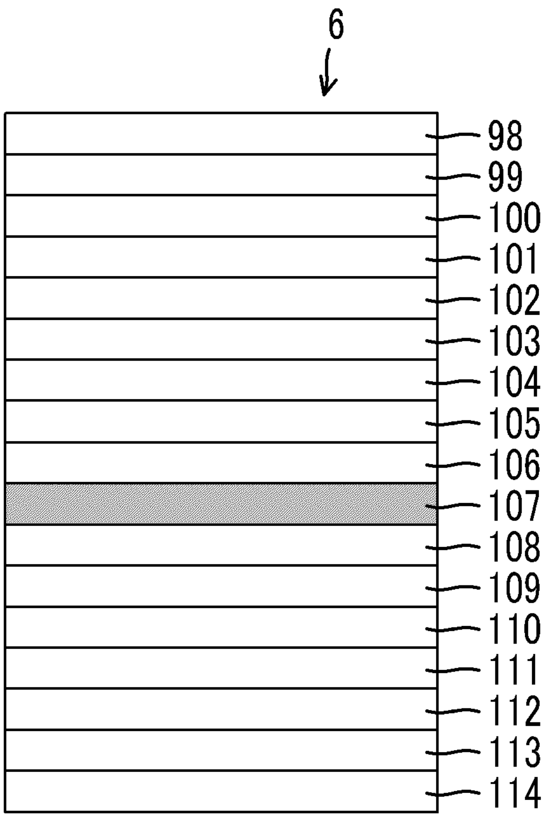
6
98
99
100
101
102
103
104
105
106
107
108
109
110
111
112
113
114

FIG. 7

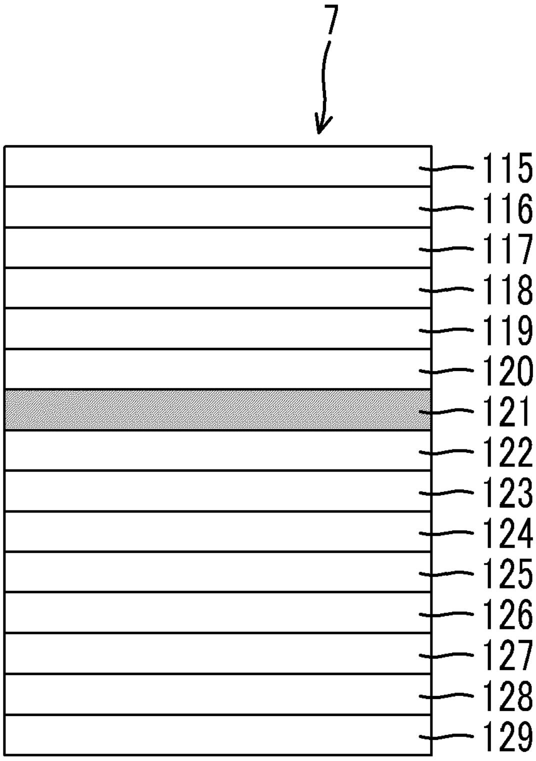
7
115
116
117
118
119
120
121
122
123
124
125
126
127
128
129

FIG. 8

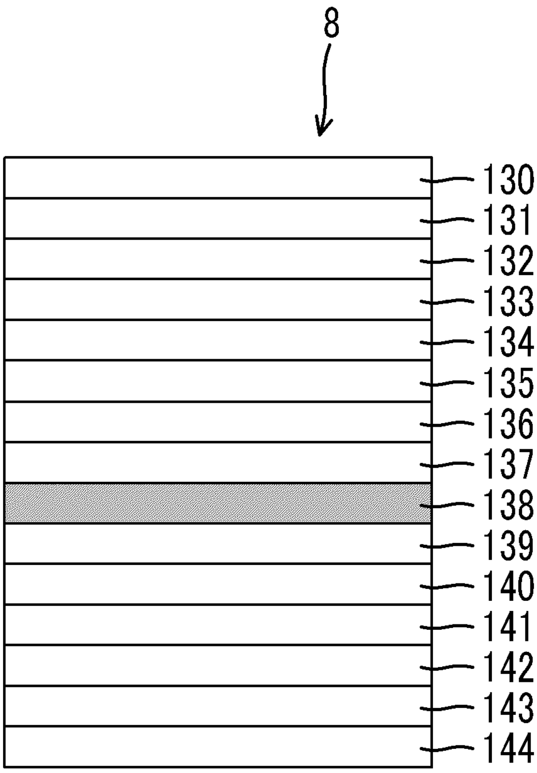
8
130
131
132
133
134
135
136
137
138
139
140
141
142
143
144

FIG. 9

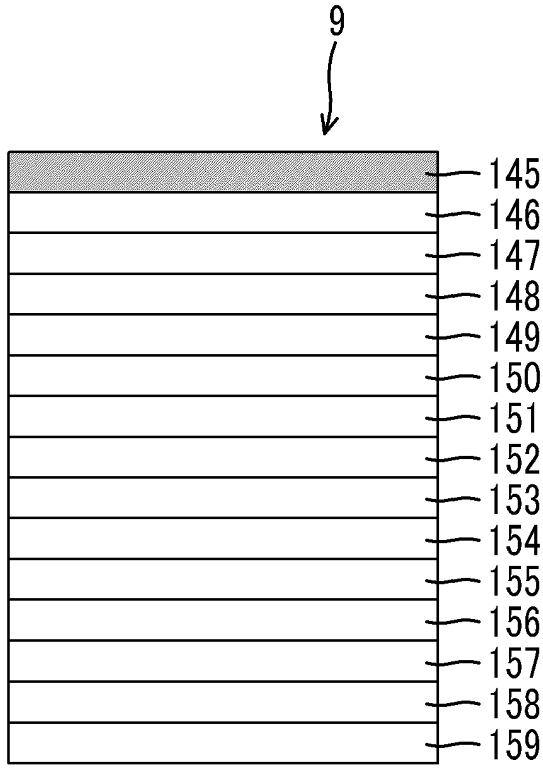
9
145
146
147
148
149
150
151
152
153
154
155
156
157
158
159

FIG. 10

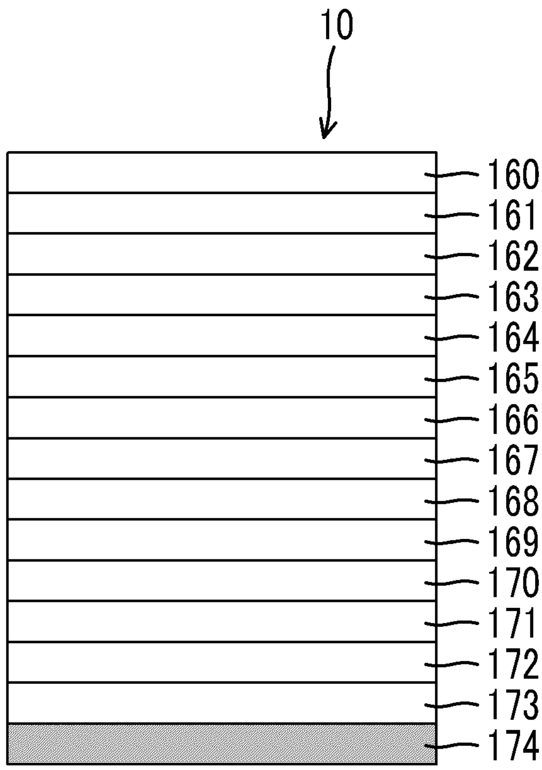
10
160
161
162
163
164
165
166
167
168
169
170
171
172
173
174

FIG. 11

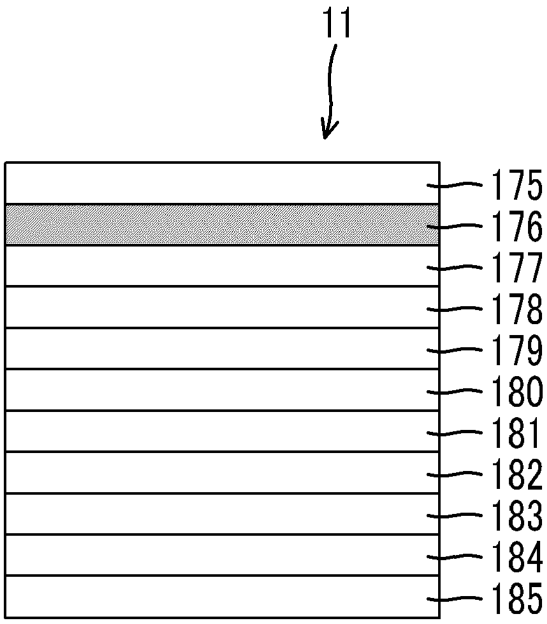
11
175
176
177
178
179
180
181
182
183
184
185

FIG. 12

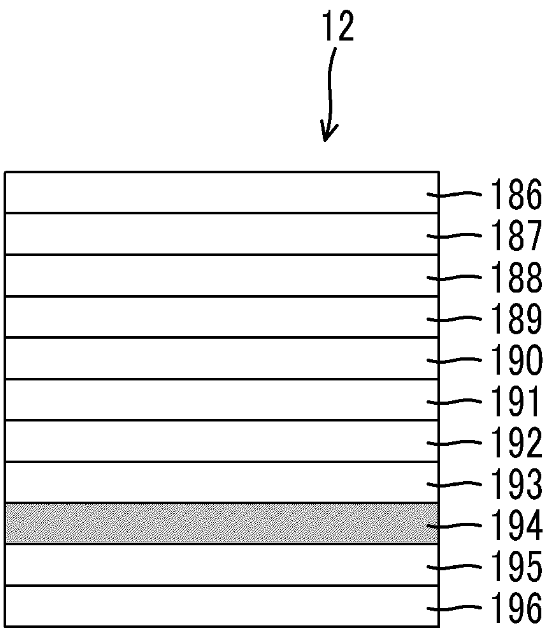
12
186
187
188
189
190
191
192
193
194
195
196

FIG. 13

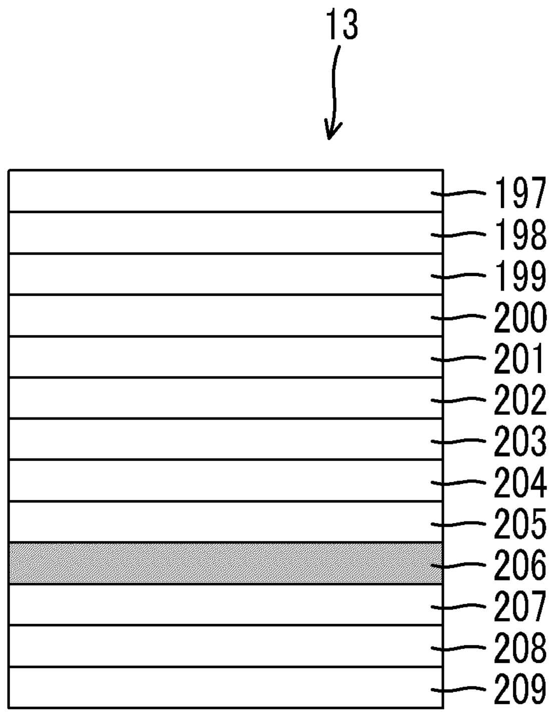

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019159570 A
- JP 2016081035 A
- JP 5376885 B
- WO 2019142539 A
- JP 2009263616 A
- WO 2017122503 A
- JP 2012215689 A
- JP 2009191179 A
- JP 10291969 A
- JP H10291969 A
- JP 4225898 B
- JP 2001233842 A
- JP 2000080068 A
- JP 2006342166 A
- JP 2016006475 A
- JP 2002029162 A
- JP 2008013646 A
- JP 2009288703 A
- JP 2009242604 A
- JP 2009263617 A
- WO 2019131572 A
- JP 2013045045 A
- JP 2013043352 A
- JP 2012232459 A
- JP 2012128157 A
- JP 2011131409 A
- JP 2011131404 A
- JP 2011126162 A
- JP 2011075705 A
- JP 2009286981 A
- JP 2009263567 A
- JP 2009075248 A
- JP 2007164206 A
- JP 2006096811 A
- JP 2004075970 A
- JP 2002156505 A
- JP 2001272503 A
- WO 2012018087 A
- WO 2012098967 A
- WO 2012086659 A
- WO 2011105594 A
- JP 2017142412 A
- WO 2009003164 A
- JP 10045804 A
- JP H10405804 A
- JP 8231761 A
- JP H08231761 A
- JP 6095766 B
- WO 2015005398 A
- JP 4260332 B
- JP 4001009 A
- JP H04001009 A
- JP 2006122889 A


### Non-patent literature cited in the description


- **MARUZEN**. The Fourth Series of Experimental Chemistry 29 Polymer Material. 1992, 225-232
- *Journal of the American Chemical Society*, 2009, vol. 131 (33), 11875-11881